# EUROPEAN PATENT SPECIFICATION

(11) **EP 0 739 351 B1**
(45) Date of publication and mention of the grant of the patent: **10.04.2002**
(21) Application number: 95908491.4
(22) Date of filing: 11.01.1995
(51) Int. Cl.: C07H 21/04, C07F 9/143, C07F 9/24, C07F 9/572, C07F 9/59, C07F 9/6503, C07F 9/6512, C07F 9/6533, C07F 9/6547, C07F 9/6561, C07H 21/00, C07F 9/6506, C07F 9/6521

(54) **MONOMERIC DIOLS AND PHOSPHATE LINKED OLIGOMERS FORMED THEREFROM**
MONOMERE DIOLE UND DARAUS GEBILDETE PHOSPHATVERKNÜPFTE OLIGOMERE
DIOLS MONOMERES ET OLIGOMERES A LIAISON PHOSPHATE FORMES A PARTIR DE CES DERNIERS

(30) Priority: 11.01.1994 US 179970
(43) Date of publication of application: 30.10.1996
(73) Proprietor: ISIS PHARMACEUTICALS, INC., Carlsbad, CA 92008 (US)
(72) Inventor: COOK, Phillip, Dan, Vista, CA 92084 (US); ACEVEDO, Oscar, L., San Diego, CA 92129 (US); DAVIS, Peter, W., Carlsbad, CA 92008 (US); ECKER, David, J., Leucadia, CA 92024 (US); HEBERT, Normand, Cardiff, CA 92007 (US)
(74) Representative: Hallybone, Huw George
(86) International application number: PCT/US95/00449
(87) International publication number: WO 95/18820

(56) References cited:
- EP-A- 0 313 219
- WO-A-92/02532
- US-A- 5 212 295
- V. A. KORSHUN ET.AL.: "Reagent for Introducing Pyrene Residues in Oligonucleotides" BIOCONJUGATE CHEMISTRY, vol. 3, no. 6, November 1992, pages 559-62, XP002073793
- J. GRZYBOWSKI ET. AL. : "Synthesis and antibody-mediated detection of oligonucleotides containing multiple 2,4-dinitrophenyl reporter groups." NUCLEIC ACIDS RESEARCH, vol. 21, no. 8, August 1993, pages 1705-12, XP002073794
- M-L FONTANEL ET. AL.: "End-Attachment of Phenol-Oligoncleotide Conjugates to diazotized Cellulose." BIOCONJUGATE CHEMISTRY, vol. 4, no. 5, September 1993, pages 380-5, XP002073795
- P. S. NELSON ET.AL.: "A new and versatile reagent for incorporating multiple primary aliphatic amines into synthetic oligonucleutides." NUCLEIC ACIDS RESEARCH, vol. 17, no. 18, 1989, pages 7179-86, XP002073796
- C. MACKELLAR ET. AL.: "Synthesis and physical properties of ant-HIV antisense oligonucleotides bearing terminal lipophilic groups." NUCLEIC ACIDS RESEARCH, vol. 20, no. 13, 1992, pages 3411-7, XP002073797
- K. AGUSTYNS ET.AL.: "Influence of the incorporation of (S)-9-(3,4-dihydroxybutyl)adenine on the enzymatic stability and base pairing properties of oligodeoxynucleotides." NUCLEIC ACIDS RESEARCH, vol. 19, no. 10, 1991, pages 2587-93, XP002073798

## Description

This invention is directed to alkylene glycol monomeric units and to oligomers constructed from these units. The oligomers can be synthesized to have either random or predefined sequences of monomeric units and can be joined via phosphate linkages, including phosphorothioate, phosphodiester, and phosphoramidate linkages. Each of the monomeric units can include a chemical moiety thereon for binding of the oligomeric structures to proteins, nucleic acid, and other biological targets. In preferred embodiments, the compounds of the invention act as inhibitors of enzymes such as phospholipase A₂ and are used for the treatment of inflammatory diseases including atopic dermatitis and inflammatory bowel disease.

Phospholipases A₂ (PLA₂) are a family of enzymes that hydrolyze the *sn*-2 ester linkage of membrane phospholipids resulting in release of a free fatty acid and a lysophospholipid (see, Dennis, E.A., The Enzymes, Vol. 16, pp. 307-353, Boyer, P.D., ed., Academic Press, New York, 1983). Elevated levels of type II PLA₂ are correlated with a number of human inflammatory diseases. The PLA₂-catalyzed reaction is the rate-limiting step in the release of a number of pro-inflammatory mediators. Arachidonic acid, a fatty acid commonly linked at the *sn*-2 position, serves as a precursor to leukotrienes, prostaglandins, lipoxins and thromboxanes. The lysophospholipid can be a precursor to platelet-activating factor. PLA₂ is regulated by pro-inflammatory cytokines and, thus, occupies a central position in the inflammatory cascade (*see, e.g.*, Dennis, *ibid.;* Glaser, *et al., TiPs Reviews* **1992,** *14*, 92; and Pruzanski, *et al., Inflammation* **1992,** *16*, 451).

All mammalian tissues evaluated thus far have exhibited PLA₂ activity. At least three different types of PLA₂ are found in humans: pancreatic (type I), synovial fluid (type II) and cytosolic. Studies suggest that additional isoenzymes exist. Type I and type II, the secreted forms of PLA₂, share strong similarity with phospholipases isolated from the venom of snakes. The PLA₂ enzymes are important for normal functions including digestion, cellular membrane remodeling and repair, and in mediation of the inflammatory response. Both cytosolic and type II enzymes are of interest as therapeutic targets. Increased levels of the type II PLA₂ are correlated with a variety of inflammatory disorders including rheumatoid arthritis, osteoarthritis, inflammatory bowel disease and septic shock, suggesting that inhibitors of this enzyme would have therapeutic utility. Additional support for a role of PLA₂ in promoting the pathophysiology observed in certain chronic inflammatory disorders was the observation that injection of type II PLA₂ into the footpad of rats (Vishwanath, *et al., Inflammation* **1988,** *12*, 549) or into the articular space of rabbits (Bomalaski, *et al., J. Immunol.* **1991,** *146*, 3904) produced an inflammatory response. When the protein was denatured before injection, no inflammatory response was produced.

The type II PLA₂ enzyme from synovial fluid is a relatively small molecule (about 14 kD) and can be distinguished from type I enzymes (*e.g.,* pancreatic) by the sequence and pattern of its disulfide bonds. Both types of enzymes require calcium for activity. The crystal structures of secreted PLA₂ enzymes from venom and pancreatic PLA₂, with and without inhibitors, have been reported (Scott, *et al*., *Science* **1990,** *250,* 1541). Recently, the crystal structure of PLA₂ from human synovial fluid has been solved (Wery, *et al*., *Nature* **1991**, *352*, 79). The structures clarify the role of calcium and amino acid residues in catalysis. The calcium acts as a Lewis acid to activate the scissile ester carbonyl and bind the lipid, and a His-Asp side chain dyad acts as general base catalyst to activate a water molecule nucleophile. This is consistent with the absence of any acyl enzyme intermediates, and is also comparable to the catalytic mechanism of serine proteases. The catalytic residues and the calcium ion are at the end of a deep cleft (ca. 14 Å) in the enzyme. The walls of this cleft contact the hydrocarbon portion of the phospholipid and are composed of hydrophobic and aromatic residues. The positively-charged amino-terminal helix is situated above the opening of the hydrophobic cleft. Several lines of evidence suggest that the N-terminal portion is the interfacial binding site. (*see, e.g.,* Achari, *et al., Cold Spring* Harbor *Symp. Quant. Biol*. **1987,** *52,* 441; Cho, et *al., J. Biol. Chem.* **1988,** *263*, 11237; Yang, *et al., Biochem. J.* **1989,** *262,* 855; and Noel, *et al*., *J*. *Am. Chem. Soc.* **1990,** *112*, 3704).

Much work has been reported in recent years on the study of the mechanism and properties of PLA₂-catalyzed hydrolysis of phospholipids. In *in vitro* assays, PLA₂ displays a lag phase during which the enzyme adsorbs to the substrate bilayer and a process called interfacial activation occurs. This activation may involve desolvation of the enzyme/lipid interface or a change in the physical state of the lipid around the cleft opening. The evidence favoring this hypothesis comes from studies revealing that rapid changes in PLA₂ activity occur concurrently with changes in the fluorescence of a membrane probe (Burack, *et al., Biochemistry* **1993**, *32*, 583). This suggests that lipid rearrangement is occurring during the interfacial activation process. PLA₂ activity is maximal around the melting temperature of the lipid, where regions of gel and liquid-crystalline lipid coexist. This is also consistent with the sensitivity of PLA₂ activity to temperature and to the composition of the substrate, both of which can lead to structurally distinct lipid arrangements separated by a boundary region. Fluorescence microscopy was used to simultaneously identify the physical state of the lipid and the position of the enzyme during catalysis (Grainger, *et al*., *FEBS Lett.* **1989**, *252,* 73). These studies clearly show that PLA₂ binds exclusively at the boundary region between liquid and solid phase lipid.

While the hydrolysis of the secondary ester bond of 1,2-diacylglycerophospholipids catalyzed by the enzyme is relatively simple, the mechanistic and kinetic picture is clouded by the complexity of the enzyme-substrate interaction. A remarkable characteristic of PLA₂ is that maximal catalytic activity is observed on substrate that is aggregated (*i.e.*, phospholipid above its critical micelle concentration), while low levels of activity are observed on monomeric substrate. As a result, competitive inhibitors of PLA₂ either have a high affinity for the active site of the enzyme before it binds to the substrate bilayer or partition into the membrane and compete for the active site with the phospholipid substrate. Although a number of inhibitors appear to show promising inhibition of PLA₂ in biochemical assays (*see*, *e*.*g*., Yuan, et *al., J. Am. Chem. Soc.* **1987,** *109,* 8071; Lombardo, *et al*., *J. Biol. Chem.* **1985,** *260,* 7234; Washburn, *et al., J. Biol. Chem.* **1991,** *266,* 5042; Campbell, *et al., J. Chem. Soc., Chem. Commun.* **1988,** 1560; and Davidson, *et al., Biochem. Biophys. Res. Commun,* **1986,** *137*, 587), reports describing *in vivo* activity are limited (*see, e.g.,* Miyake, *et al., J. Pharmacol. Exp. Ther.* **1992**, *263,* 1302).

Traditional structure activity relationship type drug discovery gives unambiguous products but yet requires the preparation of numerous individual test candidates. The preparation of each structure requires significant amounts of time and resources. Another drug discovery approach, de novo design of active compounds based on high resolution enzyme structures, generally has not been successful. Yet another approach involves screening complex fermentation broths and plant extracts for a desired biological activity. The advantage of screening mixtures from biological sources is that a large number of compounds can be screened simultaneously, in some cases leading to the discovery of novel and complex natural products with activity that could not have been predicted otherwise. One disadvantage is that many different samples must be screened and numerous purifications must be carried out to identify the active component, which often is present only in trace amounts.

In order to maximize the advantages of each classical approach, new strategies for combinatorial unrandomization have been developed by several groups. Selection techniques have been used with libraries of peptides (*see, e.g.*, Geysen, *et al., J. Immun. Meth.* **1987,** *102*, 259; Houghten, *et al., Nature* **1991**, *354*, 84; and Owens, *et al*., *Biochem. Biophys. Res. Commun*. **1991,** *181*, 402) and nucleic acids (*see*, *e.g*,, Wyatt, *et al*., (in press) *Proc. Matl. Acad. Sci. USA*; and Ecker, *et al*., *Nucleic Acids Res.* **1993,** *21*, 1853). These selection techniques involve iterative synthesis and screening of increasingly simplified subsets of oligomers. In using these selection techniques, subsets are assayed for activity in either cell-based assays, or for binding or inhibition of purified protein targets.

One technique, called SURF (Synthetic Unrandomization of Randomized Fragments; *see, e.g.,* Ecker, *et al*., *ibid.*, involves the synthesis of subsets of oligomers containing a known residue at one fixed monomer position and equimolar mixtures of residues at all other positions. For a library of oligomers four residues long containing three monomers (A, B, C), three subsets would be synthesized (NNAN, NNBN, NNCN, where N represents equal incorporation of each of the three monomers). Each subset is then screened in a functional assay and the best subset is identified (e.g., NNAN). A second set of libraries is synthesized and screened, each containing the fixed residue from the previous round, and a second fixed residue (e.g. ANAN, BNAN, CNAN). Through successive rounds of screening and synthesis, a unique sequence with activity in the assay can be identified.

K. Agustyns *et al*. in Nucleic Acids Research vol. 19(10), pp 2587-93 (1991) describe the preparation of 1-(N-6-benzoyladenin-9-yl)-3-(O-2-cyanoethyl-N,N-diisopropylphosphoramidite)-4-(methoxytrityl)hydroxyl butane. The 3,4-dihydroxybutyl adenine linker in this document can dimerised via a phosphoramidite which is then oxidised to a phosphonate group The 3,4-dihydroxybutyl adenine linking groups and their dimers can be incorporated into oligonucleotides. No pharmaceutical applications of the compounds are given.

It is known from EP-A-0313219 to incorporate a 3-amino-1,2-propanediol group into an oligonucleotide via protection of the 1-hydroxy group by trityl, and reacting the 2-hydroxy group with 2-cyanoethoxy-N,N-diisopropyl phosphoramidite.

C. Mackellar et *al*. in Nucleic Acids Research vol. 20(13), pp 3411-17 (1992) describe 1-(4-4'-dimethoxytrityl)-2-(cyanoethyl-N,N-diisopropylphosphoramidite)-octadecane-1,2-ol. This compound is used for the introduction of lipophilic groups into oligonucleotides, but no pharmaceutical use has been described.

P.S. Nelson *et al.* in Nucleic Acids Research vol. 17(18), pp 7179-86 (1989) describe 1-trityl-2-(2-cyanoetnyl-N,N-diisopropylphosphoramidite)-3-Fmoc-amino-propane. No pharmaceutical use of the compound is described.

J. Grzybowski *et al*. in Nucleic Acids Research vol. 21(8), pp 1705-12 (1993) describe 1-(dimethoxytrityl)-2-(2-cyanoethyl-N,N-diisopropylphosphoramidite)-6-dinitrofluorophenyl-hexane compound 2g). This compound is used for colorimetric labelling of oligonucleotides. No pharmaceutical application of the oligonucleotides is described.

M-L. Fontanel *et al*. in Bioconjugate Chemistry vol, 4(5), pp 380-5 (1993) describe 1-dimethoxytrityl-2-(2-cyanoethyl-N,N-diisopropylphosphoramidite-6-(4-ethyloxycarbonyloxyphenylethyl)carbonylamino)-hexane. The preparation of an oligonucleotide modified by four linker groups derived from compound 10 joined by phosphonyl groups is also described.

V.A. Korshun *et al.* in Bioconjugate Chemistry vol 3(6), pp 559-62 (1992) describe 1-dimethoxytrityl-2-(2-cyanoethyl-N,N-diisopropylphosphoramidite)-3-(pyrenylpropylcarbonylamino)-propane (see chart 1, compound 5). The use of this compound for labelling oligonucleotides is described.

It is an object of this invention to provide novel alkane glycol monomeric units.

It is another object of the invention to provide novel alkane glycol monomeric units that can be incorporated into novel oligomeric structures.

It is a further object to provide novel alkane glycol monomeric units that can be linked together via phosphorus-containing backbones.

It is still another object to provide novel alkane glycol based oligomers that include a diversity of functional moieties thereon for binding to biological sites of interest.

In a first aspect, the present invention provides a compound of structure I: 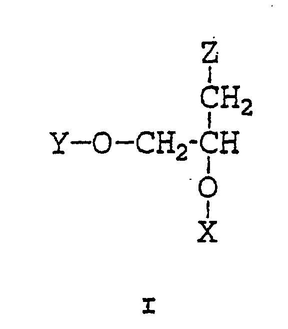 wherein X, Y and Z are as defined in claim 1.

In preferred embodiments, Y is an acid labile hydroxyl protecting group such as a trityl, methoxytrityl, dimethoxytrityl or trimethoxytrityl group.

In one preferred group of compounds, Z includes a nitrogen-containing heterocycle such as an imidazole or carbazole ring. In a further preferred group, Z includes a purine or pyrimidine nucleobase. Particularly preferred are compounds wherein X is an activated phosphite, Y is an acid labile hydroxyl protecting group, and Z is adenine, guanine, cytosine, uridine or thymine.

The present invention also provides oligomeric compounds of structure II: 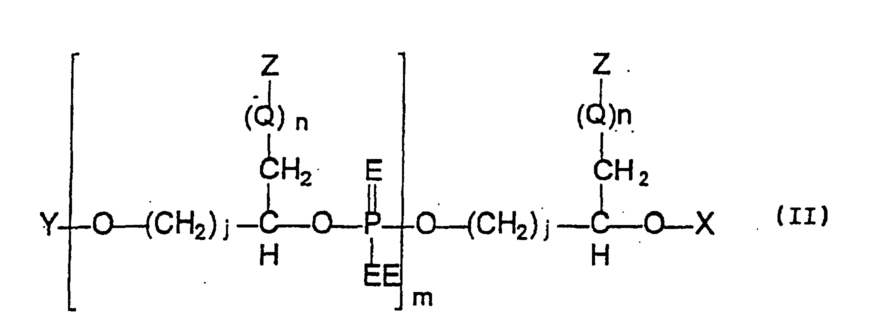 wherein X, Y, Z, E, Q, EE, j, m and n are as defined in claim 5.

The invention further includes processes for preparing randomized oligomeric compounds according to the present invention including the steps of selecting a group of monomers of formula III, 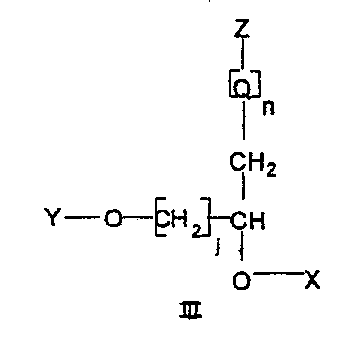 wherein X, Y, Z, Q, j and n are as defined in claim 27, and covalently bonding at least two of the monomers of said group to form said oligomeric compound.

The Z moiety of at least one monomer of said group is different from the Z moiety of another monomer of said group. Compounds prepared by this process preferably are randomized oligomeric compounds having from 2 to 50 monomers, more preferably 2 to about 25 monomers.

Further compounds of the invention include chimeric oligomeric compounds as defined in claim 31, having a central region comprising a phosphodiester or a phosphorothioate oligodeoxynucleotide interspaced between flanking regions.

The compounds of the invention can be used as inhibitors of various enzymes including phospholipase A₂ enzyme. As inhibitors of phospholipase A₂, the compounds are useful for the treatment of inflammatory diseases including atopic dermatitis and inflammatory bowel disease. The oligomeric compounds of the invention can be used in diagnostics since they are capable of specifically hybridizing to nucleic acid of interest in the etiology of diseases. The compounds of the invention also can be used as research probes and primers, especially for the study of enzyme biochemistry and protein-nucleic acid interactions.

The numerous objects and advantages of the present invention may be better understood by those skilled in the art by reference to the accompanying figures, in which:

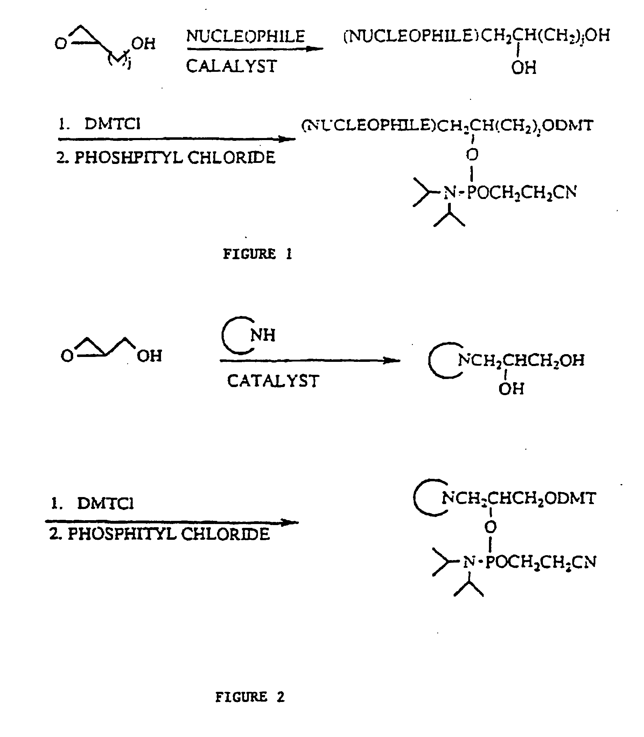

Figures 1-4 describe synthetic processes for activated phosphite monomers according to the invention.

The monomeric compounds of the invention can be considered for identification purposes as substituted alkane glycols, *i.e.*, substituted alkane diols, wherein the alkane portion has from 2 to 7 carbon atoms. In preferred embodiments, the hydroxyl groups of the alkane glycols are used to link adjacent monomers and form oligomeric structures. During oligomer synthesis, one of the glycol hydroxyl groups typically is blocked with a protecting group and the other hydroxyl group is reacted with an activated phosphate group such as a β-cyanoethyl phosphoramidate group. As used herein, the term activated phosphate group is intended to denote a phosphate group that bears a chemical modification thereon to enhance its reactivity with nucleophiles. Similarly, the term activated phosphite group denotes a phosphite group that bears a chemical modification to enhance its reactivity with nucleophiles. Numerous such modifications are known in the art.

The monomeric compounds of the invention preferably are covalently bound using phosphate linkages. This permits coupling via either solution phase or solid phase chemistries. Representative solution phase techniques are described in United States Patent No. 5,210,264, issued May 11, 1993 and commonly assigned with this invention. Representative solid phase techniques are those typically employed for DNA and RNA synthesis utilizing standard phosphoramidite chemistry. (*see*, *e.g.*, Protocols For Oligonucleotides And Analogs, Agrawal, S., ed., Humana Press, Totowa, NJ, 1993.) A preferred synthetic solid phase synthesis utilizes phosphoramidites as activated phosphates. The phosphoramidites utilize P^{III} chemistry. The intermediate phosphite compounds are subsequently oxidized to the P^{v} state using known methods. This allows for synthesis of the preferred phosphodiester or phosphorothioate phosphate linkages depending upon oxidation conditions selected. Other phosphate linkages can also be generated. These include phosphorodithioates, phosphotriesters, alkyl phosphonates, phosphoroselenates and phosphoamidates.

The alkane glycol moieties can be substituted with various functional groups. When the monomeric compounds are linked together, these functional groups provide diverse properties ("diversity") to the resulting oligomeric compounds. The functional groups include hydrogen-bond donors and acceptors, ionic moieties, polar moieties, hydrophobic moieties, aromatic centers, and electron-donors and acceptors. Together, the properties of the individual monomers contribute to the uniqueness of the oligomers in which they are found. Thus, a library of such oligomers would have a myriad of properties, i.e., "diversity." Collectively, the properties of the individual monomers that together form an oligomer contribute to the uniqueness of such oligomer and impart certain characteristics thereto for interaction with cellular, enzymatic or nucleic acid target sites. The functional groups can be directly linked to the alkane glycol "core" or "backbone" portion of the monomeric units or they can be connected thereto via a suitable tether. As will be recognized, the core portion of the monomeric units is formed from an alkane glycol moiety such as an ethylene glycol moiety. Hence, the oligomeric compounds of the invention are formed from a plurality of alkane glycol units, *e.g.,* ethylene glycol units, that bear functional groups and are linked together via phosphate linkages.

In a preferred process for preparing compounds of the invention, the alkane glycol cores are generated by ring-opening an epoxide compound. Preferred compounds are those where the variable j in structure I, above, is 1. This preference stems from the fact that stereospecific monomeric compounds wherein j = 1 can be prepared and, when incorporated into oligomeric structures, maintain their stereospecificity. Other members of this series, *e,g.*, j = 2 to 6, are prepared by methods analogous to those used to prepare compounds wherein j = 1. Since larger homologs are generally less stable, they preferably are prepared and used *in situ*. Such preparation and use can be effected generally in accordance with the methods of Ko, *et al., J. Org. Chem*. **1986,** *51*, 5413; Ko, *et. al., J. Org. Chem.* **1987,** *52,* 667; and Klunder, et. *al., J. Org. Chem.* **1989**, *54*, 1295. Thus, 3,4-epoxy-1-butanol, 4,5-epoxy-1-pentanol, 5,6-epoxy-1-hexanol, 6,7-epoxy-1-heptanol, and 7,8-epoxy-1-octanol can be prepared and used *in situ* to provide compounds of structure I wherein j = 2, 3, 4, 5, and 6, respectively. Glycidol (2,3-epoxy-1-propanol or oxiranemethanol) is particularly suitable for the preparation of compounds wherein j = 1 since upon ring opening it can supply both a primary hydroxyl group and a secondary hydroxyl group that can be included in a phosphate linkage in an oligomeric compound of the invention. The primary hydroxy group derives from the alkanol, *i.e.*, -CH₂OH, portion of glycidol, whereas the secondary hydroxyl group is generated upon ring opening of glycidol's epoxide ring.

Monomeric compounds of the invention can be prepared by nucleophilic addition of a functional group (or a tether for such functional group) to an epoxide ring-containing starting material. Generally, this addition is effected in a suitable solvent in the presence of a catalyst, as depicted in Figure 1. Where necessary, transient protecting groups are used to protect reactive functional groups.

Nucleophilic addition to the epoxide ring is an Sₙ2 reaction that occurs exclusively at the C-1 carbon. In compounds of structure I wherein j =1, racemic or nonracemic glycidol can be used as a starting material. When nonracemic (*i.e.*, chiral) glycidol is used, the product is also nonracemic (*see,* Hanson, Chemical *Reviews* **1991,** *91*, 437). This affords a simple way to acquire optically active monomers as well as oligomers incorporating such monomers.

For purposes of assigning names, compounds of the invention having structure I can be considered as substituted alkane compounds. For example, whenj = 1 , the resulting compound can be considered a substituted propane compound. Opening of an epoxide ring by nucleophilic addition of a functional group or a tether group yields a substituted diol compound, for example, a 1-substituted 2,3-diol propane having the functional group or the tether linked to the C1 carbon of the alkane moiety. The 1-substituted propane 2,3-diol has both a primary hydroxyl group (*i*.*e*., the hydroxyl linked to the C3 carbon, the hydroxy methyl group of the glycidol moiety) and a secondary hydroxyl group (*i.e.,* the hydroxyl group linked to the C2 carbon resulting from the opening of the epoxide ring of glycidol).

Although compounds of the invention having structure II wherein j = 1 are considered as propane compounds on a monomeric level, they are considered as substituted ethylene glycol compounds on an oligomeric level since only two of the three carbon atoms of the monomeric propane unit are included in the oligomeric backbone. By extension, butanol monomeric compounds (j = 2) become substituted propylene glycol oligomeric compounds, and so on for higher analogs. Thus, when a propane diol monomer is incorporated into oligomeric compounds of the invention, the C2 and C3 carbons of the propane diol moiety become part of the oligomeric ethylene glycol core whereas the C1 carbon of the propane moiety becomes an integral part of either a tether or a functional group extending from the core.

To facilitate inclusion into phosphate-linked oligomeric compounds of the invention, alkane diol-containing monomers that bear functional groups are activated with either an activated phosphite or an active phosphate moiety. Prior to phosphitylation of a secondary hydroxyl group (*e.g.*, when j = 1, the C2 propane diol hydroxyl group), the primary hydroxyl group (*e*.*g*., when j = 1, the C3 propane diol hydroxyl group) is protected. Protection of the primary hydroxyl is effected either before or after ring opening of the epoxide ring. Since addition of a protecting group to the methylhydroxyl group of glycidol renders its epoxide ring less reactive to unassisted nucleophilic addition, protection of the primary hydroxyl group normally will be effected after opening unless other considerations override such post-ring opening protection. When using nucleophiles that can react with the primary hydroxyl group, protection of the hydroxyl group before ring opening ("pre-ring opening") is effected. Such pre-ring opening protection is utilized, for example, for the addition of Grignard reagents and other like reagents.

An acid labile protecting group such as a member of the trityl family preferably can be used for protection of the primary hydroxyl group, whether such protection is effected before or after ring opening. The trityl family includes at least trityl, monomethoxytrityl, dimethoxytrityl and trimethoxytrityl. The dimethoxytrityl group is preferred and can be added by reacting the primary hydroxyl group with 4,4'-dimethoxytrityl chloride. Other hydroxyl protecting groups can be used, such as those described by Beaucage, *et al., Tetrahedron* **1992,** *48*, 2223.

In other aspects of the present invention the use of acid labile groups which are stable to the trichloroacetic acid treatment used for DMT removal such as BOC-type protecting groups are used. They are stable to extended TCA treatment, but are removed by trifluoroacetic acid solutions (e.g. 5% in CH₂Cl₂). Another protecting group class which is compatible to this methodology is the Allyl class. These groups are cleaved using transition metal catalysts. This type of protecting group is particularly valuable in cases where the selective deprotection of a particular functional group is desired while the oligomer is still attached to the solid support, allowing a new reactive site to be uncovered. Additional protecting group tactics are possible: e.g. photolabile protecting groups are also compatible with this methodology.

Following protection of the primary hydroxyl group and ring opening, in either order, the secondary hydroxyl group is converted to an activated phosphorus moiety, preferably an activated phosphite moiety. Phosphitylation can be effected with a suitable reagent such as chloro-β-cyanoethoxy-N,N-diisopropylaminophosphine. Phosphitylation yields a protected monomer that bears either an activated phosphite moiety or an activated phosphate moiety and is suitable for incorporation in to phosphate linked oligomeric compounds of the invention.

Oligomeric compounds of the invention can be synthesized by the methods illustrated in Figures 1-4. The depicted synthetic strategy places an emphasis on the ease with which widely different functional groups can be incorporated onto a rigid intermediate. The monomers, as activated phosphates or activated phosphites, are oligomerized either in predetermined sequences using, for example, standard oligonucleotide-type synthetic procedures on a DNA synthesizer or in random sequences using, for example, a combinatorial technique such as the above-described SURF technique.

Monomer units bearing protected or unprotected functional groups can be prepared as described in the examples below. If the functional group is such that it will react with other moieties or reagents during phosphitylation or oligomerization, the functional group can be protected with a protecting group, preferably a base labile protecting group that is removed upon completion of oligomer synthesis.

A first group of preferred monomeric compounds of the invention are prepared by nucleophilic addition of an active nitrogen-containing heterocycle to glycidol. For the purposes of this patent application, nitrogen-containing heterocycles are single or multi-cyclic compounds containing at least one nitrogen atom. The heterocycle can contain heteroatoms other than nitrogen atoms. Such other heteroatoms include but are not limited to oxygen and sulfur. A particularly preferred group of heterocycles are synthetic and natural purine and pyrimidine nucleobases, *e.g.,* adenine, guanine, cytosine, uridine, thymine, xanthine, hypoxanthine, 2-aminoadenine, 6-methyl and other alkyl derivatives of adenine and guanine, 2-propyl and other alkyl derivatives of adenine and guanine, 5-halo uracil and cytosine, 6-aza uracil, cytosine and thymine, 5-uracil (pseudo uracil), 4-thiouracil, 8-halo, amino, thiol, thiolalkyl, hydroxyl and other 8 substituted adenines and guanines, 5-trifluoromethyl and other 5 substituted uracils and cytosines and 7-methylguanine. Other purines and pyrimidines include those disclosed in United States Patent Number 3,687,808, those disclosed in the *Concise Encyclopedia Of Polymer Science And Engineering,* J.I. Kroschwitz, Ed. John Wiley & Sons, **1990** at pages 858-859 and those disclosed by Englisch, *et al., Angewandte Chemie*, *International Edition* **1991**, *30*, 613.

Such nitrogen-containing heterocycles can be added to either glycidol or R-(+)-glycidol in a suitable solvent in the presence of the catalyst such as potassium carbonate, as illustrated in Figure 2. Where necessary, exocyclic functional groups on the nitrogen heterocycles are protected via transient protection. Particularly useful transient protecting groups for nucleobase exocyclic functional groups are chlorotrimethylsilane, benzoyl chloride, and isobutyryl chloride. The protected compound is then tritylated with 4,4'-dimethoxytrityl chloride in pyridine to protect its primary hydroxyl group. Phosphitylation with chloro-β-cyano-ethoxy-N,N-diisopropylaminophosphine and diisopropylethylamine in tetrahdrofuran (THF) yields the corresponding phosphoramidite monomer, which can be incorporated into an oligomeric compound of the invention.

Other nitrogen-containing heterocycles that can be used as functional groups include imidazole, pyrrole, imidazolyl, pyrazole, indole, 1H-indazole, β-carboline, carbazole, phenothiazine, and phenoxazine. A more preferred groups of the nitrogen heterocycle includes imidazole, pyrrole or carbazole. Imidazole is especially preferred.

In preparing monomeric compounds including such heterocycles, protection of exocyclic functional groups with base labile protecting groups is effected as with the nucleobases noted above, the resulting compound is tritylated with 4, 4'-dimethoxytrityl chloride, and phosphitylated with chloro-β-cyanoethoxy-N,N-diisopropylaminophosphine to give the desired monomeric compound.

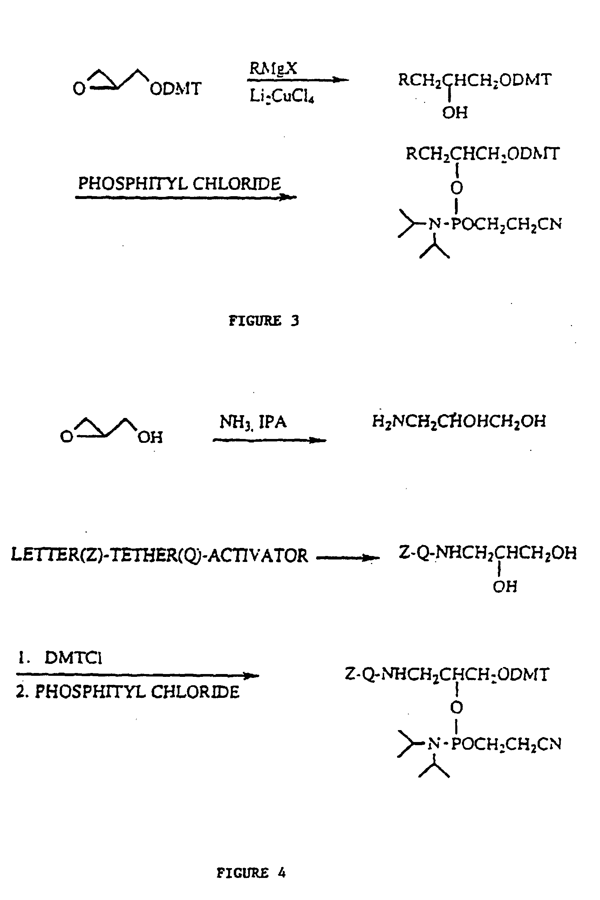

Further preferred monomeric compounds of the invention can be prepared by addition of alkyl, alkenyl, or aryl organometallic reagents to an epoxide ring-containing starting material, *e.g.,* to the 2,3-epoxy ring of glycidol. This is illustrated in Figure 3. For preparation of such compounds, the primary hydroxy group of the starting material (*e.g.*, the methylhydroxyl group of glycidol) is protected with an acid labile protecting group prior to opening the epoxide ring. As above, suitable as a protecting group is one of the trityl family of protecting groups. Thus, for example, addition of an organomagnesium reagent to 1-O-dimethoxytrityl glycidol can be effected in the presence of dilithium tetrachlorocuprate in a suitable solvent. Phosphitylation using chloro-β-cyanoethoxy-N,N-diisopropylaminophosphine then yields the monomeric compound as the phosphoramidite.

Still further preferred monomeric compounds of the invention can be prepared by nucleophilic addition of ammonia to glycidol. This is illustrated in Figure 4. The product of this reaction, 1-amino-2,3-propandiol, can be protected with benzoylchloride and the protected compound converted to the DMT-phosphoramidite for incorporation into an oligomer. Alternatively, the amino group of 1-amino-2,3-propandiol can be further functionalized. Functional groups can be attached to the nitrogen directly or via a tether, either by alkylation or acylation. Following functionalization, protection with 4,4'-dimethoxytrityl chloride and phosphitylation with chloro-β-cyanoethoxy-N,N-diisopropylaminophosphine is effected to give the phosphoramidite monomer for inclusion into an oligomeric compound of the invention.

In using the amino group of the above noted 1-amino alkane diol compound as a point for connecting a tether to the alkane diol, the amino group can be reacted either by acylation or alkylation. In one such reaction, the amino group is reacted with a bifunctional linking group, that is, a compound having a first reactive group for linking to the alkane glycol monomeric compound and a second reactive group for linking to the functional group that is being attached to the alkane glycol monomeric compound. One group of bifunctional linking groups or tethers that are particularly preferred are the cyclic anhydrides, including succinic anhydride, maleic anhydride, glutaric anhydride. Thus, for example, the above-noted 1-amino-2,3-propandiol can be reacted with succinic anhydride to form an amide linkage between the propane diol and the succinyl tether group. The other end of the succinyl tether then can be functionalized by, for example, the formation of an ester or amide link with a moiety bearing the desired functional group. This is followed by reaction with 4,4'-dimethoxytrityl chloride and then chloro-β-cyanoethoxy-N,N-diisopropylaminophosphine to give the phosphoramidite monomer for inclusion into oligomeric compounds of the invention.

Other acyl groups can be used in addition to an amide acyl group to link a tether to the above-described 1-amino-2,3-propandiol. Upon acylation of the 1-amino group, such other groups form carbamoyl, thiocarbamoyl, ureido, thioureido, and sulfonamido linkages. Carbamates and thiocarbamates are formed by the reaction of an appropriate chloroformate or thiochloroformate compound with the amino group of the 1-amino-2,3-propandiol. Ureas are formed by reaction of amino-2,3-propandiol with isocynates or thioisocyanates, and sulfonamides are formed by reaction of 1-amino-2,3-propandiol with a sulfonyl chloride. The other end of the tether (*i*.*e*., the one not used in linking the tether to amine) is selected so as to be reactive with the functional group that is being attached to the monomeric unit. Acyl linkages, including esters, amides, carbamates, ureas and the like, are also useful for this attachment.

Another group of particularly useful bifunctional linking moieties or tethers are certain heterobifunctional or homobifunctional linkers available from Pierce, Rockford Il. These include various di-imidates, N-hydroxysuccinimide esters and sulfo-N-hydroxysuccinimide esters. Representative di-imidates include dimethyl adipimidate, dimethyl pimelimidate, dimethyl suberimidate and the like. Representative N-hydroxysuccinimide esters and sulfo-N-hydroxysuccinimide esters include disuccinimidyl suberate, bis(sulfosuccinimidyl) suberate and the like.

A further preferred group of tether compounds include alkyl, alkenyl and aryl compounds. These can be used to alkylate the amino group of 1-amino alkane diol compounds. Alternatively, they can be added to alkane diol compounds directly, e.g, by addition of alkyl, alkenyl or aryl organometallic reagents to the epoxide ring of a suitable starting material. The tether, in turn, can be further functionalized with a functional group.

Preferred functional groups are: alkyl, alkenyl, and alkynyl groups that are optionally substituted with one or more halogens, OR₁, SR₁, NR₁R₂, C(=NH)NR₁R₂, NHC(=NH)NR₁R₂, CH=O, C(=O)OH, C(=O)NR₁R₂, CH(NH₂) (C(=O)OH); aryl and aralkyl groups that are optionally substituted with one or more halogens, OH, SH, SCH₃, NR₁R₂; adamantyl; NR₁R₂; heterocycle; purine; pyrimidine; phosphate; polyether; polyethylene glycol or metal coordination groups.

Alkyl, alkenyl, and alkynyl groups according to the invention include but are not limited to substituted and unsubstituted straight chain, branch chain, and alicyclic hydrocarbons, including methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl and other higher carbon alkyl groups. Further examples include 2-methylpropyl, 2-methyl-4-ethylbutyl, 2,4-diethylpropyl, 3-propylbutyl, 2,8-dibutyldecyl, 6,6-dimethyloctyl, 6-propyl-6-butyloctyl, 2-methylbutyl, 2-methylpentyl, 3-methylpentyl, 2-ethylhexyl and other branched chain groups, allyl, crotyl, propargyl, 2-pentenyl and other unsaturated groups, cyclohexane, cyclopentane, adamantane as well as other alicyclic groups, 3-penten-2-one, 3-methyl-2-butanol, 2-cyanooctyl, 3-methoxy-4-heptanal, 3-nitrobutyl, 4-isopropoxydodecyl, 4-azido-2-nitrodecyl, 5-mercaptononyl, 4-amino-1-pentenyl as well as other substituted groups.

Aryl groups according to the invention include but are not limited to substituted and unsubstituted aromatic hydrocarbyl groups such as phenyl and naphthyl groups. Aralkyl groups include but are not limited to groups having both aryl and alkyl functionality, such as benzyl and xylyl groups.

Metal coordination groups according to the invention include but are not limited to hydroxamic acids, catecholamide, acetylacetone, 2,2'-bipyridine, 1,10-phenanthroline, diacetic acid, pyridine-2-carboxamide, isoalkyldiamine, thiocarbamato, oxalate, glycl, histidyl and terpyridyl. Other metal coordination groups are known, as for example see Mellor, D.P., *Chemistry of Chelation and Chelating Agents* in *International Encyclopedia of Pharmacology and Therapeutics,* Section 70, The Chelation of Heavy Metals, Levine, W.G. Ed., Pergamon Press, Elmford, N.Y., **1979.**

Solid supports according to the invention include controlled pore glass (CPG), oxalyl-controlled pore glass *(see, e.g.,* Alul, *et al., Nucleic Acids Research* **1991**, *19*, 1527), TentaGel Support -- an aminopolyethyleneglycol derivatized support *(see, e.g.,* Wright, *et al., Tetrahedron Letters* **1993,** *34*, 3373) or Poros -- a copolymer of polystyrene/divinylbenzene.

A number of substituent groups can be introduced into compounds of the invention in a protected (blocked) form and subsequently de-protected to form a final, desired compound. In general, protecting groups render chemical functionality inert to specific reaction conditions and can be appended to and removed from such functionality in a molecule without substantially damaging the remainder of the molecule. *See, e.g.*, Green and Wuts, Protective Groups in Organic Synthesis, 2d edition, John Wiley & Sons, New York, 1991. For example, amino groups can be protected as phthalimido groups or as 9-fluorenylmethoxycarbonyl (FMOC) groups and carboxyl groups can be protected as fluorenylmethyl groups. Representative hydroxyl protecting groups are described by Beaucage, *et al*., *Tetrahedron* **1992,** *48,* 2223.

Substituent groups according to the invention include but are not limited to halogen (Cl, Br, F), hydroxyl (OH), thiol (SH), keto (C=O), carboxyl (COOH), ethers, thioethers, amidine (C(=NH)NR₃R₄, guanidine (NHC(=NH)NR₃R₄, glutamyl CH(NR₃R₄) (C(=O)OR₅), nitrate (ONO₂), nitro (NO₂), nitrile (CN), trifluoromethyl (CF₃), trifluoromethoxy (OCF₃), O-alkyl, S-alkyl, NH-alkyl, N-dialkyl, O-aralkyl, S-aralkyl, NH-aralkyl, amino (NH₂), azido (N₃), hydrazino (NHNH₂), hydroxylamino (ONH₂), sulfoxide (SO), sulfone (SO₂), sulfide (S-), disulfide (S-S), silyl, heterocyclic, alicyclic and carbocyclic. Preferred substituents include halogens, alcohols and ethers (OR₁), thiols and thioethers (SR₂), amines (NR₃R₄), amidines [C(=NH)NR₃R₄], guanidines [NHC(=NH)NR₃R₄], aldehydes (CH=O), acids [C(=O)OH], esters [C(=O)OR₅], amides [C(=O)NR₃R_{4]} and glycine [CH(NH₂) (C(=O)OH)].

Particularly preferred functional groups according to the invention include monomeric units of at least the following specific functional moieties: thymine, uracil, adenine, guanine, cytosine imidazole, carbazole, aminoethyl, carboxyethyl, phenyl, short and long chain alkyls, and glycine. Other preferred functional groups include amino, benzyl, and tetraethylene glycol groups.

The compounds of the invention can include conjugate groups covalently bound to primary or secondary hydroxyl groups. Conjugate groups of the invention include inter-calators, reporter molecules, polyamines, polyamides, polyethylene glycols, polyethers, groups that enhance the pharmacodynamic properties of oligomers, and groups that enhance the pharmacokinetic properties of oligomers. Typical conjugates groups include cholesterols, phospholipids, biotin, phenanthroline, phenazine, phenanthridine, anthraquinone, acridine, fluoresceins, rhodamines, coumarins, and dyes. Groups that enhance the pharmacodynamic properties, in the context of this invention, include groups that improve oligomer uptake, enhance oligomer resistance to degradation, and/or strengthen sequence-specific hybridization with RNA. Groups that enhance the pharmacokinetic properties, in the context of this invention include groups that improve oligomer uptake, distribution, metabolism or excretion. Representative conjugate groups are disclosed in WO-A-9307883 and US-A-5218105.

Monomeric compounds of the invention can be used to prepare oligomeric compounds having either preselected sequences or sequences determined via combinatorial strategies. One useful combinatorial strategy is the above-noted SURF strategy, which is disclosed and claimed in WO-A-9304204.

To synthesize a combinatorial library having a large degree of chemical diversity is an important aspect of the present invention. Chemical diversity is introduced at one level by varying the nature of the phosphorus linkage. Phosphorus linkages amenable to the present invention include phosphodiester (OPO), phosphorothioate (OPS), phosphoramidate (OPN), phosphorothioamidate (SPN), and alkylphosphonate (OPC). The combinatorial library can be prepared with a single type of phosphorus linkage, or with different linkages at each position of the oligomer. For example, a single OPS linkage can be selectively introduced at any position in a OPO oligomer. In fact, all possible combinations of OPO, OPS, OPN, SPN and OPC linkages can be introduced selectively into the oligomers. The presence or absence of a type of linkage at a particular position in an oligomer may have a profound effect on the properties of the molecule.

In the case of phosphoramidate linked libraries, a further level of diversity is possible due to the ability to have substituents on the nitrogen atom of the OPN linkage. Thus it is possible to introduce a wide variety of amines each time a OPN linkage is present in an oligomer. It is possible to have the same amine substituents at each OPN linkage or a different amine at each position.

As was discussed earlier, chemical diversity can be generated at several levels in SURF libraries. We have described above the preparation of a large number of monomers. These monomers have been prepared to explore two aspects of chemical diversity: first a wide number of functional groups are available, covering a range of chemical properties. Second, these functional groups are attached to different linker portions designed to display, or present them in space in different ways, allowing variable flexibility. The following section describes a third level of diversity, the inter-residue linkage itself. It is to be understood that the methods used to introduce any of the linkage types are compatible. Specifically, by using the proper conditions, it is possible to introduce phosphate linkages including OPO, OPS, OPN, SPN and OPC linkages at will at any position in an oligomer, independently of the sequence of the oligomer. This is possible because the chemical reaction which defines the nature of the linkage is separate from the attachment of the monomer to the reactive group on the solid support (whether linker or the previous monomer). Specifically, when a phosphoramidite monomer is treated with tetrazole and added to the solid support which has a free hydroxyl group, a phosphite triester is obtained. This phosphite triester can then be treated with Iodine (formulated in the same way as for DNA synthesis on an automated synthesizer), giving a phosphodiester after ammonia deprotection. Alternatively, the phosphite triester can be treated with benzodithiole-3-one-1,1-dioxide to give the corresponding phosphorothioate.

Hydrogen phosphonate chemistry has the advantage of allowing additional chemical modifications to be introduced into oligomers. Oligonucleotide phosphodiesters and phosphorothioates have been prepared using this approach,(see Froehler, B. C., Matteucci, M. D. *Tetrahedron Lett.* **1986**, *27,* 469-472), as well as oligonucleotide phosphoramidates (see Froehler, B. C. *Tetrahedron Lett.* **1986,** *27*, 5575-5579. Letsinger, R. L., Singman, C. N., Histand, G., Salunkhe, M. *J. Am. Chem. Soc*. **1988**, *110,* 4470-4471. The synthesis of oligomers containing both phosphodiesters and phosphoramidates was reported, as well as the use of phosphoramidite chemistry in conjunction with the synthesis of phosphoramidates (see Jung, P. M., Histand, G., Letsinger, R. L. *Nucleosides & Nucleotides,* **1994,** *13,* 1597-1605). In this latter work, alternating phosphodiester and phosphoramidate oligomers were prepared by coupling phosphoramidites and H-phosphonates to the growing oligomer, followed by the appropriate oxidation step. In general, however, all the examples described thus far have incorporated the same amine substitution at all phosphoramidate linkages in the oligomer. These studies have shown the feasibility of using the phosphoramidate bond as an additional site for the incorporation of diverse functional groups. A wide variety of amines can be used in the oxidative step, and the monomers of the present invention support the necessary chemistry. Thus, for the preparation of combinatorial libraries incorporating phosphoramidate linkages, the monomers of the present invention are converted to the corresponding H-phosphonate monoesters. In one aspect of the present invention this is accomplished using PCl₃ and imidazole as the phosphitylating reagent (see Garegg, P. J., Regberg, T., Stawinski, J., Strömberg, R. *Chem. Scr.* **1986,** *26*, 59-62). These H-phosphonates monomers are oligomerized on solid support by activation with pivaloyl chloride, adamantoyl chloride or other appropriate activating agent. The intermediate H-phosphonate diesters are oxidized to the phosphate diesters in high yields using iodine in aqueous pyridine. This allowed for the comparison of the coupling efficiency of the H-phosphonate and phosphoramidite methods. Essentially the same coupling efficiency is achieved with both methodologies. The H-phosphonate diesters are converted to phosphoramidates by the use of a 10% solution of the appropriate amine in pyridine/CCl₄ (1:1). Under these conditions, a H-phosphonate diester is oxidized to a phosphoryl chloride via an Arbuzov reaction, followed by displacement of the chloride by a primary or secondary amine. The second step has proven to be quite general, with a wide variety of amines giving satisfactory yields. Moreover, the yield of phosphoramidate is comparable to the yield of phosphodiester.

Several types of libraries are available through this methodology. The simplest kind is a library made from a set of monomers of the present invention (a set of 4 to 16 or more monomers is typically used) of 2 to 10 or more monomer units in length, which is substituted at phosphorus with a single amine. These libraries are prepared by split bead synthesis, following the H-phosphonate synthesis protocol rather than phosphoramidite chemistry. The intermediate H-phosphonate diesters are left intact until the final step. At that point the oligomer library pools are oxidized with CCl₄/Pyridine containing 10% of the appropriate primary or secondary amine. This has the result of converting all the interresidue linkages to phosphoramidates. The library therefore is composed of all possible sequences of the monomers, separated into subsets unique at a fixed position, linked together by a constant phosphoramidate linkage. It should be evident that the final properties of the library will be determined by the choice of amine used in the oxidation step. Thus, water solubility, pharmacokinetics and pharmacodynamics of the library components can be modulated by the choice of amine. It is also possible to prepare oligomer libraries with mixed linkages by having an intermediate oxidation step (see Gryaznov, S. M., Sokolova, N. I. *Tetrahedron Lett.* **1990,** *31,* 3205-3208; Gryaznov, S. M., Potapov, V. K. *Tetrahedron Lett*. **1991,** *32*, 3715-3718; Farooqui, F., Sarin, P. S., Sun, D., Letsinger, R. L. *Bioconjugate Chem.,* **1991,** *2*, 422-426; Iso, Y., Yoneda, F., Ikeda, H., Tanaka, K., Fuji, K. *Tetrahedron Lett.* **1992,** *33*, 503-506). Thus, a portion of the oligomer library is synthesized by H-phosphonate chemistry, which can be oxidized with (R₂NH, CCl₄/Py or S8, CS₂/TEA or H₂O, CCl₄/Py), and a second portion of the library synthesized and oxidized with a second set of reagents. This creates a chimeric library, where a segment of the random oligomers in each subset has a different linkage than the rest of the molecule. By extension of this methodology, it is possible to incorporate a different linkage at each position of the oligomer library by having a different oxidation step after each monomer coupling. The linkage can be combinatorialized by performing a separate oxidation on a portion of the H-phosphonate diester-linked solid support, followed by pooling of the subsets in the same way that the monomer positions are randomized. Thus, each monomer and the linkage between them can be randomized by a split synthesis strategy.

Illustrative of the SURF strategy is a 2'-O-methyl oligonucleotide library (see, Ecker et. al., *ibid*.) shown in Table I, below. Table I describes the selection of a 2'-O-methyl oligonucleotide for binding to an RNA hairpin. The K_{D}'s, *i.e.*, the binding constants, were determined by gel shift. "X" is used to indicate the position being varied and underlining is used to indicate positions that become fixed during successive iterations of the SURF strategy.

**TABLE I**

| K_{D} (mM) | | | | |
|---|---|---|---|---|
| **Subsets** | **X=A** | **X=C** | **X=G** | **X=T** |
| **Round 1** | | | | |
| NNNNXNNNN | 22 | 10 | >100 | >100 |
| **Round 2** | | | | |
| NNNNCNXNN | >10 | 4 | >10 | >10 |
| **Round 3** | | | | |
| NNXNCNCNN | >10 | 0.5 | >10 | >10 |
| **Round 4** | | | | |
| NNCXCNCNN | >10 | 0.15 | >10 | >10 |
| **Round 5** | | | | |
| NNCCCXCNN | 0.08 | >1 | 0.4 | >1 |
| **Round 6** | | | | |
| NNCCCACXN | 0.05 | >0.5 | 0.08 | >0.5 |
| **Round 7** | | | | |
| NXCCCACAN | >0.1 | >0.1 | 0.03 | >0.1 |
| **Round 8** | | | | |
| NGCCCACAX | 0.05 | 0.02 | 0.05 | 0.04 |
| **Round 9** | | | | |
| XGCCCACAC | 0.03 | 0.05 | 0.02 | 0.01 |

This SURF strategy has not been used for libraries except those that employ naturally-occurring nucleotides as phosphodiesters or phosphorothioates as monomeric units. Other combinatorial strategies have only been used for libraries that employ amino acids as monomeric units.

One aspect of the present invention is the inclusion of monomeric compounds having structure I in the above-described SURF strategy. The functional groups appended to these monomeric compounds can be incorporated into the libraries while retaining the advantages of automated phosphoramidite oligomer synthesis. These functional groups can effect interactions of the following types: hydrogen-bond donor and acceptor, ionic, polar, hydrophobic, aromatic, and electron donors and acceptors. Preferred functional groups include aminoethyl, carboxyethyl, adenylmethyl, thyminylmethyl, imidazolylmethyl, benzyl, myristyl, isopropyl, and tetraethylene glycol groups.

One advantage of the present invention is that the simple design of monomeric compounds of the invention allows for combining rational drug design with screen mechanisms for thousands of compounds. This is achieved by using the compounds of the invention in a combinatorial technique such as the SURF strategies.

In one preferred embodiment, functional groups appended to the monomeric compounds of the invention are selected for their potential to interact with, and preferably inhibit, the enzyme PLA₂. Thus, the compounds of the invention can be used for topical and/or systematic treatment of inflammatory diseases including atopic dermatitis and inflammatory bowel disease. In selecting the functional groups, advantage can be taken of PLA₂'s preference for anionic vesicles over zwitterionic vesicles. In selecting the backbone that bears these functional groups, further advantage can be taken of fact that the natural substrate of PLA₂ contains a phosphate group. Therefore, phosphodiester or phosphorothioate and other phosphate linked oligomers preferably are selected, providing a negatively charged compound for binding with the positively charged interfacial binding site of PLA₂.

Certain compounds of the invention include aromatic functional groups to facilitate binding to the cleft of the PLA₂ enzyme. (*see,* Oinuma, *et al., J. Med. Chem.* **1991**, *34*, 2260; Marki, et *al., Agents Actions* **1993**, *38,* 202; and Tanaka, *et al., J. Antibiotics* **1992**, *45*, 1071). Benzyl and 4-hexylbenzyl groups are preferred aromatic groups. The compounds of the invention can further include hydrophobic functional groups such as tetraethylene glycol groups. Since the PLA₂ enzyme has a hydrophobic channel, hydrophobicity is believed to be an important property of inhibitors of the enzyme.

In certain embodiments of the invention, phosphoramidite monomeric compounds having structure I are incorporated into libraries of oligomeric compounds and increasingly less complex subsets of oligomers are identified in combinatorial screening techniques such as the above-described SURF technique by successive rounds of screens. In one preferred embodiment, a library of oligomeric compounds functionalized with aminoethyl, carboxyethyl, adenylmethyl, thyminylmethyl, tetraethylene glycol, imidazolylmethyl, benzyl, isopropyl, myristyl or 4-hexylbenzyl groups are prepared and assayed for inhibition of PLA₂ activity. The PLA₂ assay can be effected using a combinatorial screening strategy such as the SURF strategy. For this assay, the oligomer libraries are screened for inhibition of human type II PLA₂ enzymatic activity. Typically, these libraries contain about 8000 different compounds. Successive iterations of the SURF technique is effected to select unique oligomers from the library. The libraries additionally can be screened in other in vitro assays to determine further mechanisms of inhibition.

Upon identification of oligomers in a first phase of screening, further modifications can be made to the contents of the oligomer libraries. For example, if a first iteration of screening results in an active compound that contains a benzyl group, then in subsequent iterations of the screen this aromatic residue can then be varied using substituted benzyl groups. In this way, structural activity is identified in a stepwise manner to define potent inhibitors of the enzymatic activity.

To maximize the identification of a tight binding oligomeric inhibitor of PLA₂ via a combinatorial approach, an array of functional groups typically are included in a randomized library. The oligomers are assembled in a manner analogous to oligonucleotide synthesis by the coupling of monomeric, phosphoramidate units wherein the normal nucleotide structure is replaced by more diverse chemical groups. In some of the monomeric units, the nucleobases of nucleotides have been retained. In other, the nucleobases are replaced with other functional groups selected to provide different ligand-ligand interactions than that provided by the nucleobases. The sugar moiety of a normal nucleotide is replaced by an alkylene glycol unit, *e.g.*, an ethylene glycol unit, to form a unique backbone. This methodology provides for a convergent preparation of a large number of monomers bearing a wide variety of functional groups. Where necessary, functional groups are protected with base labile protecting groups to allow one-step deprotection of the oligomer upon completion of the synthesis.

As noted above, monomeric compounds having structure I can be linked with one another to form homopolymeric structures or they can be linked with nucleotides and/or other moieties to form heteropolymeric structures. For example, chimeric structures can be formed that include one or more regions or "stretches" of the monomeric units of invention joined to one or more regions or "stretches" of naturally occurring or synthetic oligonucleotides or to other synthetic or natural oligomeric compounds such as peptides, peptoids, peptide nucleic acids, oligo and/or polysaccharides. Further, oligomeric compounds having structure II can be incorporated into chimeric structures along with the compounds disclosed in the patent application entitled "Oligonucleotide Mimics Having Nitrogen-Containing Linkages," bearing attorney docket ISIS-1014, and the patent application entitled "Pyrrolidine-Containing Monomer and Oligomers," bearing attorney docket ISIS-1237. The foregoing patent applications are filed concurrently with this application, are commonly assigned, and are incorporated herein by reference.

In the combinatorial synthesis of oligomeric compounds of the invention, the monomers are incorporated into libraries, including libraries suitable for screening for PLA₂ inhibition. The libraries are further useful for screening against other targets of interest. In non-combinatorial synthesis of oligomeric compounds of the invention, the monomeric units are combined in a predetermined sequences using the standard conditions normal used for oligonucleotide synthesis.

To detect an active sequence generated via a combinatorial technique, the concentration of the active molecule is selected to be of sufficiently great that the molecule can be detected within the sensitivity of the chosen assay. As will be recognized, the number of unique oligomer sequences within a subset produced via a combinatorial technique depends on the length of the oligomer and the number of different monomers employed. The number of sequences can be determined by raising the number of monomers to a power equal to the number of random positions. This is illustrated in Table II. Table II also indicates the concentration of each sequence when the subset concentration is 100 µM, a typical high-test concentration. We have found that the number of monomers and their length can be based upon an estimate of the'expected IC₅₀ (*i.e*., a concentration at which 50% of enzyme activity is inhibited) that is desirable in a final oligomeric compound. For an expected IC₅₀ of 100 nM, the complexities shown in Table II are acceptable, that is, the libraries shown in Table II have complexities that would allow detection of a unique sequence with an IC₅₀ of about 100 nM or less.

**TABLE II**

| **Complexity of Libraries** | | |
|---|---|---|
| **Length** | **Sequences Per Subset** | **nM Each Sequence At 100 µM Subset** |
| **5 Monomers** | | |
| 4-mer | 125 | 800 |
| 5-mer | 625 | 160 |
| **6 Monomers** | | |
| 4-mer | 216 | 463 |
| 5-mer | 1,296 | 77 |
| **7 Monomers** | | |
| 4-mer | 343 | 291 |
| **8 Monomers** | | |
| 4-mer | 512 | 195 |
| **10 Monomers** | | |
| 4-mer | 1,000 | 100 |

If five monomers are selected for a library, then the library will have a length of five monomer units, XNNNN, where N is an equal molar mixture of monomer units and X is a different monomer unit in each of the five subsets. For ease in synthesis, the fixed position can be selected as the right end of the molecule. After assay for inhibition of PLA₂ activity as described below, position X is fixed with the residue giving the greatest inhibition and the next subset is synthesized and screened. The fixed position then shifts towards the left end of the oligomer as unrandomization proceeds. Five rounds of synthesis and screening are required to determine a unique inhibitor.

The monomer units of the invention are linked to form oligomeric compounds using standard phosphoramidite chemistry that is used for standard synthesis of oligonucleotides. Since the coupling rates of functionalized alkylene glycol monomers may vary, the reactivity of the individual monomers can adjusted such that equal molar incorporation of each monomer at each randomized position is effected. Adjusting for the reactivity of the monomers can be effected as in the examples below. A further technique for effecting such adjustment is disclosed in the United States patent application entitled "Random Oligonucleotide Libraries And Methods Of Making The Same," bearing attorney docket ISIS-1009. The foregoing patent application is being filed concurrently with this application, is commonly assigned, and is incorporated herein by reference.

In a SURF screening strategy the amount of oligomer is selected such that the concentration of each subset in the initial round of screening is relatively high (about 100 µM). It is presently preferred to synthesize oligomers using a DNA synthesizer. On such synthesizers the oligomers are most conveniently synthesized on a 1 to 4 µmol scale. Given the concentration of a subset of libraries at about 100 µm, the assays preferably are performed in a small volume of less than about 200 µL.

Exemplary compounds of the invention are illustrated in the following examples, which are not intended to be limiting.

### EXAMPLE 1

### 1-(1-Thymine)-2,3-propandiol

To a stirred solution of thymine (4.2g, 33mmol) in dry dimethylformamide (DMF, 30ml) was added R-(+)-glycidol (2.2g, 30mmol), and potassium carbonate (50 mg, 0.36mmol). The suspension was heated to 80°C for five hours then evaporated. The products were diluted with methanol (25ml) and filtered. The filtrate was evaporated and the residue purified by silica gel column chromatography. Elution with ethyl acetate:methanol (9:1, v/v), pooling of appropriate fractions, and evaporation furnished the N(1) substituted material free from N(1),N(3) disubstituted material to yield 3.08g (60%). ¹H NMR (DMSO-d₆) : δ, 1.8 (s, 3, CH₃); 3.35 (bm, 3, CHOHCH₂OH) ; 3.7 and 3.9 (2 m, 2, NCH₂); 4.7 (bs, 1, CH₂OH, exchanges with D₂O); 5.0 (bs, 1, CHOH, exchanges with D₂O); 7.4 (s, 1, H-6); 11.2 (bs, 1, NH, exchanges with D₂O). Anal. calcd. for C₈H₁₂N₂O₄ (200.193) : 47.99% C, 6.04% H, 13.99% N; found: 47.35% C 6.10% H, 13.67% N.

### EXAMPLE 2

### 1-(1-Thymine)-3-O-dimethoxytrityl-2-propanol

To a stirred solution of 1-(1-thymine)-2,3-propandiol (2.079g, 12.4mmol) in dry pyridine (30ml) was added 4,4'-dimethoxytrityl chloride (4.4g, 13mmol). The suspension was stirred at room temperature for four hours. The reaction mixture was evaporated and the residue purified by silica gel column chromatography. Elution with ethyl acetate:hexane:tri-ethylamine (3/2/1, v/v/%), pooling of appropriate fractions, and evaporation gave a yield of 3.39g (54%). ¹H NMR (DMSO-d₆): δ, 1.8 (s, 3, CH₃); 2.9 (m, 2, CH₂ODMT); 3.8 (s, 6, OCH₃); 3.9 (m, 2, NCH₂); 5.3 (d, 1, OH, exchanges with D₂O); 6.9 (m, 4, trityl); 7.3 (m, 9, trityl); 11.2 (s, 1, NH, exchanges with D₂O).

### EXAMPLE 3

### 1-(1-Thymine)-3-O-dimethoxytrityl-2-O-[(N,N-diisopropylamino)-2-cyanoethoxyphosphite]propane

A stirred solution of 1-(1-thymine)-3-O-dimethoxytrityl-2-propanol (3.39g, 6.7mmol) and N,N-diisopropylethylamine (2.4ml, 14mmol) in dry THF (35ml) was cooled to 10°C in an ice bath. Chloro-β-cyanoethoxy-N,N-diisopropylaminophosphine (1.5ml, 6.7mmol) was added. After stirring at room temperature for four hours the reaction mixture was evaporated and the residue purified by silica gel column chromatography. Elution with ethyl acetate:hexane:triethylamine (1:1:1, v/v/%), pooling of appropriate fractions, and evaporation gave a yield of 2.78g (61%). ¹H NMR (CD₃CN) : δ, 1.2 (m, 12, 6 CH₃) ; 1.75 (d, 3, CH₃) ; 2.45 and 2.6 (2 t, 2, CH₂ODMT) ; 3.8 (d, 6, OCH₃) ; 6.9 and 7.3 (2 m, 14, trityl); 9.3 (bs, 1, NH, exchanges with D₂O). ³¹P NMR (CD₃CN); δ, 150.19 and 150.66. Anal. calcd. for C₃₈H₄₇N₄O₇P (702.786) : 64.94% C, 6.74% H, 7.97% N; found: 64.60% C, 6.91% H, 7.80% N.

### EXAMPLE 4

### 1-(1-Imidazole)-2,3-propandiol.

A rapidly stirred solution of imidazole (13.6 g, 0.2 mole) in DMF (250 mL) was treated with powdered potassium carbonate (12g) and heated to 70°C for 30 min. To this solution was added glycidol (14.8g, 0.2 mole) in one portion and the mixture stirred at this temperature for 36 hr. The resulting yellow suspension was filtered and the filtrate was rotary evaporated to afford a clear red syrup. The syrup was coevaporated with acetonitrile (100 mL) and then flash-chromatographed on a 10.5X10 cm silica gel column. A step gradient elution of ethyl acetate-methanol (9:1, 2L then 4:1, 2L) gave the imidazole product as an amorphous solid, 16g, (56%). ¹H NMR (DMSO-d6) : δ, 7.55, 7.10 and 6.84 (3 s, 3, imidazole); 5.08 (d, 1, CHOH, exchanges with D₂O) ; 4.85 (t, 1, CH₂OH, exchanges with D₂O) ; 4.05 and 3.83 (2 dd, 2, CH₂) ; 3.55 (m, 1, methine); 3.30 and 3.18 (2 m, 2, CH₂OH). Anal. calcd. for C₆H₁₀N₂O₂ (142.157) : 50.69%C, 7.09% H, 19.71% N; found: 50.59% C, 7.07% H, 19.59% N.

### EXAMPLE 5

### 1-(1-Imidazole)-3-O-dimethoxytrityl-2-propanol

To a stirred solution of 1- (1-imidazole) -2,3-propandiol (1.0g, 7.0mmol) in dry pyridine (15ml) and dry DMF (15ml) was added 4,4'-dimethoxytrityl chloride (2.61g, 7.7mmol). The suspension was stirred at room temperature for four hours. An additional equivalent of the trityl compound was added (2.61g, 7.7mmol). Stirring was continued for an additional 23 hours and the mixture was evaporated. The residue was purified by silica gel column chromatography. Elution with methanol:ethyl acetate:triethylamine (1/19/1, v/v/%), pooling of appropriate fractions, and evaporation gave a yield of 432mg (14%). ¹H NMR (DMSO-d₆): δ, 2.78, 2.95 (2 m, 1, CH₂ODMT); 3.7 (s, 6, 2 OCH₃); 3.85(bm, 1, CHOH); 3.95 and 4.1 (2 m, 2, NCH₂); 5.25 (d, 1, OH, exchanges with D₂O); 6.7 to 7.5 (m, 13/3, DMT/imidazole).

### EXAMPLE 6

### 1-(1-Imidazole)-3-O-dimethoxytrityl-2-O-[(N,N-diisopropylamino)-2-cyanoethoxyphosphite]propane

A solution of 1-(1-imidazole)-3-O-dimethoxytrityl-2-propanol (432mg, 0.97mmol) and N-N-diisopropylethylamine (252mg, 3.5mmol) in dry DMF (35ml) was cooled to 5°C in an ice bath. Chloro-β-cyanoethoxy-N,N-diisopropylaminophosphine (233mg, 1.1mmol) was added and the reaction mixture was stirred at room temperature for one half hour. The reaction mixture was evaporated and the residue purified by silica gel column chromatography. Elution with ethyl acetate:hexane:tri-ethylamine (7/3/1, v/v/%), pooling of appropriate fractions, and evaporation gave a yield of 498mg (80%). ¹H NMR (CD₃CN); δ, 0.9 to 1.2 (m, 12, CH₃); 3.8 (d, 6, OCH₃); 6.75 to 7.5 (M, 13/3, ODMT/imidazole). ³¹P NMR (CD₃CN); δ, 149.83 and 150.68.

### EXAMPLE 7

### 1-(1-Carbazole)-2,3-propandiol

A rapidly stirred solution of carbazole (14g, 83 mmole) and R-(+)-glycidol (6.20g/83mmol) in anhydrous DMF (300 mL) was treated with powdered potassium carbonate (2.3 g) and the mixture heated to 70°C for 18 hr. The resulting yellow suspension was filtered and then rotary evaporated to afford a yellow syrup. The syrup was coevaporated with acetonitrile (100 mL) and then flash-chromatographed on a 10.5 X 10 cm silica gel column. Elution with ethyl acetate gave the carbazole product as an amorphous solid, 8.25 g, (41%). ¹H NMR (DMSO-d₆): δ, 8.13, 7.60, 7.23 and 7.10 (4 m, 8, carbazole); 5.03 and 4.90 (2 s, 2, hydroxyls, exchange with D₂O); 4.47 and 4.27 (2 dd, 2, CH₂); 3.90 (m, 1, methine); 3.40 (m, 2, CH₂OH). Anal. calcd. for C₁₅H₁₅NO₂ (241.289): 74.66% C, 6.27% H, 5.80% N; found: 74.32% C, 6.25% H, 5.76% N.

### EXAMPLE 8

### 1-(1-Carbazole)-3-O-dimethoxytrityl-2-propanol

To a solution of 1-(1-carbazole)-2,3-propandiol (1.1g, 4.5mmol) in dry pyridine (40ml) was added 4,4'-dimethoxytrityl chloride (1.54g, 4.5mmol). The reaction mixture was stirred at room temperature for three hours and evaporated. The residue was purified by silica gel column chromatography. Elution with ethyl acetate:hexane:triethylamine (1/9/1,v/v/%), pooling of appropriate fractions, and evaporation gave a yield of 976mg (39%). ¹H NMR (DMSO-d₆); δ, 2.9 to 3.1 (2 m, 2, CH₂ODMT); 3.7 (s, 6, 2 OCH₃); 4.1 (m, 1, CHOH); 4.3 to 4.5 (2 m, 2, NCH₂); 5.2 (d, 1, OH, exchanges with D₂O); 6.8 to 8.2 (m, 21, carbazole and trityl). Anal. calcd. for C₃₆H₃₃NO₄ (543.661): 79.53% C, 6.12% H, 2.58% N; found: 79.28% C, 6.34% H, 2.70%N.

### EXAMPLE 9

### 1-(1-Carbazole)-3-O-dimethoxytrityl-2-O-[(N,N-diisopropylamino)-2-cyanoethoxyphosphite]propane

Chloro-β-cyanoethoxy-N,N-diisopropylaminophosphine (403mg, 1.7mmol) was added to a solution of 1-(1-carbazole)-3-O-dimethoxytrityl-2-propanol (916mg, 1.7mmol) and N,N-diiso-propylethylamine (445mg, 3.4mmol) in dry THF (40ml) at 5°C. At four hours the reaction mixture was evaporated and the residue was purified by silica gel column chromatography. Elution with ethyl acetate:hexane:triethylamine (1/19/1, v/v/%), pooling of appropriate fractions, and evaporation gave a yield of 850mg (68%). ¹H NMR (CD₃CN); δ, 0.7 to 1.05 (3 d, 12, CH₃) ; 3.75 (d, 6, OCH₃) ; 6.7 to 8.1 (m, 21, carbazole and trityl). ³¹P NMR (CD₃CN) ; δ, 149.1 and 149.5. Anal. calcd. for C₄₅H₅₀N₃O₅P (743.881): 72.66% C, 6.77% H, 5.65% N; found: 72.62% C, 6.76% H, 5.61% N.

### EXAMPLE 10

### 1-(1-Uracil)-2,3-propandiol

Uracil (21.56g, 192mmol) is dissolved in dry DMF (500ml) with heating and R-(+)-glycidol (15.1g, 203mmol) is added. The suspension is heated to 70°C. At four hours the reaction is evaporated and the residue purified by silica gel column chromatography. Elution with ethyl acetate:methanol (19/1, v/v), pooling of appropriate fractions and evaporation gave a yield of 15.62g (44%). ¹H NMR (DMSO-d₆); δ, 3.35 (m, 3, CHOHCH₂OH); 3.7 and 3.9 (2 m, 2, NCH₂); 5.5 (m, 1, H-5); 7.5 (d, 1, H-6); 11.2 (bs, 1, NH).

### EXAMPLE 11

### 1- (1-Uracil)-3-O-dimethoxytrityl-2-propanol

Dimethoxytritylchloride (29.8g, 88mmol) is added to a solution of 1-(1-uracil)-2,3,-propandiol (15.62g, 84mmol) in dry pyridine (150ml). The reaction mixture is stirred at room temperature for twenty two hours and evaporated. The residue was purified by silica gel column chromatography. Elution with ethyl acetate:hexane:triethylamine (3/2/1, v/v/%), pooling of appropriate fractions, and evaporation gave a yield of 1.1g (4%). ¹H NMR (DMSO-d₆); δ, 2.8 to 3.0 (2 m, 2, CH₂ODMT); 3.7 (s, 6, 2 OCH₃); 3.85 (m, 1, CHOH); 3.95 (m, 2, NCH₂); 5.3 (d, 1, CHOH, exchanges with D₂O); 5.41 (d, 1, H-5); 6.85 (d, 4, trityl); 7.3 and 7.4 (2 m, 10, H-6 and trityl); 11.2 (s, 1, NH, exchanges with D20).

### EXAMPLE 12

### 1-(1-Uracil)-3-O-dimethoxytrityl-2-O-[(N,N-diisopropylamino)-2-cyanoethoxyphosphite]propane

A stirred solution of 1- (1-uracil)-3-O-dimethoxytrityl-2-propanol (598mg, 1.2mmol) and N,N-diisopropylethylamine (158mg, 1.2mmol) in dry THF (35ml) was cooled to 10°C in an ice bath. Chloro-β-cyanoethoxy-N,N-diisopropylaminophosphine (286mg, 1.2mmol) was added. After stirring at room temperature for four hours the reaction mixture is evaporated and the residue purified by silica gel column chromatography. Elution with ethyl acetate:hexane:triethylamine (1:1:1, v/v/%), pooling of appropriate fractions, and evaporation gave a yield of 368mg (42%). ¹H NMR (CD₃CN) ; δ, 1.2 (m, 12, CH₃) ; 2.45 and 2.6 (2 t, 2, CH₂ODMT) ; 3.8 (d, 6, OCH₃) ; 6.9 and 7.3 (2 m, 15, trityl and H-6) ; 9.3 (bs, 1, NH, exchanges with D₂O). ³¹P NMR (CD₃CN) ; δ, 154.88 and 155.2.

### EXAMPLE 13

### 1-[9-(N6-Benzoyl)adenine]-2,3-propandiol.

To a stirred solution of adenine (30.0g, 0.22mol) in dry DMF (500ml) was added R-(+)-glycidol (22.0 ml, 0.33 mol), and potassium carbonate (7.6g, 55mmol). The suspension was heated to 85°C for 20 hours then concentrated. The residue was purified by crystallization from methanol to yield 18.1g (39%) of the intermediate 1-(9-adenine)-2,3-propandiol. ¹H NMR (DMSO-d₆) ; δ, 3.3 to 3.4 (m, 2, CH₂OH) ; 3.8 to 3.9 (m, 1, CHOH) ; 3.9 to 4.1 and 4.3 to 4.4 (2m, 2, NCH₂) ; 4.8 to 4.9 (t, 1, CH₂OH, exchanges with D₂O) ; 5.1 to 5.2 (d, 1, CHOH, exchanges with D₂O) ; 7.3 (s, 2, NH₂, exchanges with D₂O) ; 8.0 and 8.1 (2s, 2, C(2)-H, C(8)-H). Anal. calcd. for C₈H₁₁N₅O₂ (209.207) : 45.93% C, 5.30% H, 33.48% N; found: 45.81% C, 5.42% H, 32.93% N.

To a suspension of 1- (9-adenine)-2,3-propandiol (18.0g, 85.0mmol) at 0°C was added chlorotrimethylsilane (32.7ml, 257mmol). After 15 minutes benzoyl chloride (30.0ml, 259mmol) was added and the mixture was stirred at room temperature for 3 hours. The mixture was cooled to 0°C and 100ml cold water was added. After 15 minutes 100ml cold concentrated ammonium hydroxide was added. After stirring at room temperature for one hour the mixture was evaporated and the residue purified by silica gel column chromatography. Elution with methanol:ethyl acetate (1/10, v/v), pooling of appropriate fractions, and evaporation gave a yield of 4.1g (14.8%). ¹H NMR (DMSO-d₆): δ, 3.3 to 3.5 (m, 2, CH₂OH) ; 3.8 TO 4.0 (m, 1, CHOH) ; 4.1 to 4.2 and 4.4 to 4.5 (2m, 2, NCH₂) ; 4.9 to 5.0 (t, 1, CH₂OH, exchanges with D₂O) ; 5.2 to 5.3 (d, 1, CHOH, exchanges with D₂O) ; 7.4 to 8.2 (2m, 5, benzoyl); 8.4 to 8.8 (2s, 2, C(2)-H, C(8)-H); 11.2 (bs, 1, NH, exchanges with D₂O). Anal. Calc. for C₁₅H₁₅N₅O₃ (313.315) : 57.50% C, 4.82% H, 22.35% N; found: 57.24% C, 4.81% H, 21.97% N.

### EXAMPLE 14

### 1-[9-(N6-Benzoyl)adenine]-3-O-dimethoxytrityl-2-propanol.

To a solution of 1-[9-(N6-benzoyl)adenine]-2,3-propandiol (4.0g, 13mmol) in anhydrous pyridine (50ml) was added 4,4'-dimethoxytrityl chloride (4.7g, 14mmol). The mixture was stirred at room temperature for 4 hours. Methanol was added (5ml) and the mixture was evaporated. The residue was purified by silica gel column chromatography. Elution with hexane/ethyl acetate/triethylamine (2/8/.1, v/v/v), pooling of appropriate fractions and evaporation yielded 4.2g (53%). ¹H NMR (DMSO-d6): δ, 2.9 to 3.1 (2m, 2, CH₂ODMT); 3.7 to 3.8 (s, 6, OCH₃); 4.1 to 4.6 (m, 3, NCH₂CHOH) ; 5.4 to 5.5 (d, 1, CHOH, exchanges with D₂O); 6.8 to 8.2 (4m, 18, trityl, benzoyl); 8.3 to 8.8 (2s, 2, C(2)-H, C(8)-H); 11.2 (bs, 1, NH, exchanges with D₂O).

### EXAMPLE 15

### 1-[9-(N6-Benzoyl)adenine]-3-O-dimethoxytrityl-2-O-[(N,N-diisopropylamino)-2-cyanoethoxyphosphite]propane

To a solution of 1-[9-(N6-benzoyl)adenine]-3-O-dimethoxytrityl-2-propanol (4.2g, 6.8mmol) in anhydrous THF (50ml) was added diisopropylethylamine (2.9ml) and chloro-β-cyanoethoxy-N,N-diisopropylaminophosphine (1.5ml, 6.7mmol). The mixture was stirred at room temperature for 20 hours. The residue was evaporated and purified by silica gel column chromatography. Elution with hexane/ethyl acetate/triethylamine (2/8/.1, v/v/v), pooling of appropriate fractions, and evaporation gave a yield of 4.54g (82%). ¹H NMR (CD₃CN); δ, 0.9-1.1 (m, 12, CH3); 2.4 to 2.6 (m, 2, CH₂CN); 3 to 3.2 (m, 2, CH₂ODMT); 3.4 to 3.7 (m, 3, OCH₂CH₂CN, (CH₃)₂CH) ; 3.7 to 3.8 (d, 6, OCH3) ; 4.3 to 4.6 (m, 3, NCH₂CHOH) ; 6.8 to 7.7 (3m, 18, trityl, benzoyl); 7.9 to 8.8 (2d, 2, C(2)-H, C(8)-H). ³¹P NMR (CD₃CN); δ, 149.80 and 150.75.

### EXAMPLE 16

### 1-[9-(2-Amino-6-chloro)purine]-2,3-propandiol.

To a solution of 2-amino-6-chloropurine (21.3g, 125mmol) in 200ml anhydrous THF was added R-(+)-glycidol (14.0g, 187mmol) and potassium carbonate (3.5g, 25mmol). The reaction was stirred at 85°C for 3 hours. The reaction mixture was filtered. The filtrate was evaporated and the residue purified by silica gel column chromatography. Elution with ethyl acetate/methanol (9/1, v/v) pooling of appropriate fractions and evaporation yielded 11.74g (38%). ¹H NMR (DMSO-d₆); δ, 3.2 to 3.5 (m, 2, CH₂OH); 3.7 to 5.3 (2m, 3, NCH₂CHOH); 4.8 to 4.9 (t, 1, CH₂OH, exchanges with D2O); 5.2 to 5.3 (d, 1, CHOH, exchanges with D2O); 6.8 to 6.9 (s, 2, NH₂, exchanges with D₂O); 8.0 (s, 1, C(8)H). Anal. calcd. for C₈H₁₀N₅O₂Cl (243.652): 39.44% C, 4.14% H, 28.74% N; found: 39.41% C, 4.12% H, 28.45% N.

### EXAMPLE 17

### 1-(9-Guanine)-2,3-propandiol.

1- [9- (2-Amino-6-chloro)purine] -2,3-propandiol (2.62g, 11 mmol) is stirred in 1N HCl (aq, 100ml) at 85°C for 3 hours. The reaction mixture was cooled in an ice bath and rendered basic with concentrated ammonium hydroxide to pH 10. The product was filtered off as a crystalline solid. Yield was 2.04g (84%). ¹H NMR (DMSO-d₆); δ, 3.2 to 3.4 (m, 2, CH₂OH); 3.7 TO 4.2 (M, 3, NCH₂CHOH); 4.7 to 4.9 (t, 1, CH₂OH, exchanges with D₂O); 5.1 to 5.2 (d, 1, CHOH, exchanges with D₂O); 6.6 (s, 2, NH₂, exchanges with D₂O); 7.7 (s, 1, C(8)-H); 10.7 (s, 1, NH, exchanges with D₂O). Anal. calcd. for C₈H₁₁N₅O₃ (225.207): 42.67% C, 4.92% H, 31.10% N; found: 42.28% C, 4.88% H, 30.85%N.

### EXAMPLE 18

### 1-[9-(N2-Isobutyryl)guanine]-2,3-propandiol.

To a solution of 1-(9-guanine)-2,3-propandiol (17.0g, 75.5 mmol) in pyridine (400ml) at 0°C was added chlorotrimethylsilane (28.6ml, 225mmol). The reaction mixture was stirred for 15 minutes at 0°C then for 30 minutes at room temperature. The mixture was recooled to 0°C and isobutyryl chloride (23.7ml, 226mmol) was added. The mixture was stirred for 2 hours at room temperature. The reaction was cooled to 0°C and 100ml ice cold water was added followed in 15 minutes by 100ml ice cold ammonium hydroxide. The reaction was stirred at room temperature for 30 minutes, evaporated to near dryness, methanol added to precipitate unwanted byproducts and filtered. The filtrate was evaporated and the residue purified by silica gel column chromatography. Elution with ethyl acetate/methanol (9/1, v/v), pooling of appropriate fractions, and evaporation gave a yield of 13.8g (62%). ¹H NMR (DMSO-d₆) ; δ, 1 to 1.2 (d, 6, CH₃); 2.7 to 2.9 (m, 1, COCH) ; 3.3 to 3.5 (m, 2, CH₂OH); 3.8 to 3.9 (m, 1, CHOH); 3.9 to 4.3 (2m, 2, NCH₂); 4.8 TO 4.9 (t, 1, CH2OH, exchanges with D₂O); 5.1 to 5.2 (d, 1, CHOH, exchanges with D₂O); 7.9 (s, 1, C(8)-H); 11.6 to 12.2 (2s, 2, NH, CONH, both exchange with D2O) . Anal. calcd. for C₁₂H₁₇N₅O₄ (295.297): 48.81% C, 5.80% H, 23.72% N; found: 48.36% C, 5.62% H, 23.10% N.

### EXAMPLE 19

### 1-[9-(N2-Isobutyryl)guanine-3-O-dimethoxytrityl-2-propanol.

To a suspension of 1- [9-(N2-isobutyryl)guanine]-2,3-propandiol (5.3g, 18mmol) in pyridine (100ml) was added 4,4'-dimethoxytritylchloride (6.69g, 20mmol). The reaction mixture was allowed to stir for 4 hours at room temperature. After quenching with methanol the mixture was evaporated. The residue was purified by silica gel column chromatography. Elution with methanol:ethyl acetate:triethylamine (5/95/1, %/%/%), pooling of appropriate fractions, and evaporation gave a yield of 4.9g (45%). ¹H NMR (DMSO d₆); δ, 1.1 to 1.2 (d, 6, CH₃) ; 2.7 to 3.1 (m, 3, CH₂ODMT, COCH); 3.7 to 3.9 (s, 6, OCH₃) ; 4.1 to 4.3 (m, 3, NCH₂CHOH) ; 5.4 TO 5.5 (d, 1, CHOH, exchanges with D₂O) ; 6.7 to 8.0 (m, 14, trityl, C(8)-H); 11.5 to 12 (2bs, 2, NH, CONH, both exchange with D20).

### EXAMPLE 20

### 1-[9-(N-Isobutyryl)guanine]-3-O-dimethoxytrityl-2-O-[(N,N-diisopropylamino)-2-cyanoethoxyphosphite]propane

To a solution of 1-[9-(N2-isobutyryl)guanine]-3-O-dimethoxytrityl-2-propanol (13.12g, 22mmol) and diisopropylethylamine (10 ml) in THF (200ml), at 0°C, was added chloro-β-cyanoethoxy-N,N-diisopropylaminophosphine (5.6 ml, 25mmol). The reaction mixture was stirred at room temperature for 20 hours. The reaction was concentrated and the residue purified by silica gel column chromatography. Elution with ethyl acetate with 1% triethylamine, pooling of appropriate fractions and evaporation gave a yield of 8.62g (49%). ¹H NMR (CD₃CN) ; δ, 1.0 to 1.2 (m, 18, CH₃) ; 2.4 to 2.8 (m, 2, CH₂CN) ; 2.8 to 3.0 (m, 1, COCH); 3.1 to 3.3 (m, 2, CH₂ODMT) ; 3.4 to 3.8 (m, 9, OCH₂CH₂CN, (CH₃)₂CH, OCH₃) ; 4.2 to 4.4 (m, 3, NCH₂CHO) ; 6.7 to 7.7 (m, 14, trityl, C(8) -H); 12.3 (BS, 2, NH, CONH, both exchange with D20). ³¹P NMR (CD₃CN) ; d, 150.15 and 150.48.

### EXAMPLE 21

### 1-(1-Cytosine)-2,3-propandiol

A solution of cytosine (11.0g, 99mmol), R-(+)-glycidol (8.9g, 120mmol), and potassium, carbonate (6.9g, 50mmol) was stirred in DMF (300ml) at 85°C for 22 hours. The crude reaction mixture was filtered and the filtrate evaporated. This residue was crystallized from methanol to give a yield of 10.5g (57%). ¹H NMR (DMSO d₆); δ, 3.2 to 3.5 (m, 3, CHOHCH₂OH); 3.6 to 3.7 (m, 1, NCH₂); 3.8 to 4.0 (dd, 1, NCH₂); 4.7 to 4.8 (t, 1, CH₂OH, exchanges with D₂O); 4.9 to 5.1 (d, 1, CHOH, exchanges with D₂O); 5.6 to 5.7 (d, 1, C(5)-H); 7.0 to 7.1 (bs, 2, NH₂, exchanges with D₂O); 7.4 to 7.5 (d, 1, C(6)-H).

### EXAMPLE 22

### 1-[1-(N4-Benzoyl)cytosine]-2,3-propandiol

To an ice cold suspension of 1-(1-cytosine)-2,3-propandiol (11.11g, 60mmol) in pyridine (400ml) was added chlorotrimethylsilane. This mixture was stirred for 30 minutes at ice bath temperature. Benzoyl chloride was added and the mixture was stirred at room temperature for 2 hours. The mixture was cooled to 0°C and ice cold water (100ml) was added. After stirring for 15 minutes ice cold ammonium hydroxide (100ml) was added. The reaction mixture was stirred for 15 minutes and concentrate. Water (400ml) was added and the mixture was heated to almost boiling. The desired product was filtered as a solid. After drying the yield was 15.31g (88%). ¹H NMR (DMSO d6) ; δ, 3.3 to 3.6 (m, 3, CHOHCH₂OH) ; 3.7 to 3.9 (bs, 1, NCH₂); 4.1 to 4.3 (d, 1, NCH₂); 4.7 to 4.9 (t, 1, CH₂OH, exchanges with D₂O); 5.1 (d, 1, CHOH, exchanges with D₂O); 7.2 to 7.3 (d, 1, C(5)-H); 7.5 to 7.7 (m, 3, benzoyl); 8.0 to 8.1 (d, 3, C(6)-H, benzoyl) ; 10.5 to 11 (bs, 1, NH, exchanges with D₂O).

### EXAMPLE 23

### 1-[1-(N4-Benzoyl)cytosine]-3-O-dimethoxytrityl-2-propanol.

A solution of 1-[1(N4-benzoyl)cytosine]-2,3-propandiol (7.0g, 24mmol) and 4,4'-dimethoxytrityl chloride (9.84, 29mmol) in pyridine (100ml) was stirred at room temperature for 24 hours. The reaction mixture was filtered and the filtrate is evaporated. The residue was purified by silica gel column chromatography. Elution with ethyl acetate:hexane:triethyl amine (6/4/1, v/v/%), pooling of appropriate fractions and evaporation gave a yield of (52%). ¹H NMR (DMSO d₆); δ, 2.9 to 3.1 (m, 2, CH₂ODMT); 3.6 to 3.9 (m, 7, OCH₃, CHOH); 4 to 4.3 (2m, 2, NCH₂); 5.3 to 5.4 (d, 1, OH, exchanges with D₂O) ; 6.8 to 8.2 (3m, 20, trityl, benzoyl, C(5)-H, C(6)-H); 11.2 (s, 1, NH, exchanges with D₂O).

### EXAMPLE 24

### 1-[1-(N4-Benzoyl)cytosine]-3-O-dimethoxytrityl-2-O-[(N,N-diisopropylamino)-2-cyanoethoxyphosphite]propane

To a solution of 1-[1-(N4-benzoyl)cytosine]-3-O-dimethoxytrityl-2-propanol (7.48g, 13mmol) and diisopropylethylamine (5.5ml) in THF (50ml) was added chloro-β-cyanoethoxy-N-N-diisopropylaminophosphine (3.2ml, 14mmol). The mixture was stirred at room temperature for 2 hours. The mixture was filtered and the filtrate was purified by silica gel column chromatography. Elution with ethyl acetate:hexane:triethylamine (1/1/1, v/v/%), pooling of appropriate fractions and evaporation gave a yield of 5.7g (57%). ¹H NMR (CD₃CN); δ, 1 to 1.2 (m, 12, CH₃); 2.4 to 2.7 (2t, 2, CH₂CN); 3.1 TO 3.3 (m, 2, CH₂ODMT); 3.5 to 4.0 (m, 11, OCH₂CH₂CN, CH(CH₃)₂, OCH₃, NCH₂CH); 4.1 to 4.4 (m, 2; NCH₂); 6.6 to 8.0 (m, 20, trityl, benzoyl, C(5)-H, C(6)-H). ³¹P NMR (CD₃CN); δ, 154.5 and 155.1.

### EXAMPLE 25

### 1-O-Dimethoxytritylglycidol.

Glycidol (4.4ml, 67mmol) and triethylamine (19ml, 136mmol) was stirred in 120ml anhydrous dichloromethane. Dimethoxytritylchloride (22.7g, 67mmol) was added and the mixture was stirred at room temperature for 8 hours. The mixture was filtered and the filtrate evaporated. The residue was dissolved in dichloromethane and washed with water and then brine. Coarse silica gel was added and the material was evaporated. This material was purified by silica gel column chromatography. Elution with hexane:ethyl acetate:triethylamine (19/1/1, v/v/%), pooling of appropriate fractions, and evaporation gave a yield of 16.6g (65%). ¹H NMR (DMSO d₆); δ, 2.6 to 2.9 (2t, 2, ring CH₂); 3.1 to 3.2 (m, 1, CH); 3.2 to 3.4 (2m, 2, CH₂); 3.7 (s, 1, OCH₃); 6.8 TO 7.4 (3m, 13, trityl).

### EXAMPLE 26

### 1-O-Dimethoxytrityl-4-phenyl-2-butanol

Benzylmagnesiumchloride (55ml, 2M solution, 110mmol) was cooled to -78°C and added to a solution of dilithiumtetrachlorocuprate (11ml, 0.1M solution, 12mmol) in THF (200ml) at -78°C. To this mixture was added 1-O-dimethoxytrityl glycidol (8.28g, 22mmol) in THF (20ml) at -78°C. The mixture was stirred at room temperature for 18 hours. The mixture was cooled to 0°C and concentrated ammonium chloride (10ml) was added slowly. The mixture was diluted with water and extracted with-ethyl acetate. The ethyl acetate extracts were dried over magnesium sulfate, filtered, and concentrated. The residue was purified by silica gel column chromatography. Elution with ethyl acetate:hexane (1:9, v/v), pooling of appropriate fractions, and concentration gave a yield of 1.37g (17%). ¹H NMR (CDCL₃); δ, 1.6 to 1.8 (m, 2, C₆H₅CH₂) ; 2.3 to 2.4 (d, 1, OH, exchanges with D₂O) ; 2.5 to 2.8 (m, 2, CH₂CHOH) ; 3.0 to 3.2 (m, 2, CH₂ODMT) ; 3.8 (S, 6, OCH₃); 6.8 TO 7.5 (m, 18, trityl and phenyl).

### EXAMPLE 27

### 1-O-Dimethoxytrityl-2-O-[(N,N-diisopropylamino)-2-cyanoethoxyphosphite]-4-phenylbutane

1-O-Dimethoxytrityl-4-phenyl-2-butanol (1.37g,2.9mmol) and diisopropylethylamine (1.5ml, 8.6mmol) were stirred in THF (35ml) and chloro-β-cyanoethoxy-N,N-diisopropylaminophosphine (0.65 ml, 2.9 mmol) was added. The mixture was stirred at room temperature for 10 hours and filtered. The material was concentrated onto silica coarse gel and dried. The material was purified by silica gel column chromatography. Elution with ethyl acetate:hexane:triethylamine (1:9:1, v/v/%), pooling of appropriate fractions, and evaporation gave a yield of 0.94g (49%). ¹H NMR (CDCL₃) ; δ, 1.0 to 1.3 (m, 12, CH₃); 1.8 to 2.1 (m, 2, C₆H₅CH₂) ; 2.3 to 2.8 (2m, 4, CH₂CN, C₆H₅CH₂CH₂); 3.0 to 3.3 (2m, 2, CH₂ODMT) ; 3.5 to 3.9 (m, 10, CH(CH₃)₂, POCH₂, OCH₃); 3.9 to 4.2 (m, 1, CH₂CH) ; 6.8 to 7.5 (3m, 18, phenyl and trityl). ³¹P NMR (CDCL₃) ; δ, 148.48 and 148.69.

### EXAMPLE 28

### 1-Dimethoxytrityl-2-undecanol

Octylmagnesium chloride (9.4ml, 2M solution, 18.8 mmol), was added to a solution of dilithium tetrachlorocuprate (1.9ml, 0.1M solution, 0.11mmol) in anhydrous THF (15ml) at - 78°C. 1-O-Dimethoxytrityl glycidol was added and the mixture was allowed to warm to room temperature and stir for 2 hours. The mixture was cooled to 0°C and saturated ammonium chloride was added. Water was added and the mixture was extracted with ethyl acetate. The ethyl acetate extracts were combined and dried over magnesium sulfate. This material was filtered and concentrated. The residue was purified by silica gel column chromatography. Elution with ethyl acetate/hexane/tri-ethylamine (5/95/1, v/v/%), pooling of appropriate fractions, and evaporation gave a yield of 1.1g (60%). ¹H NMR (CDCL₃); δ, 0.8 to 0.9 (t, 3, CH₃); 1.2 to 1.4 (m, 16, (CH₂)₇CH₂); 2.3 TO 2.4 (d, 1, OH, exchanges with D₂O); 3.0 to 3.2 (m, 2, CH₂ODMT); 3.7 to 3.9 (s, 7, OCH₃, CHOH) ; 6.8 to 7.5 (m, 14, DMT).

### EXAMPLE 29

### 1-Dimethoxytrityl-2-O-[(N,N-diisopropylamino)-2-cyanoethoxyphosphite]undecane

A mixture of HPLC purified 1-dimethoxytrityl-2-undecanol (1.06g, 2.2mmol), chloro-β-cyanoethoxy-N,N-diisopropylaminophosphine (0.5ml, 2.2mmol), and triethylamine (1.0ml, 5.7mmol) in THF (50ml) was stirred for 20 hours at room temperature. The mixture was concentrated and purified by silica gel column chromatography. Elution with ethyl acetate/hexane/triethylamine (1/9/1, v/v/%), pooling of appropriate fractions, and evaporation gives the title compound.

### EXAMPLE 30

### Fluorenyl succinic acid half ester

To a solution of 9-fluorenylmethanol (3.92g, 20mmol), succinic anhydride (2.20g, 22mmol), and 4-dimethylaminopyridine (0.25g, 2mmol) in dichloromethane (25ml) was added triethylamine (3ml). The mixture was stirred at room temperature for four hours. The reaction was diluted with dichloromethane (50 ml) and extracted with saturated sodium bicarbonate (3x50ml). The combined extracts were back extracted with ethyl acetate. The resulting sodium bicarbonate solution was acidified with 3N HCl and refrigerated. A precipitate fell out of the solution which yielded 3.05g (56%) of the title compound after drying.

### EXAMPLE 31

### Fluorenyl-[N-(amino-2,3-propandiol)] succinimide half ester

To a suspension of fluorenyl succinic acid half ester (3.05g, 11.24mmol) in DMF (50ml) was added 1-hydroxybenzotriazole (2.28g, 16.86 mmol) and 1-(3-dimethylaminopropyl) -3-ethylcarbodiimide-HCl (3.23g, 16.84mmol) sequentially. The mixture was stirred at room temperature for two hours and transferred via cannula to a flask containing 1-amino-2,3-propanediol (1.02g, 11.24mmol). The reaction was stirred for three hours at room temperature. The mixture was concentrated and partitioned between ethyl acetate (100ml) and water (50ml). The layers were separated and the aqueous layer was extracted with ethyl acetate (3x50ml). The ethyl acetate extracts were combined, dried over sodium sulfate, filtered, and concentrated. ¹H NMR: ( CDCl₃, 200 MHz) δ 7.8-7.26 (8H, m, ArH), 4.43 (2H, d, J=6.5 Hz, CHCH₂OCO), 4.23 ( 1H, t, J=6.5 Hz, CHCH₂OCO), 2.70 (4H, s, OCOCH₂CH₂COO).

### EXAMPLE 32

### Fluorenyl-[N-(1-amino-3-O-dimethoxytrityl-2-propanol)succinimide] half ester

A solution of fluorenyl-[N-(2,3-propandiol)] succinimide half ester (0.74g, 2.0mmol) in pyridine (20ml) was treated with 4,4'-dimethoxytritylchloride (0.68g, 2.0mmol). The mixture was stirred overnight at room temperature. Thin layer chromatography (TLC) analysis of the product shows complete conversion to a higher migrating spot, which gives the characteristic red color when sprayed with 10% sulfuric acid in methanol and heated.

### EXAMPLE 33

### Fluorenyl-N-{3-O-dimethoxytrityl-2-O-[(N,N-diisopropylamino)-2-cyanoethoxyphosphite]propane} succinimide ester

The title phosphoramidite is prepared from fluorenyl-[N-(3-O-dimethoxytrityl-2-propanol)] succinimide half ester utilizing the procedure of example 27.

### EXAMPLE 34

### 1-[(N-Benzoyl)amino]-2,3-propandiol.

R-(+)-glycidol (10ml, 0.15mol) and isopropyl alcohol saturated with ammonia (70ml) were sealed in a bomb and stirred at room temperature for 5 days. The bomb was cooled and opened. The solution was evaporated and the residue dissolved in pyridine (100ml). This solution was treated with benzoyl chloride (47ml, 0.4mol) for 30 minutes and filtered. The filtrate was evaporated and dissolved in methanol (400ml) then treated with ammonium hydroxide (100ml). The mixture was stirred for 4 hours and evaporated. The residue was purified by silica gel column chromatography. Elution with ethyl acetate:methanol (19/1, v/v), pooling of appropriate fractions and evaporation gave a yield of 9.9g (34%) ¹H NMR (DMSO d₆); δ, 3.1 to 3.5 (m, 4, CH₂CHOHCH₂OH); 3.6 to 3.7 (m, 1, CHOH); 4.6 to 4.7 (t, 1, CH₂OH, exchanges with D₂O); 4.8 to 4.9 (d, 1, CHOH, exchanges with D₂O); 7.4 TO 7.6 and 7.8 to 7.9 (m, 5, enzoyl); 8.4 to 8.5 (t, 1, NH).

### EXAMPLE 35

### 1-[(N-Benzoyl)amino]-3-O-dimethoxytrityl-2-O-[(N,N-diisopropylamino)-2-cyanoethoxyphosphite]propane

1-[(N-Benzoyl)amino]-2,3-propandiol is treated as per the procedure of Example 5 with dimethoxytrityl chloride in pryidine to give 1-[(N-benzoyl)amino]-3-O-dimethoxytrityl-2-propanol. The resulting 1-[(N-benzoyl)amino]-3-O-dimethoxytrityl-2-propanol is further phosphitylated with chloro-β-cyanoethoxy-N,N-diisopropylaminophosphine as per Example 6 to give the title compound.

### EXAMPLE 36

### 1-(1-Pyrrole)-3-O-dimethoxytrityl-2-O-[(N,N-diisopropylamino)-2-cyanoethoxyphosphite]propane

Pyrrole is treated as per the procedure of Example 4 with glycidol in the presence of potassium carbonate to give 1-(1H-pyrrole)-2,3-propandiol. The resulting 1-(1-pyrrole)-2,3-propandiol is further treated as per the procedure of Example 5 with dimethoxytrityl chloride in pryidine to give 1-(1-pyrrole)-3-O-dimethoxytrityl-2-propanol. The resulting 1-(1-pyrrole)-3-O-dimethoxytrityl-2-propanol is further phosphitylated, with chloro-β-cyanoethoxy-N,N-diisopropylaminophosphine as per Example 6 to give the title compound.

### EXAMPLE 37

### 1-(10-Phenoxazine)-3-O-dimethoxytrityl-2-O-[(N,N-diisopropylamino)-2-cyanoethoxyphosphite]propane

Phenoxazine is treated as per the procedure of Example 4 with glycidol in the presence of potassium carbonate to give 1-(1-phenoxazine)-2,3-propandiol. The resulting 1-(1-phenoxazine)-2,3-propandiol is treated as per the procedure of Example 5 with dimethoxytrityl chloride in pryidine to give 1-(1-phenoxazine)-3-O-dimethoxytrityl-2-propanol. The resultingl-(1-phenoxazine)-3-O-dimethoxytrityl-2-propanolis phosphitylated with chloro-β-cyanoethoxy-N,N-diisopropylaminophosphine as per Example 6 to give the title compound.

### EXAMPLE 38

### 1-(1-Pyrazole)-3-O-dimethoxytrityl-2-O-[(N,N-diisopropylamino)-2-cyanoethoxyphosphite]propane

Pyrazole is treated as per the procedure of Example 4 with glycidol in the presence of potassium carbonate to give 1-(1-pyrazole)-2,3-propandiol. The resulting 1-(1-pyrazole) - 2,3-propandiol is treated as per the procedure of Example 5 with dimethoxytrityl chloride in pryidine to give 1-(1-pyrazole)-3-O-dimethoxytrityl-2-propanol. The resulting 1-(1-pyrazole)-3-O-dimethoxytrityl-2-propanol is phosphitylated with chloro-β-cyanoethoxy-N,N-diisopropylaminophosphine as per Example 6 to give the title compound.

### EXAMPLE 39

### 1-(1-Indole)-3-O-dimethoxytrityl-2-O-[(N,N-diisopropylamino)-2-cyanoethoacypbosphite]propane

Indole is treated as per the procedure of Example 4 with glycidol in the presence of potassium carbonate to give 1-(1-indole)-2,3-propandiol. The resulting 1-(1-indole)-2,3-propandiol is treated as per the procedure of Example 5 with dimethoxytrityl chloride in pryidine to give 1-(1-indole)-3-O-dimethoxytrityl-2-propanol. The resulting 1-(1-indole)-3-O-dimethoxytrityl-2-propanol is phosphitylated with chloro-β-cyanoethoxy-N,N-diisopropylaminophosphine as per Example 6 to give the title compound.

### EXAMPLE 40

### 1-(1-Indazole)-3-O-dimethoxytrityl-2-O-[(N,N-diisopropylamino)-2-cyanoethoxyphosphite]propane

1H-Indazole is treated as per the procedure of example 4 with glycidol in the presence of potassium carbonate to give 1-(1H-indazole)-2,3-propandiol. The resulting 1-(1H-indazole)-2,3-propandiol is treated as per the procedure of Example 5 with dimethoxytrityl chloride in pryidine to give 1-(1-indazole)-3-O-dimethoxytrityl-2-propanol. The resulting 1-(1-indazole)-3-O-dimethoxytrityl-2-propanol is phosphitylated with chloro-β-cyanoethoxy-N,N-diisopropylaminophosphine as per Example 6 to give the title compound.

### EXAMPLE 41

### 1-(9-Purine)-3-O-dimethoxytrityl-2-O-[(N,N-diisopropylamino)-2-cyanoethoxyphosphite]propane

Purine is treated as per the procedure of example 4 with glycidol in the presence of potassium carbonate to give 1- (9-purine) -2,3-propandiol. The resulting 1- (9-purine) -2,3-propandiol is treated as per the procedure of example 5 with dimethoxytrityl chloride in pryidine to give 1-(9-purine)-3-O-dimethoxytrityl-2-propanol. The resulting 1-(9-purine)-3-O-dimethoxytrityl-2-propanol is phosphitylated with chloro-β-cyanoethoxy-N,N-diisopropylaminophosphine as per Example 6 to give the title compound.

### EXAMPLE 42

### 1-(10-Phenothiazine)-3-O-dimethoxytrityl-2-O-[(N,N-diisopropylamino)-2-cyanoethoxyphosphite]propane

Phenothiazine is treated as per the procedure of Example 4 with glycidol in the presence of potassium carbonate to give 1-(10-phenothiazine)-2,3-propandiol. The resulting 1-(10-phenothiazine)-2,3-propandiol is treated as per the procedure of example 5 with dimethoxytrityl chloride in pryidine to give 1-(10-phenothiazine)-3-O-dimethoxytrityl-2-propanol. The resulting 1-(10-phenothiazine)-3-0-dimethoxytrityl-2-propanol is phosphitylated with chloro-β-cyanoethoxy-N,N-diisopropylaminophosphine as per Example 6 to give the title compound.

### EXAMPLE 43

### 1-(9-β-Carboline)-3-O-dimethoxytrityl-2-O-[(N,N-diiuopropylamino)-2-cyanoethoxyphosphite]propane

β-Carboline is treated as per the procedure of Example 4 with glycidol in the presence of potassium carbonate to give 1-(9-β-carboline)-2,3-propandiol. The resulting 1-(9-β-carboline)-2,3-propandiol is treated as per the procedure of Example 5 with dimethoxytrityl chloride in pryidine to give 1-(9-β-carboline)-3-O-dimethoxytrityl-2-propanol. The resulting 1-(9-β-carboline)-3-O-dimethoxytrityl-2-propanol is phosphitylated with chloro-β-cyanoethoxy-N,N-diisopropylaminophosphine as per Example 6 to give the title compound.

### EXAMPLE 44

### 1-(10-Phenothiazine)-3-O-dimethoxytrityl-2-O-[(N,N-diisopropylamino)-2-cyanoethoxyphosphite]propane

Phenothiazine is treated as per the procedure of Example 4 with glycidol in the presence of potassium carbonate to give 1-(10-phenothiazine)-2,3-propandiol. The resulting 1-(10-phenothiazine)-2,3-propandiol is treated as per the procedure of Example 5 with dimethoxytrityl chloride in pryidine to give 1-(10-phenothiazine)-3-O-dimethoxytrityl-2-propanol. The resulting 1-(10-phenothiazine)-3-0-dimethoxytrityl-2-propanol is phosphitylated with chloro-β-cyanoethoxy-N,N-diisopropylaminophosphine as per Example 6 to give the title compound.

### EXAMPLE 45

### 1-[1-(5-Propynyl)uracil]-3-O-dimethoxytrityl-2-O-[(N,N-diisopropylamino)-2-cyanoethoxyphosphite]propane

5-Propynyl uracil is treated as in the procedure of Example 9 with glycidol in the presence of potassium carbonate to give 1-[1-(5-propynyl)uracil)-2,3-propandiol. The resulting 1-[1-(5-propynyl)uracil]-2,3-propandiol is treated with dimethoxytrityl chloride in pyridine as in Example 10 to givel-[1-(5-propynyl)uracil]-3-O-dimethoxytrityl-2-propanol. The resulting 1- [1- (5-propynyl)uracil]-3-O-dimethoxytrityl-2-propanol is phosphitylated with chloro-β-cyanoethoxy-M,N-diisopropylaminophosphine as per Example 11 to give the title compound.

### EXAMPLE 46

### 1-[1-(6-Aza)thymine]-3-O-dimethoxytrityl-2-O-[(N,N-diisopropylamino)-2-cyanoethoxyphosphite]propane

6-Azathymine is treated as in the procedure of Example 1 with glycidol in the presence of potassium carbonate to give 1-[1-(6-aza)thymine)-2,3-propandiol. The resulting 1-[1-(6-aza)thymine]-2,3-propandiol is treated with dimethoxytrityl chloride in pyridine as in Example 2 to give 1-[1-(6-aza)-thymine]-3-O-dimethoxytrityl-2-propanol. The resulting 1-[1-(6-aza)thymine)-3-O-dimethoxytrityl-2-propanol is phosphitylated with chloro-β-cyanoethoxy-N,N-diisopropylaminophosphine as per Example 3 to give the title compound.

### EXAMPLE 47

### 1- (9-Hypoxanthine)-3-O-dimethoxytrityl-2-O-[(N,N-diisoprapylamino)-2-cyanoethoxyphosphite]propane

Hypoxanthine is treated as in the procedure of Example 1 with glycidol in the presence of potassium carbonate to give 1-(hypoxanthine-9-yl)-2,3-propandiol. The resulting 1-(9-hypoxanthine)-2,3-propandiol is treated with dimethoxytrityl chloride in pyridine as in Example 2 to give 1-(9-hypoxanthine)-3-O-dimethoxytrityl-2-propanol. The resulting 1-(9-hypoxanthine)-3-O-dimethoxytrityl-2-propanol is phosphitylated with chloro-β-cyanoethoxy-N,N-diisopropylaminophosphine as per Example 3 to give the title compound.

### EXAMPLE 48

### 1- [9- (2,6-Diamino)purine]-3-O-dimethoxytrityl-2-O-[(N,N-diisopropylamino)-2-cyanoethoxyphosphite]propane

2,6-Diaminopurine is treated as in the procedure of Example 16 with glycidol in the presence of potassium carbonate to give 1- [9- (2,6-diamino)purine]-2,3-propandiol. The resulting 1-[9-(2,6-diamino)purine]-2,3-propandiol is protected as in Example 18 by treatment with chlorotrimethylsilane and isobutyryl chloride to give 1-[9-(N2,N6-diisobutyryl-2,6-diamino)purine]-2,3-propandiol. The resulting 1-[9-(N2,N6-diisobutyryl-2,6-diamino)purine]-2,3-propandiol is treated with dimethoxytrityl chloride in pyridine as in Example 19 to give 1-[9-(N2,N6-diisobutyryl-2,6-diamino)-purine [] -3-O-dimethoxytrityl-2-propanol. The resulting 1-[9-(N2,N6-diisobutyryl-2,6-diamino)purine]-3-O-dimethoxytrityl-2-propanol is then phosphitylated with chloro-β-cyanoethoxy-N,N-diisopropylaminophosphine as per Example 24 to give the title compound.

### EXAMPLE 49

### 1-[9-(7-Methylguanine)]-3-O-dimethoxytrityl-2-O-[(N,N-diisopropylamino)-2-cyanoethoxyphosphite]propane

7-Methylguanine is treated as in the procedure of Example 16 with glycidol in the presence of potassium carbonate to give 1-[9-(7-methylguanine)]-2,3-propandiol. The resulting 1-[9-(7-methylguanine)]-2,3-propandiol is protected as in Example 18 by treatment with chlorotrimethylsilane and isobutyryl chloride to give 1- [9-(N2-isobutyryl)-(7-methylguanine)] -2,3-propandiol. The resulting 1- [9- (N2-isobutyryl) - (7-methylguanine)]-2,3-propandiol is treated with dimethoxytrityl chloride in pyridine as in Example 19 to give 1-[9-(N2-isobutyryl)-(7-methylguanine)]-3-O-dimethoxytrityl-2-propanol. The resulting 1-[9-(N2-isobutyryl)-(7-methylguanine)]-3-0-dimethoxytrityl-2-propanol is then phosphitylated with chloro-β-cyanoethoxy-N,N-diisopropylaminophosphine as per Example 20 to give the title compound.

### EXAMPLE 50

### N-(α-Fmoc)-N'-(amino-2,3-propandiol)glycinamide

A solution of N-α-Fmoc-glycine-pentafluorophenyl ester (4.0 g, 8.6 mmole) in dry dimethylformamide was treated with 1-amino-2,3-propandiol (0.78 g, 8.6 mmole) under an atmosphere of argon. The mixture was stirred at room temperature for 6 hr and the solvent evaporated under reduced pressure. The syrup which resulted was kept under vacuum overnight and then dissolved in 40 ml hot ethyl acetate. The hot solution was filtered, the filtrate cooled to -20°C and kept in ice overnight. The material which had crystallized was filtered, washed with cold ethyl acetate and the filtrate evaporated to a minimum volume. A second incubation at this temperature yielded a second crop of crystals. Yield of white crystals is 2.2 g (69%). ¹H nmr (dimethylsulfoxide-d₆) : 8.0-7.2 (m, 8, aromatic); 5.0-4.5 (m, 4, N-H and OH, exchangeable with D₂O) ; 4.3 (m, 3, Fmoc); 3.7 (m, 2, glycine) ; 3.5-3.0 (m, 5, propane).

### EXAMPLE 51

### N-(α-Fmoc)-N'-{3-O-dimethoxytrityl-2-O-[(N,N-diisopropylamino)-2-ayanoethoxyphosphite]aminopropane}glycinamide

A solution of N-(α-Fmoc)-N'-(amino-2,3-propandiol)-glycinamide (2.0mmol) in pyridine (20ml) is treated with 4,4'-dimethoxytritylchloride (0.68g, 2.0mmol) as per the procedure of Example 32. The mixture is stirred overnight at room temperature to give N-(α-Fmoc) -N'-(1-amino-3-O-dimethoxytrityl-2-propanol)glycinamide that is isolated by silica gel chromatography. The resulting N-(α-Fmoc)-N'-(1-amino-3-O-dimethoxytrityl-2-propanol)glycinamide is treated as per the procedure of Example 27 to give the title compound.

### EXAMPLE 52

### 1-Dimethoxytrityl-2-O-[(N,N-diisopropylamino)-2-cyanoethoxyphosphite]heptadecane

Dodecylbromide is reacted with metallic magnesium in ethyl ether to yield dodecylomagnesium bromide. The dodecylmagnesium bromide is reacted with 1-O-dimethoxytritylglycidol (see Example 25) in THF in the presence of dilithiumtetrachlorocuprate to give l-dimethoxytrityl-2-heptadecanol. The resulting 1-dimethoxytrityl-2-heptadecanol is reacted with chloro-β-cyanoethoxy-N,N-diisopropylaminophosphine in the presence of diisopropylethylamine in THF to give the title compound.

### EXAMPLE 53

### 1-Amino-3-O-dimethoxytrityl-2-propanol

1-O-Dimethoxytritylglycidol (from Example 25, 10ml, 0.15mol) and isopropyl alcohol saturated with ammonia (70ml) are sealed in a bomb and stirred at room temperature for 5 days. The bomb is cooled, opened and the solution evaporated to give the crude product.

### EXAMPLE 54

### 1-[(N-Palmitoyl)amino]-3-O-dimethoxytritylmethyl-2-O-[(N,N-diisopropylamino)-2-cyanoethoxyphosphite]propane

1-Amino-3-O-dimethoxytrityl-2-propanol (from Example 53) is dissolved in 5 ml dry pyridine and chlorotrimethylsilane (0.227 ml, 194 mg, 1.79 mmol), is added with stirring for 1 hour. Palmitic acid (359 mg, 1.40 mmol), hydroxybenzotriazole (209 mg, 1.55 mmol) and dimethylaminopropylethylcarbodiimide (EDC) (281 mg, 1.80 mmol) are dissolved in 5 ml DMF (if necessary, 5 ml CH₂Cl₂ co-solvent is added) and stirred for 1 hour. This solution is then added to the pyridine solution of the crude 1-amino-3-O-dimethoxytrityl-2-propanol, and the solution stirred until complete disappearance of the starting material. The reaction is stopped by addition of 5 ml saturated NaHCO₃ and after 15 min. the solution is diluted with water (100 ml), extracted with ethyl acetate (2X 75 ml), washed with NaHCO₃, brine, dried and evaporated. The residue is purified by silica gel chromatography to give 1-[(N¹-palmitoyl)amino]-3-O-dimethoxytritylmethyl-2-propandiol. The resulting 1- [(N¹-palmitoyl)amino] -3-O-dimethoxytritylmethyl-2-propandiol is phosphitylated with chloro-β-cyanoethoxy-N,N-diisopropylaminophosphine as per Example 20 to give the title compound.

### EXAMPLE 55

### 1-[(N-Isobutyroyl)amino]-3-O-dimethoxytritylmethyl-2-O-[(N,N-diisopropylamino)-2-cyanoethoxyphosphite]propane

The title compound is prepared as per the procedure of Example 54 by the use of isobutyric acid as the carboxylic acid component.

### EXAMPLE 56

### 1-[(N-Phenylacetyl)amino]-3-O-dimethoxytritylmethyl-2-O-[(N,N-diisopropylamino)-2-cyanoethoxyphosphite]propane

The title compound is prepared as per the procedure of Example 54 by the use of phenylacetic acid as the carboxylic acid component.

### EXAMPLE 57

### Glutaric acid monofluorenylmethyl ester.

Fluorenylmethanol (0.90 g, 4.5 mmol) and dimethylaminopyridine (50 mg) are dissolved in 10 ml dry pyridine. Glutaric anhydride (5.0 mmol) is added and the solution stirred overnight. The solvent is removed under reduced pressure, sodium bicarbonate added (50 ml), the solution extracted with ethyl acetate (50 ml), and the organic layer discarded. The aqueous layer is acidified to pH 2, extracted with ethyl acetate (2 X 100 ml), washed with brine, and the solvent removed under reduced pressure. The residue is purified by flash chromatography to give the product.

### EXAMPLE 58

### 1-[(N-(Fluorenylmethylglutaroyl)amino]-3-O-dimethoxytritylmethyl-2-O-[(N,N-diisopropylamino)-2-cyanoethoxyphosphite]-propane

The title compound is prepared as per the procedure of Example 54 using glutaric acid monofluorenylmethyl ester (Example 57) as the carboxylic acid component.

### EXAMPLE 59

### 2-(1-Thymine)acetic acid

Methyl bromoacetate (25.5 g, 15.2 ml, 160 mmol) was added to a suspension of K₂CO₃ (44.2 g, 320 mmol) and thymidine (20.2 g, 160 mmol) in 500 ml dry DMF with stirring overnight. The suspension was filtered and the solvent removed under reduced pressure. The residue was suspended in 120 ml H₂O and 30 ml 4 N HCl, stirred for 30 min and filtered again. The solid was suspended in 250 ml H₂O, to which was added 100 ml 2.5 M NaOH. The solution was heated to boiling, cooled and acidified to pH 1 with concentrated HCl. The precipitate was dried in vacuo to give 13.6 g (73.6 mmol, 46%) pure product. ¹H NMR: ( DMSO-d6, 200 MHz) δ 7.48 (s, 1H, H6), 4.37 (s, 2H, CH₂), 1.76 (s, 3H, CH₃).

### EXAMPLE 60

### 1-{N-[2-(1-Thymidine)acetyl]amino}-3-O-dimethoxytritylmethyl-2-O-[(N,N-diisopropylamino)-2-cyanoethoxyphosphite]propane

The title compound is prepared as per the procedure of Example 54 using thymidine-2-acetic acid as the carboxylic acid component.

### EXAMPLE 61

### N-Fmoc-3-Aminopropionic Acid.

Sodium bicarbonate (2.52 g, 30 mmol) and 3-aminopropionic acid (1.00 g, 11.2 mmol) were dissolved in 50 ml water and 50 ml dioxane was added. A solution of fluorenylmethyl chloroformate ( 3.10 g, 12.0 mmol) in 50 ml dioxane was added dropwise with stirring. After 6 hours the solution was diluted with water (100 ml) and saturated bicarbonate solution (50 ml), extracted once with diethyl ether, and the aqueous layer acidified to pH 2 with concentrated HCl. The cloudy solution was extracted with ethyl acetate (2 X 100 ml), washed with brine and dried with MgSO₄. After evaporation a mixture of the title product and the peptide dimer was obtained. The pure product was obtained by flash chromatography. ¹H NMR: ( CDCl₃, 200 MHz) δ 7.95-7.26 (8H, m, ArH), 7.40-7.15 (3H, m, CHCH₂O), 3.20 (2H, t, J=8 Hz, CH₂N), 2.40 (2H, t, J=8 Hz, HOOCCH₂).

### EXAMPLE 62

### 1-[N-(N-Fmoc-3-aminopropionoyl)amino]-3-O-dimethoxytritylmethyl-2-O-[(N,N-diisopropylamino)-2-cyanoethoxyphosphite]propane

The title compound is prepared'as per the procedure of Example 54 using N-Fmoc-3-aminopropionic acid as the carboxylic acid component.

### EXAMPLE 63

### 2-(N-Imidazolyl)acetic acid.

Imidazole (3.7 g, 54 mmol) was added to a suspension of sodium hydride (2.6 g of a 60% dispersion in oil, 60 mmol) in 50 ml dry THF. Bromoacetic acid (3.4 g, 24 mmol) was then added and the mixture stirred overnight. Water (1 ml) was then added and the solvent removed under reduced pressure. The residue was taken up in water (50 ml, pH >10), extracted with ether and the organic layer discarded. The aqueous layer was acidified to pH 1 with concentrated HCl and extracted again with ether. The aqueous layer was evaporated to dryness. The oily residue was dissolved in absolute ethanol (EtOH) to precipitate NaCl, and recrystallized from acetone/methanol to give 1.22 g (7.5 mmol, 30%) pure product as the hydrochloride. ¹H NMR: ( DMSO-d6, 200 MHz) δ 9.20 (s, H2), 7.76 (d, J = 1.5 Hz), 7.69 (d, J = 1.5 Hz), 5.20 (s, CH₂) .0

### EXAMPLE 64

### 1-{N-[2- (N-Imidazolyl)acetyl]amino}-3-O-dimethoxytritylmethyl-2-O-[(N,N-diisopropylamino)-2-cyanoethoxyphosphite]propane

The title compound is prepared as per the procedure of Example 54 using N-imidazolyl-2-acetic acid as the carboxylic acid component.

### EXAMPLE 65

### 2-(9-Adenine)acetic acid ethyl ester.

Sodium hydride (8.20 g 60% in oil, 205 mmol) was added to a suspension of adenine (25.0 g, 185 mmol) in 500 ml DMF. After 2 hours vigorous mechanical stirring H₂ evolution stopped and a thick slurry was obtained. Ethyl bromoacetate (55.6 g, 36.9 ml, 333 mmol) was added dropwise over 3 hours, and stirring continued for a further hour. Water (10 ml) and H₂SO₄ were added to pH 4. The solvent was evaporated and the residue suspended in 500 ml H₂O, filtered and washed with water. The residue was recrystallized from 400 ml ethanol to give 23.8 g (108 mmol, 58%) pure product.

### EXAMPLE 66

### 2-[9-(N2-Benzoyl)adenine)acetic acid.

To a suspension of 2- (9-adenine) acetic acid ethyl ester (6.06 g, 27.4 mmol) in 250 ml dry pyridine was added benzoyl chloride (9.60 ml, 11.6 g, 82 mmol), and the solution stirred for 4 hours at room temperature. Methanol (25 ml) was added and the solvents evaporated. The residue was dissolved in ethyl acetate (2 X 250 ml), washed with 0.1 N HCl, H₂O, NaHCO₃ saturated, brine, and dried with Na₂SO₄. The organic extracts were evaporated and the solid residue was redissolved in 250 ml THF at 0°C, to which was added 100 ml 1M NaOH. The solution was stirred at 0°C for 1 hour and acidified to pH 1 with concentrated HCl, and the aqueous portion extracted once with ether. The product, which began to crystallize almost immediately, was collected by filtration to yield 4.96 g (61%). ¹H NMR: ( DMSO-d6, 200 MHz) δ 8.86, 8.84 (d, H2, H8), 8.1 (d, 2H, J = 7.0 Hz, ArH), 7.69-7.58 (m, 3H, Ar-H), 5.22 (s, 2H, CH₂).

### EXAMPLE 67

### 1-{{N-{2-[9-(N2-Benzoyl)adenine]acetyl}amino}}-3-O-dimethoxytritylmethyl-2-O-[(N,N-diisopropylamino)-2-cyanoethoxyphosphite]propane

The title compound is prepared as per the procedure of Example 54 using 2-[9-(N2-benzoyl)adenine]acetic acid as the carboxylic acid component.

### EXAMPLE 68

### 2-[1-(N4-Benzoyl)cytosine]acetic acid.

Cytosine hemihydrate (12.0g, 100 mmol) was dried by coevaporation with pyridine, redissolved in dry pyridine (250 ml), and benzoyl chloride added dropwise (70.3 g, 500 mmol) with cooling. The solution was stirred overnight, water added and the solvent removed *in vacuo.* The residue was dissolved in 700 ml H₂O containing 55 g NaOH. Once complete dissolution had occurred stirring was continued for one hour. The solution was then acidified to pH 4, and the white precipitate collected, boiled in 1 L ethanol and filtered again to give 16.1 g benzoylcytosine. Fifteen grams of this was suspended in 500 ml DMF with 9.7 g (70 mmol) K₂CO₃ and methyl bromoacetate (10.7 g, 70 mmol). The suspension was stirred for 3 days, filtered and the solvent removed. Water was added (100 ml) and 10 ml 4N HCl. The suspension was stirred 15 min and filtered. The solid was resuspended in 200 ml H₂O containing 4.8 g NaOH. The suspension was stirred 45 min until all the solid had dissolved. The solution was then acidified to pH 2, the solid collected by filtration and dried to give 10.6 g product (43%). The product was identified by NMR.

### EXAMPLE 69

### 1-{{N-{2- [1- (N4-Benzoyl)cytosine]acetyl}amino}}-3-O-dimethoxytritylmethyl-1-amino-2-O-[(N,N-diisopropylamino)-2-cyanoethoxyphosphite]propane

The title compound is prepared via the procedure of Example 54 using 2-[1-(N4-benzoyl)cytosine]acetic acid as the carboxylic acid component.

### EXAMPLE 70

### 2-[9-(N2-Isobutyroyl)guanine]acetic acid.

To a suspension of 2-amino-6-chloropurine (10 mmol) and K₂CO₃ (15 mmol) in DMF (25 ml) is added ethyl bromoacetate (10 mmol). The mixture is stirred vigorously for 24 hrs, filtered and the solvent evaporated. The residue is resuspended in 25 ml pyridine and isobutyroyl chloride added (20 mmol). After stirring for 18 hrs, water is added and the solvent removed. The residue is suspended in 1N HCl and heated to reflux for 1 hr. The suspension is then cooled to 0°C, NaOH added to pH 12, and the suspension stirred for 1 hr. The solution is acidified to pH 3, and the product is collected by filtration.

### EXAMPLE 71

### 1-{{N-(2-[9-(N2-Isobutyroyl)guanine]acetyl}amino}}-3-O-dimethoxytritylmethyl-1-amino-2-O-[(N,N-diisopropylamino)-2-cyanoethaxyphosphite]propane

The title compound is prepared via the procedure of Example 54 using 2-[9-(N2-isobutyroyl)guanine]acetic acid as the carboxylic acid component.

### EXAMPLE 72

### Benzyl 3,6,9,12-tetraoxatridecanoate.

Triethyleneglycol monomethyl ether (10 mmol) and benzyl bromoacetate (11 mmol) are added to a suspension of anhydrous K₂CO₃ (15 mmol) in 50 ml anhydrous DMF. The suspension is stirred at room temperature overnight. Water is added and the emulsion is extracted with ethyl acetate (3 X 200 ml), washed with water, brine, and dried with MgSO₄. The solvent is evaporated and the residual oil purified by flash chromatography to give the title compound.

### EXAMPLE 73

### 3,6,9,12-Tetraoxatridecanoic acid.

Benzyl 3,6,9,12-tetraoxatridecanoate (5 mmol) is dissolved in methanol (50 ml) and 10% palladium on carbon is added (100 mg catalyst/mmol). The suspension is shaken under 30 psi H₂ until the starting material is consumed. The suspension is filtered through a short pad of Celite, washed thoroughly with methanol, and the solvent evaporated. The product is used directly without purification.

### EXAMPLE 74

### 1-[N-(3,6,9,12-Tetraoxatridecanoyl)amio]-3-O-dimethoxytritylmethyl-2-O-[(N,N-diisopropylamino)-2-cyanoethoxyphosphite]propane

The title compound is prepared via the procedure of Example 54 using 3,6,9,12-tetraoxatridecanoic acid as the carboxylic acid component.

### EXAMPLE 75

### Benzyl bis-[(2-pyridyl) -2-ethyl]aminoacetate.

To a suspension of K₂CO₃ (15 mmol) in 25 ml DMF was added 2,2'-bis(2-pyridylethyl)-amine (10 mmol) followed by benzyl bromoacetate (12 mmol). The suspension was stirred for 4 hours at room temperature. Water was then added, and the suspension extracted with ethyl acetate (2 X 100 ml), washed with 5% Na₂CO₃, water, brine, dried with MgSO₄ and the solvents removed. The product was obtained as an oil in quantitative yield. Product was identified by NMR.

### EXAMPLE 76

### Bis(2-(2-pyridyl)ethyl)aminoacetic acid.

Benzyl bis-[(2-pyridyl)-2-ethyl]aminoacetate (5 mmol) is dissolved in methanol (50 ml) and 10% Palladium on carbon is added (100 mg catalyst/mmol). The suspension is shaken under 30 psi H₂ until the starting material is consumed. The suspension is filtered through a short pad of Celite, washed thoroughly with methanol, and the solvent evaporated. The product is used directly without purification.

### EXAMPLE 77

### 1-{{N-{Bis[2-(2-pyridyl)ethyl]aminoacetyl}amino}}-3-O-dimethoxytritylmethyl-2-O-[(N,N-diisopropylamino)-2-cyanoethoxyphosphite]propane

This compound is prepared via the procedure of Example 54 using bis(2-(2-pyridyl)ethyl)-aminoacetic acid as the carboxylic acid component.

### EXAMPLE 78

### 1-[N-(Toluenesulfonyl)amino]-3-O-dimethoxytritylmethyl-2-O-[(N,N-diisopropylamino)-2-cyanoethoxyphosphite]propane

Chlorotrimethylsilane (6.0 mmol) is added dropwise to a solution of 1-amino-3-O-dimethoxytrityl-2-propanol (5.0 mmol) in 25 ml dry pyridine. After stirring one hour, toluenesulfonyl chloride (6.0 mmol) is added in portions, and stirring continued for two hours. The reaction is quenched with saturated aqueous NaHCO₃, and the mixture stirred until the silyl ethers were hydrolyzed. The solvent is removed *in vacuo*, and the residue partitioned between water and ethyl acetate. The organic layer is washed with NaHCO₃, water, brine and dried with Na₂SO₄. The solvent is removed and the resulting oil purified by flash chromatography, using a gradient of methanol in CHCl₃. The resulting 1-[N-(toluenesulfonyl)amino]-3-O-dimethoxytritylmethyl-2-propanol is phosphitylated with chloro-β-cyanoethoxy-N,N-diisopropylaminophosphine as per Example 20 to give the title compound.

### EXAMPLE 79

### 1-[N-(Trifluoromethanesulfonyl)amino]-3-O-dimethoxytritylmethyl-2-O-[(N,N-diisopropylamino)-2-cyanoethoxyphosphite]propane

Chlorotrimethylsilane (6.0 mmol) is added dropwise to a solution of 1-amino-3-O-dimethoxytrityl-2-propanol (5.0 mmol) and triethylamine (15 mmol) in 50 ml dry CH₂Cl₂. After 1 hour the solution is cooled to -78°C, and trifluoromethanesulfonic anhydride (5.5 mmol) is added dropwise. The cooling bath is removed and the mixture allowed to warm to room temperature. The crude product is dissolved in pyridine and NaHCO₃ solution is added to hydrolyze the TMS ether. The solvent is evaporated, the residue partitioned between ethyl acetate and water, washed with NaHCO₃, brine and dried with MgSO₄. The residue is purified by flash chromatography using a gradient of methanol in CHCl₃. The resulting 1-[N-(trifluoromethanesulfonyl)amino]-3-O-dimethoxytritylmethyl-1-amino-2-propanol is phosphitylated with chloro-β-cyanoethoxy-N,N-diisopropylaminophosphine as per Example 20 to give the title compound.

### EXAMPLE 80

### 1-[N-(Benzyl)amino]-3-O-dimethoxytritylmethyl-2-O-[(N,N-diisopropylamino)-2-cyanoethoxyphosphite]propane

Chlorotrimethylsilane (6.0 mmol) is added dropwise to a solution of 1-amino-3-O-dimethoxytrityl-2-propanol (5.0 mmol), imidazole (5 mmol) and triethylamine (15 mmol) in 25 ml dry DMF. After stirring one hour the solvent is removed *in vacuo,* and the residue redissolved in acetonitrile (25 ml) and triethylamine (10 mmol). Benzyl bromide (6.0 mmol) is added, and stirring continued overnight. The reaction is quenched with saturated aqueous NaHCO₃, and the mixture stirred until the silyl ethers were hydrolyzed. The solvent is removed *in vacuo*, and the residue partitioned between water and ethyl acetate. The organic layer is washed with NaHCO₃, water, brine and dried with Na₂SO₄. The solvent is removed and the resulting oil purified by flash chromatography, using a gradient of methanol in CHCl₃. The resulting 1-[N-(benzyl)amino]-3-O-dimethoxytritylmethyl-1-amino-2-propanol is phosphitylated with chloro-β-cyanoethoxy-N,N-diisopropylaminophosphine as per Example 20 to give the title compound.

### EXAMPLE 81

### 1-[N-(Aminocarbonyl)amino]-3-O-dimethoxytritylmethyl-2-O-[(N,N-diisopropylamino)-2-cyanoethoxyphosphite]propane

Chlorotrimethylsilane (6.0 mmol) is added dropwise to a solution of 1-amino-3-O-dimethoxytrityl-2-propanol (5.0 mmol) and triethylamine (15 mmol) in 50 ml dry CH₂Cl₂. After one hour, dimethylaminopyridine (1 mmol) is added followed by trimethylsilyl isocyanate (5.5 mmol). The solution is stirred until the starting material is consumed. The solvent is removed in vacuo and the crude product redissolved in pyridine and NaHCO₃ solution to hydrolyze the TMS ethers. The solvent is evaporated, the residue partitioned between ethyl acetate and water, washed with NaHCO₃, brine and dried with MgSO₄. The residue is purified by flash chromatography using a gradient of methanol in CHCl₃. The resulting 1-[N-(aminocarbonyl)amino]-3-O-dimethoxytritylmethyl-2-propanol is phosphitylated with chloro-β-cyanoethoxy-N,N-diisopropylaminophosphine as per Example 20 to give the title compound.

### EXAMPLE 82

### 1-[N-(Methylaminothiocarbonyl)amino]-3-O-dimethoxytritylmethyl-2-O-[(N,N-diisopropylamino)-2-cyanoethoxyphosphite]-propane

Chlorotrimethylsilane (6.0 mmol) is added dropwise to a solution of 1-amino-3-O-dimethoxytrityl-2-propanol (5.0 mmol) and triethylamine (15 mmol) in 50 ml dry CH₂Cl₂. After one hour, dimethylaminopyridine (1 mmol) is added followed by methylisothiocyanate (5.5 mmol). The solution is stirred until the starting material is consumed. The solvent is removed *in vacuo* and the crude product redissolved in pyridine and NaHCO₃ solution to hydrolyze the TMS ethers. The solvent is evaporated, the residue partitioned between ethyl acetate and water, washed with NaHCO₃, brine and dried with MgSO₄. The residue is purified by flash chromatography using a gradient of methanol in CHCl₃. The resulting 1-[N-(methylaminothiocarbonyl)amino]-3-O-dimethoxytritylmethyl-2-propanol is phosphitylated with chloro-β-cyanoethoxy-N,N-diisopropylaminophosphine as per Example 20 to give the title compound.

### EXAMPLE 83

### N-α-(FMOC)-glutamic acid γ-benzyl ester.

To a solution of γ-benzyl glutamate (10 mmol) in 50 ml dioxane and 50 ml water is added triethylamine (25 mmol), followed by a solution of fluorenylmethyl chloroformate (11 mmol) in 50 ml dioxane. The mixture is vigorously stirred until the starting material is consumed. The solution is acidified to pH 2 with concentrated HCl, extracted with ethyl acetate (2 X 250 ml), washed with brine, dried with MgSO₄ and evaporated. The product is used without purification.

### EXAMPLE 84

### N-α-(FMOC)-γ-benzyl-L-glutamic acid fluorenylmethyl ester.

N-α-(FMOC)-glutamic acid γ-benzyl ester (5 mmol), fluorenylmethanol (5.5 mmol) and dimethylaminopyridine (0.5 mmol) are dissolved in 50 ml CH₂Cl₂. Dimethylaminopropyl ethyl carbodiimide (EDC, 6.0 mmol) is added, and the solution stirred at room temperature. After complete consumption of the starting material the solution is diluted with CH₂Cl₂, washed with 1% HCl, water and brine, dried with MgSO₄ and evaporated. The residue is purified by flash chromatography using ethyl acetate and hexane as eluant.

### EXAMPLE 85

### N-α-(FMOC)-L-glutamic acid α-fluorenylmethyl ester.

N-α-(FMOC)-γ-benzyl-L-glutamic acid fluorenylmethyl ester (5 mmol) is dissolved in methanol (50 ml) and 10% Palladium on carbon is added (100 mg catalyst/mmol). The suspension is shaken under 30 psi H₂ until the starting material is consumed. The suspension is filtered through a short pad of Celite, washed thoroughly with methanol, and the solvent evaporated. The product is used directly without purification.

### EXAMPLE 86

### 1-[N-(N-α-Fmoc-α-fluorenylmethyl-γ-glutamyl)amino]-3-O-dimethoxytritylmethyl-2-O-[(N,N-diisopropylamino)-2-cyanoethoxyphosphite]propane

The title compound is prepared by the procedure of Example 54 using N-α-(FMOC)-L-glutamic acid α-fluorenylmethyl ester as the carboxylic acid component.

### EXAMPLE 87

### 2-(N-Carbazolyl)acetic acid.

The title compound is prepared as per Example 63 using carbazole as the starting heterocycle.

### EXAMPLE 88

### 1-{N-[2-(N-Carbazolyl)acetyl]amino}-3-O-dimethoxytritylmethyl-2-O-[(N,N-diisopropylamino)-2-cyanoethoxyphosphite]propane

The title compound is prepared as per the procedure of Example 54 using N-carbazolyl-2-acetic acid as the carboxylic acid component.

### EXAMPLE 89

### N-Pyrrolyl-2-Acetic acid.

The title compound is prepared as per Example 63 using pyrrole as the starting heterocycle.

### EXAMPLE 90

### 1-{N-[2-(N-Pyrrolyl) acetyl]amino}-3-)-dimethoxytritylmethyl-2-O-[(N,N-diisopropylamino)-2-cyanoethoxyphosphite]propane

The compound is prepared as per the procedure of Example 54 using N-pyrrolyl-2-acetic acid as the carboxylic acid component.

### EXAMPLE 91

### 1-(1-Imidazole)-5-dimethoxytrityl-2-pentanol

1-Buten-4-ol is treated as per the procedure of Klunder, *et al., J. Org. Chem.* **1986,** *51*, 3710 to yield 4,5-epoxy-1-pentanol. The glycol is not isolated but is treated *in situ* in dry DMF with powdered potassium carbonate and imidazole as per the procedure of Example 4 followed by treatment with dimethoxytrityl chloride as per Example 5 to give the title compound.

### EXAMPLE 92

### 1-(-Imidazole) -5-O-dimethoxytrityl-2-O-[(N,N-diisopropylamino)-2-cyanoethoxyphosphite]pentane

1-(1-Imidazole)-4-dimethoxytrityl-2-pentanol is treated as per the procedure of Example 6 to give the title compound.

### EXAMPLE 93

### 1-(1-Carbazole)-8-dimethoxytrityl-2-octanol

1-Octen-8-ol is treated as per the procedure of Klunder, *et. al., J. Org. Chem*. **1986,** *51*, 3710 to yield 4,5-epoxy-1-octanol. The glycol is not isolated but is treated *in situ* in dry DMF with powdered potassium carbonate and imidazole as per the procedure of Example 4 followed by treatment with dimethoxytrityl chloride as per Example 5 to give the title compound.

### EXAMPLE 94

### 1-(-Carbazole)-8-O-dimethoxytrityl-2-O-[(N,N-diisopropylamino)-2-cyanoethoxyphosphite]octane

1-(1-Carbazole)-4-dimethoxytrityl-2-pentanol is treated as per the procedure of Example 6 to give the title compound.

### EXAMPLE 95

### 1-(N-Morpholino)-2,3-propandiol

A rapidly stirred solution of redistilled morpholine (4.3 g, 50 mmole) and R-(+)-glycidol (3.7 g, 50 mmole) in anhydrous methyl alcohol (50 mL) was heated at reflux for 18 hr. The resulting solution was cooled and rotary evaporated to yield a light yellow syrup. This syrup was kept under vacuum for 3 hr to afford a quantitative yield of the propandiol (8.0 g). ¹H-nmr (dmso-d₆): d, 4.5 (m, 2, hydroxyls); 3.5-3.3 (m, 7); 2.4-2.1 (m, 6).

### EXAMPLE 96

### 1-(N-Morpholino)-3-O-dimethoxytrityl-2,3-propandiol

To an ice cold solution of l-(N-morpholino)-2,3-propandiol (8.0 g, 50 mmole) in anhydrous pyridine (150 mL) and triethylamine (10 mL) was added powdered dimethoxytrityl chloride (17 g, 50 mmole) over a period of five minutes. This mixture was stirred at room temperature overnight and while maintaining anhydrous conditions. The resulting solution was rotary evaporated and then chromatographed on a 7.5 x 5 cm column using ethyl acetate/methyl alcohol/triethylamine (90/9.5/0.5) as eluant Yield of amber solid foam is 16 g (69 %). ¹H-nmr (dmso-d₆): d, 7.5-6.8 (m, 13, aromatic); 3.9 (s, 6, methoxy)10.4 and 5.7 (2 m, 1, hydroxyl); 4.2-2.8 (3 m, 13, aliphatic).

### EXAMPLE 97

### 1-(N-Morpholino-3-O-dimethoxytrityl-2-O-[(N,N-diisopropylamino)-2 -cyanoethoxyphosphite]-2,3-propanediol

Chloro-β-cyanoethoxy-N,N-diisoproylaminophosphine (2.4 g, 1 mmole) was added to an ice cold solution of 1-(N-morpholino-3-O-dimethoxytrityl-2,3-propandiol (4.3 g, 1 mmole) in anhydrous tetrahydrofuran (50 mL) and diisopropyl ethylamine (5 mL). The solution was stirred at room temperature for 3 hr and then rotary evaporated. The gum which resulted was chromatographed on a 5.5 x 10 cm silica gel column using ethyl acetate/methanol/triethylamine (95/4.5/0.5) as eluent. The appropriate fractions were evaporated to yield the amidite.

### EXAMPLE 98

### 1-(N-Piperidine)-2,3-propandiol

A rapidly stirred solution of piperidine (4.3 g, 50 mmole) and R-(+)-glycidol in anhydrous methanol (50 mL) was heated to reflux for 18 hr. The mixture was rotary evaporated to yield a clear syrup which was kept under vacuum for 3 hr to afford a quantitative yield of the propandiol (8 g). ¹H-nmr (dmso-d₆): d, 4.6 and 4.3 (2 m, 2, hydroxyls); 3.6 (m, 1, methine); 3.3 and 2.3 (2 m, 4, propane);1.4 (m, 10, piperidine).

### EXAMPLE 99

### 1-(N-Piperidine)-3-O-dimethoxytrityl-2,3-propandiol

To an ice cold solution of 1-(N-piperidine)-2,3-propandiol (6.8 g, 43 mmole) in pyridine (75 mL) and triethylamine (15 mL) was added dimethoxytrityl chloride(14.4 g, 43 mmole) over a period of five minutes. This mixture was stirred at room temperature overnight and while maintaining anhydrous conditions. The resulting solution was rotary evaporated and then chromatographed on a 11.5 x 5 cm column using ethyl acetate and then ethyl acetate/methyl alcohol/triethylamine (90/9.5/0.5) as eluant. The yield of yellow solid foam is 11 g (56 %). ¹H-nmr (dmso-d₆): d, 7.4-6.8 (m, 13, aromatic); 4.5 (m, 1, hydroxyl); 3.8 (s, 6, methoxy); 2.9 (d, 1, hydroxyl); 2.2 and 1.4 (2 m, 14, propane and piperidine)

### EXAMPLE 100

### 1-(N-Piperidine-3-O-dimethaxytrityl-2-O-[(N,N-diisopropylamino)-2 -cyanoethoxyphosphite]-2,3-propanediol

Chloro-β-cyanoethoxy-N,N-diisoproylaminophosphine (2.4 g, 1 mmole) was added to an ice cold solution of 1-(N-piperidine)-3-O-d imethoxytrityl-2,3-propandiol (4.6 g, 1 mmole) and diisopropyl-ethylamine (5 mL). The reaction mixture was stirred at room temperature for 3 hr and then rotary evaporated to afford a colorless syrup. This syrup was chromatographed on a 5.5 x 10 cm column using ethyl acetate/methanol/triethylamine (95/4.5/0.5) to afford the amidite.

### EXAMPLE 101

### 1-O-Dimethoxytrityl-5-hexen-1,2-diol

To a solution of 3-O-dimethoxytrityl-R-(+)-glycidol (8.0 g, 21 mmole) in anhydrous tetrahydrofuran (80 mL) at room temperature was added lithium tetrachlorocuprate (50 mL, 0.1 M solution in THF, Aldrich) followed immediately by allylmagnesium bromide (70 mL, 1 M solution in THF, Aldrich). The mixture was allowed to stir under an atomosphere of argon gas overnight and then quenched with the slow addition of a saturated aqueous solution of ammonium chloride (approx. 20 mL). The mixture was further stirred for 30 minutes and then filtered through Celite. The filter bed was washed with additional THF and the filtrates evaporated. The resulting dark oil was chromatographed on a 5.5 x 10 cm silica gel column using hexanes/ethyl acetate/triethylamine (90/9.5/0.5) and then (80/19.5/0.5) as eluent. Evaporation of the appropriate fractions afforded the diol as a colorless oil, 8.0 g (90 %). ¹H-nmr (dmso-d₆): d, 7.5-6.8 (m, 13, aromatic); 5.8 and 5.0 (2 m, 3, alkene); 4.7 (d, 1, hydroxyl); 3.7 (s, 6, methoxy); 3.6 (m, 1, methine); 2.9 (m, 2, allyl); 2.1 and 1.6 (2 m, 4, methylene).

### EXAMPLE 102

### 1-O-Dimethoxytrityl-2-O-[(N,N-diisopropylamino)-2-cyanoethoxypho sphite]-5-hexen-1,2-diol

Chloro-β-cyanoethoxy-N,N-diisoproylaminophosphine (2.4 g, 1 mmole) was added to an ice cold solution of 1-O-dimethoxytrityl-5-hexen-1,2-diol (4.2 g, 1 mmole) in tetrahydrofuran (80 mL) and diisopropyl-ethylamine (5 mL). The mixture was stirred at room temperature for 3 hr and then rotary evaporated. The resulting oil was chromatographed on a 5.5 x 10 cm silica gel column using hexanes/ethyl acetate/ triethylamine as eluent. The appropriate fractions were evaporated to afford the amidite.

### EXAMPLE 103

### 2-O-Benzyl-1-O-dimethoxytrityl-5-hexen-1,2-diol

To a solution of 1-O-dimethoxytrityl-5-hexen-1,2-diol (2.0 g, 4.8 mmole) in anhydrous dimethylformamide (15 mL) was added 60 % sodium hydride (0.28 g, washed with hexanes), followed immediately with benzyl bromide (0.82 g, 4.8 mmole). The mixture was stirred under an atomosphere of argon and at room temperature for 1 hr and then rotary evaporated. The resulting oil was partitioned between dichloromethane (150 mL) and cold, saturated sodium bicarbonate solution (50 mL). The organic layer was further washed with water, brine and then dried over sodium sulfate, filtered and rotary evaporated to afford a clear yellow oil. This material was filtered through a short bed of silica gel on a sintered glass funnel using ethyl acetate/hexanes (4/1) to afford a colorless oil, 2.0 g (82 %). ¹H-nmr (dmso-d₆): d, 7.5-6.8 (m, 18, aromatic); 5.8 and 4.9 (2 m, 3, alkene); 4.6 (q, 2, benzyl); 3.8 (s, 6, methoxy); 3.5 (m, 1, methine); 3.1, 2.0 and 1.6 (3 m, 6, methylene).

### EXAMPLE 104

### 2-O-Benzyl-1-O-dimethoxytrityl-hexan-1,2,6-triol

A solution of 2-O-benzyl-1-O-dimethoxytrityl-5-hexen-1,2-diol (1.6 g, 3.1 mmole) in anhydrous tetrahydrofuran (25 mL) was treated with a diborane/THF complex (6.3 mL of 1 M solution in THF) at room temperature. After frothing subsided (10 minutes from the addition of diborane), the mixture was further stirred under argon for 15 minutes. The mixture was treated with methanol (10 mL), stirred for 30 minutes at room temperature and then treated with NaOH solution (10 mL, 1 N) and stirred for an additional 30 minutes. The mixture was rotary evaporated to a minimal volume and the residue which resulted was redissolved in tetrahydrofuran (50 mL), then treated with 30 % hydrogen peroxide solution (6 mL). After 30 minutes, the biphasic solution was diluted with dichloromethane (100 mL) and the organic layer washed with cold water, cold, saturated sodium bicarbonate solution and brine. The organic layer was dried over sodium sulfate, filtered and evaporated. This material was chromatographed on a 3.5 x 15 cm silica gel column using hexanes/ethyl acetate/triethylamine (60/39.5/0.5) as eluent. The appropriate fractions were rotary evaporated to yield a colorless oil, 0.7 g (42 %). ¹H-nmr (dmso-d₆): d, 7.5-6.8 (m, 18, aromatic); 4.6 (q, 2, benzyl); 4.3 (t, 1, hydroxyl); 3.8 (s, 6, methoxy); 3.5 (m, 1, methine); 3.3, 3.1 and 1.3 (3 m, 10, methylene).

### EXAMPLE 105

### 6-O-Benzoyl-2-O-benzyl-1-O-dimethoxytrityl-1,2,6-hexantriol

A solution of 2-O-benzyl-1-O-dimethoxytrityl-1,2,6-hexantriol (0.60 g, 1.14 mmole) in anhydrous pyridine was treated with benzoyl chloride(0.24 g, 1.71 mmole) and the mixture allowed to stir overnight under an atmosphere of argon. The reaction mixture was treated with a few milliliters of methyl alcohol and rotary evaporated. The resulting oil was partitioned between dichloromethane (50 mL) and cold, saturated sodium bicarbonate solution (25 mL). The organic layer was further washed with cold water and brine, then dried over sodium sulfate, filtered and rotary evaporated. The resulting material was chromatographed on a 3.5 x 15 cm silica gel column using hexanes/ethyl acetate/triethylamine (80/19.5/0.5) as eluent. The appropriate fractions were evaporated to yield a colorless oil, 0.57 g (80 %). ¹H-nmr (CDCl₃) : d, 8.1-6.7 (3 m, 23 H, aromatic); 4.6 (q, 2, benzyl); 4.3 (t, 2, methylene); 3.8 (s, 6, methoxy); 3.6 (m, 1, methine); 3.2 (m, 2, methylene); 1.7 (m, 6, methylene).

### EXAMPLE 106

### 6-O-Benzoyl-1-O-dimethoxytrityl-1,2,6-hexantriol

A solution of 6-O-Benzoyl-2-O-benzyl-1-O-dimethoxytrityl-1,2,6-hexantriol (0.5 g, 0.8 mmole) in tetrahydrofuran was added a 10 % Pd/C catalyst (0.15 g) and the mixture placed under a balloon of hydrogen gas (2-3 lbs/in² for three days. The baloon was refilled and an equivalent portion of catalyst was added at the end of the first and second days, to a total of approximately 0.5 g. The mixture was filtered through Celite, the bed was washed with additional THF and the filtrates were rotary evaporated. The residue which resulted was chromatographed on a 3.5 x 10 cm silica gel column using hexanes/ethyl acetate/triethylamine (80/19.5/0.5) as eluent. The appropriate fractions were evaporated to afford 0.4 g (32%) of the triol as a colorless glass. ¹H-nmr (CDCl₃): d 8.1 (d, 2, aromatic); 7.6 to 6.2 (m, 12, aromatic); 6.8 (d, 4, aromatic); 4.3 (t, 2, bz-O-CH₂-); 3.8 (s, 6, methoxy); 3.2 (ddd, 2, CH₂-O-DMT); 2.4 (d, 1, hydroxyl); 1.9 to 1.4 (m, 6, CH₂).

### EXAMPLE 107

### 6-O-Benzoyl-1-O-dimethoxytrityl-1,2,6-hexantriol-2-O-[(N,N-diisop ropylamino)-2-cyanoethoxyphosphite]-1,2,6-hexantriol

Chloro-β-cyanoethoxy-N,N-diisoproylaminophosphine was added to an ice cold solution of 6-O-benzoyl-1-O-dimethoxytrityl-1,2,6-hexantriol in tetrahydrofuran and diisopropylethylamine. The mixture was stirred at room temperature for 3 hr and then rotary evaporated. The resulting oil was chromatographed on a 5.5 x 10 cm silica gel column using hexanes/ethyl acetate/ triethylamine as eluent. The appropriate fractions were evaporated to afford the amidite.

### EXAMPLE 108

### 1-O-Dimethoxytrityl-4-methylpentan-1,2-diol

A mixture of isopropylmagnesium bromide (50 mL of 2 M solution in THF, Aldrich) and dilithium tetrachlorocuprate (10 mL of 0.1 M solution in THF, Aldrich) was cooled to - 68 °C under an atmosphere of argon. The mixture was treated dropwise with a solution of 3-O-dimethoxytrityl-R-(+)-glycidol (7.3 g, 20 mmole, in 30 mL THF) so as to maintain this temperature. The amber solution was warmed to room temperature and then stirred overnight. The mixture was treated with cold saturated ammonium chloride solution (approx. 30 mL) and the filtered through Celite. The Celite bed was washed several times with additional tetrahydrofuran and the filtrates were rotary evaporated The resulting yellow syrup was chromatographed on a 5.5 x 15 cm silica gel column using hexanes/ethyl acetate/triethylamine as eluent. The appropriate fractions were evaporated to yield 5.2 g () of the diol as a colorless gum. ¹H-nmr (dmso-d₆): d, 7.5-6.8 (m, 13, aromatic); 4.6 (d, 1, hydroxyl); 3.7 (s, 6, methoxy); 3.7 (m, 1, C-2 methine); 2.9 (2 m, 2, methylene); 1.7 (m, 1, C-4 methine); 1.2 (t, 2, methylene); 1.9 (2 d, 6, methyls).

### EXAMPLE 109

### 1-O-Dimethoxytrityl-2-O-[(N,N-diisopropylamino)-2-O-cyanoethoxy-p hosphite]-4-methylpentan-1,2-diol

The N,N,N',N'-tetraisopropyl-2-cyanoethyldiphosphoramidite (3.3 g, 10.8 mmole) was added to an ice cold solution of 1-O-dimethoxytrityl-4-methylpentan-1,2-diol (3.8 g, 9 mmole) and diisopropylammonium tetrazolide (1.2 g, 4.5 mmole) in anhydrous acetonitrile (40 mL). This mixture was stirred overnight at room temperature and under an atmosphere of argon. The reaction mixture was evaporated to a minimal volume and the residue partitioned between dichloromethane (100 mL)and cold, saturated sodium bicarbonate solution (50 mL). The organic layer was further washed with cold water and brine and then dried over sodium sulfate, filtered and rotary evaporated. The syrup which resulted was chromatographed on a 5.5 x 15 cm silica gel column using hexanes/ethyl acetate/triethylamine (80/19.5/0.5) as eluent. The appropriate fractions were rotary evaporated to afford 5.4 g (80 %) of the amidite as a colorless glass. ³¹P-nmr (CD₃OD): 153.3, 152.9 ppm.

### EXAMPLE 110

### 1-(N,N-Bis-cyanoethylamino)-2,3-propandiol

A solution of R-(+)-glycidol (0.5 g, 6.7 mmole) in anhydrous acetonitrile (30 mL) and triethylamine (0.5 mL) was treated with 3,3'-iminodipropionitrile (0.825 g, 6.7 mmole). The mixture was heated to 50 °C and stirred under an atmosphere of argon for 18 hr. The mixture was rotary evaporated under vacuum pump to afford 1.0 g (76 %) of a light colorless oil. ¹H-nmr (dmso-d₆) : d, 4.9 (t, 1, 1° hydroxyl); 4.7 (d, 1, 2° hydroxyl); 3.7-3.2 (m, 5, propane); 2.7 and 2.6 (2 t, 8, ethylene).

### EXAMPLE 111

### 1-([N,N-Bis-cyanoethyl]amino)-3-O-dimethoxytrityl-propan-2,3-diol

A solution of 1-([N,N-bis-cyanoethyl]amino)-2,3-propandiol in anhydrous pyridine was treated with dimethoxytrityl chloride and the mixture stirred under anhydrous conditions for 18 hr. The mixture was treated with a few milliliters of anhydrous methanol and then rotary evaporated. The resulting oil was coevaporated with toluene and then chromatographed on a silica gel column to afford the tritylated compound.

### EXAMPLE 112

### 1-([N,N-Bis-cyanoethyl]amino)-3-O-dimethoxytrityl-2-O-[(N,N-diiso propylamino)-2-O-cyanoethoxyphosphite]-propan-2,3-diol

Chloro-β-cyanoethoxy-N,N-diisoproylaminophosphine was added to an ice cold solution of 1-([N,N-bis-cyanotheyl]amino)-3-O-dimethoxytrityl-propan-2,3-diol and diisopropylethylamine in anhydrous tetrahydrofuran. The mixture was stirred at room temperature and under an atmosphere of argon for 3 hr, then rotary evaporated. The resulting mixture was chromatographed on a silica gel column and the appropriate fractions collected and rotary evaporated to afford the phosphoramidite.

### EXAMPLE 113

### 1-(N,N'-Bis-carboxamidoethylamino)-3-O-dimethoxytrityl-propan-2,3 -diol

A solution of 1-([N,N-bis-cyanoethyl]amino)-3-0-dimethoxytrityl-propan-2,3-diol in tetrahydrofuran was treated with aqueous 1 M sodium hydroxide solution at room temperature. To the biphasic mixture was added 30 % hydrogen peroxide and then stirred at room temperature overnight. The resulting mixture was diluted with dichloromethane and the organic layer was further washed with cold, saturated sodium bicarbonate solution, water and brine. The organic layer was rotary evaporated and the resulting oil was filtered through a short bed of silica gel on a sintered glass funnel to afford the bis-carboxamide.

### EXAMPLE 114

### 1-([N,N'-Bis-carboxamidoethyl]amino)-3-O-dimethoxytrityl-2-O-[(N,N-diisopropylamino)-2-O-cyanoethoxyphosphite]-propan-2,3-diol

Chloro-β-cyanoethoxy-N,N-diisoproylaminophosphine was added to an ice cold solution of 1-([N,N'-bis-carboxamidoethyl]amino)-3-O-dimethoxytrityl-propan- 2,3-diol in anhydrous tetrahydrofuran and diisopropylethylamine. The resulting mixture was stirred at room temperature and under an atmosphere of argon for 3 hr, then rotary evaporated. The resulting mixture was chromatographed on a silica gel column and the appropriate fractions were collected and rotary evaporated to afford the phosphoramidite.

### EXAMPLE 115

### Standard Oligomer Coupling Cycle Using Standard DNA Synthesis Protocols

The oligomeric macromolecules of the invention are synthesized on an automated DNA synthesizer (Applied Biosystems model 380B) as is done with standard oligonucleotides using standard phosphoramidate chemistry with oxidation by iodine (see *Oligonucleotide synthesis, a practical approach,* M.J. Gait. Ed., Oxford University Press, New York, New York, **1990**). For phosphorothioate oligomers, the standard oxidation bottle is replaced by 0.2 M solution of 3H-1,2-benzodithiole-3-one 1,1-dioxide in acetonitrile for the step wise thiation of the phosphite linkages. The thiation wait step is increased to 68 sec and is followed by the capping step. After cleavage from the CPG column and deblocking in concentrated ammonium hydroxide at 55°C (18 hr), the oligomers are purified by precipitation twice out of 0.5 M NaCl solution with 2.5 volumes ethanol. Analytical gel electrophoresis is effected in 20% acrylamide, 8 M urea, 454 mM Tris-borate buffer, pH=7.0. Phosphodiester and phosphorothioate oligomers are judged from polyacrylamide gel electrophoresis as to material length.

### EXAMPLE 116

### Synthesis of Sequence Specific Ethylene Glycol Oligomer Having Phosphodiester Linkages.

"Aforvirsen" is an anti-papilloma agent having the nucleobase sequence: TTG CTT CCA TCT TCC TCG TC. An ethylene glycol phosphodiester linked oligomer of this preselected sequence is prepared using the T, A, C and G reagents from Examples 3, 15, 24 and 20, respectively, as per the procedure of Example 115 using iodine as the oxidation reagent to give the phosphodiester linked oligomeric compound having the "Aforvirsen" sequence.

### EXAMPLE 117

### Synthesis of Sequence Specific Ethylene Glycol Oligomer Having Phosphorothioate Linkages

"Aforvirsen" is an anti-papilloma agent having the nucleobase sequence: TTG CTT CCA TCT TCC TCG TC. An ethylene glycol phosphorothioate linked oligomer of this preselected sequence is prepared using the T, A, C and G reagents from Examples 3, 15, 24 and 20, respectively, as per the procedure of Example 115 using 3H-1,2-benzodithiole-3-one 1,1-dioxide as the oxidation reagent to give the phosphorothioate linked oligomeric compound.
¹ Froehler, B. C. In *Methods in Molecular Biology, vo120: Protocols for Oligonucleotides and Analogs*. Agrawal, S. Ed.; Humana Press: Totowa, 1993, 63-80.

### EXAMPLE 118

### Hydrogen phosphonate Coupling: General Procedure.¹

A portion of solid support (CPG or other polymeric support e. g. TentaGel) derivatized with a DMT protected alcohol linked via a succinate linker (1µmol) was loaded into a DNA synthesis column, and attached to an automated DNA synthesizer programmed to perform the following functions.
1) Wash with dichloromethane
2) DMT group was removed with 3% trichloroacetic acid in dichloromethane.
3) Wash with dichloromethane and CH₃CN/Pyridine (1:1).
4) Coupling: addition of alternating portions of 0.2 M Adamantoyl chloride or Pivaloyl chloride in CH₃CN/Py (1:1) and 0.05 M H-phosphonate monomer in CH₃CN/Py (1:1) for 1 min.
5) Wash with CH₃CN/Pyridine (1:1).
6) Repeat steps 1 through 5 until the full sequence is completed.

The product of this sequence of reactions is an H-phosphonate diester which is oxidized by one of several methods including those described below.

### Oxidation procedure 1: Phosphodiester

The solid support-bound H-phosphonate diester is treated (manually or automatically) with equal volumes of solution A (0.2 M I₂ in THF) and solution B (N-methylmorpholine/H₂O/THF 1:1:8) for 5 min, followed by equal volumes of solution A and solution C (TEA/H₂O/THF 1:1:8) for 5 min, followed by washing with CH₃CN/Py (1:1).

### Oxidation procedure 2: Phosphorothioate

The solid support-bound H-phosphonate diester is treated (manually or automatically) with a solution of S₈ in CS₂/Lutidine for 30 min. The solid support is then washed with CH₃CN/Py (1:1).

### Oxidation procedure 3: Phosphoramidate

The solid support-bound H-phosphonate diester is treated (manually or automatically) with a solution of the required amine (10 % V/V) in CCl₄/Pyridine 1:1 for 15-30 min. The solid support is then washed with CH₃CN/Py (1:1).

### Synthesis of Phosphoranidate Library with Uniform PN-Substitution, X_{PN}(N_{PN})ₙN

The library is synthesized on Ethylene glycol CPG or on Universal Support CPG (Zeneca, Cambridge, UK) using an automated DNA synthesizer. The solid support is separated into a number of portions equal to the number of monomers used. Each monomer is coupled to the support using the H-phosphonate coupling general procedure. The portions are mixed and redivided for the next coupling. In this way, the random oligomers are synthesized until the fixed position is reached. The support is again split into portions and each monomer coupled. Additional monomers can be added after the fixed position by performing the couplings to the individual pools. Additional random positions can be added to the oligomer by further splitting each of the pools and recombining after coupling, taking care not to mix pools with different fixed positions. Once the oligomer assembly is complete, the H-phosphonate linkages in the individual subsets need to be oxidized, following general procedure 3. This has the result of producing the same phosphoramidate linkage between each residue in the library. By following oxidation general procedure 1 or 2 instead, an all phosphodiester or all phosphorothioate library is produced. The library pools are then cleaved from the solid support, purified and assayed for activity as described previously.

### Synthesis of Phoaphoramidate Library with fixed PN-Substitution, X_{PN1}-N_{PN2}-(N_{PNn})ₘ-N

Synthesis of a phosphoramidate library using 2-substituted-DMT-ethylene glycol-H-phosphonate monomers (P-Amidate: EGP, Variable amines, X_{PN1}-N_{PN2}-(N_{PNn})ₘ-N). The library is synthesized on Ethylene glycol CPG or on Universal Support CPG (Zeneca, Cambridge, UK) using an automated DNA synthesizer. The solid support is separated into a number of portions equal to the number of monomers used (X). Each monomer is coupled to the support using the H-phosphonate coupling general procedure. The portions are recombined, and the H-phosphonate diester linkage is converted to a phosphoramidate using Oxidation procedure 3. These steps are repeated (split, couple, combine), followed by Oxidation procedure 3 using a different amine for each randomized position in the oligomer. At the fixed position, the support is split, the monomers are coupled, but the subsets are kept separate for the oxidation step. After cleavage and workup, one obtains a library with randomized monomers and a different substituent at each phosphoramidate linkage. It is also possible to introduce phosphodiester and phosphorothioate linkages at any position in the oligomer library by substituting the appropriate oxidation procedure.

### Synthesis of Phoaphoramidate Library with Variable PN-Substitution, X_{PNn}-(N_{PNn})ₘ-N

The library is synthesized on Ethylene glycol CPG or on Universal Support CPG (Zeneca, Cambridge, UK) using an automated DNA synthesizer. The solid support is separated into a number of portions equal to the number of monomers used. Each monomer is coupled to the support using the H-phosphonate coupling general procedure. The portions are recombined, mixed and separated into portions. Each portion is oxidized with a different amine following oxidation procedure 3, followed by combining and mixing of the supports. These steps (split, couple, mix, split, oxidize, mix) are repeated until the fixed position. The support is then split into portions, the monomer coupled, and the resin portions split again for oxidation following procedure 3. After workup, each subset has a unique monomer residue and phosphoramidate linkage, and an equimolar amount of each monomer and amine in the randomized portion. It is also possible to incorporate phosphodiester and phosphoramidate linkages randomly into the oligomers by oxidizing a portion of each H-phosphonate diester by following procedure 1 or 2.

### EXAMPLE 119

### Preparation of 3-Octyl-propan-1,2-diol-pbosphodiesterthymidine-phosphodiester-thymidine Trimer

A controlled pore glass resin derivatized with a 3'-5' phosphodiester linked dimer of thymidine [5'-Dmt-o-T-o-p(=o)-o-T-CPG] was synthesized in a standard manner. The resin (25.6 mg, 47.5 mmole/g resin) was detritylated using 3 % trichloroacetic acid in dichloromethane and then sequentially washed with anhydrous acetonitrile and pyridine. The resin was vigorously agitated with a solution of the 3-octyl-1-O-dimethoxytritylpropandiol-2-H-phosphonate (25 mM) in adamantoyl chloride (50 mM) and pyridine for five minutes. The resin was then washed repeatedly with anhydrous pyridine and blown dry with argon gas. 1 mL of iodine/pyridine/water (2/90/8) solution was added to the column and the column was agitated vigorously. The column was subsequently washed with pyridine and acetonitrile and then blown dry with argon gas. Products were cleaved from the CPG by treatment with 1 mL concentrated ammonium hydroxide for 30 minutes. The ammonia solution was evaporated to dryness and then treated with 80 % acetic acid in water for 30 minutes to remove the trityl group. An aliquot of the solution exhibited a single peak at 26.3 min by an HPLC analysis (Vyadec C-18 column, monitor at 260 nm, linear gradient of ammonium acetate buffer (pH 7)-acetonitrile, 0-75 % acetonitrile in 52 minutes) corresponding to the phosphodiester trimer.

### Example 120

### Preparation of 3-Octyl-propan-1,2-diol-morpholinophosphoramidate-thymidine-phosphodiester-thymidine Trimer

A 1-O-dimethoxytrityl-3-octyl-propandiol-2-O-P(H)-O-T-O-P(=O)-O-T-CPG resin prepared as described in Examples 118 and 119 is vigorously shaken with 1 mL of a solution of morpholine/carbon tetrachloride/pyridine (1/5/5) for 30 minutes. The resin is sequentially washed with pyridine and then acetonitrile and then blown dry with argon gas. Products are cleaved from the CPG by treatment with 1 mL concentrated ammonium hydroxide for 30 minutes. The ammonia solution is evaporated to dryness and then treated with 80 % acetic acid in water for 30 minutes to remove the trityl group. An aliquot of this solution is used for HPLC analysis of the resulting morpholinophosphoramidate trimer.

### Example 121

### Preparation of 3-(N-Piperidine)-propan-1,2-diolphosphodiester-thymidine-phosphodiester-thymidine Trimer.

A controlled pore glass resin derivatized with a 3'-5' phosphodiester linked dimer of thymidine [5'-Dmt-o-T-o-p(=o)-o-T-CPG] was synthesized in a standard manner. The resin (24.6 mg, 47.5 mmole/g resin) was detritylated using 3 % trichloroacetic acid in dichloromethane and then sequentially washed with anhydrous acetonitrile and pyridine. The resin was vigorously agitated with a 1 mL solution of the 1-(N-piperidine)-3-O-dimethoxytritylpropandiol-2-H-phosphonate(25 mM) in adamantoyl chloride (50 mM) and pyridine for five minutes. The resin was then washed repeatedly with anhydrous pyridine and blown dry with argon gas. The column was added 1 mL of iodine/pyridine/water (2/90/8) solution and agitated vigorously. The column was subsequently washed with pyridine and acetonitrile and then blown dry with argon gas. Products were cleaved from the CPG by treatment with 1 mL concentrated ammonium hydroxide for 30 minutes. The ammonia solution was evaporated to dryness and the residue treated with 80 % acetic acid in water for 30 minutes to remove the trityl group An aliquot of the solution exhibited a single peak at 12.4 min by an HPLC analysis under the conditions described before.

### EXAMPLE 122

### Preparation of 1-N-Piperidine-3-O-dimethoxytrityl-propandiol-2-morpholinophosphoramidate-thymidine-phosphodiester-thymidine Trimer

A 3-O-dimethoxytrityl-1-N-piperidine-propandiol-2-O-P(H)-O-T-O-P(=O )-O-T-CPG resin prepared as described in Examples 118 and 119 is vigorously shaken with 1 mL of a solution of morpholine/ carbon tetrachloride/ pyridine (1/5/5) for 30 minutes. The resin is sequentially washed with pyridine and then acetonitrile and then blown dry with argon gas. Products are cleaved from the CPG by treatment with 1 mL concentrated ammonium hydroxide for 30 minutes. The ammonia solution is evaporated to dryness and then treated with 80 % acetic acid in water for 30 minutes to remove the trityl group. An aliquot of this solution is used for HPLC analysis of the resulting morpholinophosphoramidate trimer.

### EXAMPLE 123

### Incorporation Of Monomeric Units Into Oligomeric Structure Using Combinatorial Technique - General Procedure

A solid support (universal support from Cambridge Research Biochemicals) is separated into portions of equal weight. The number of portions equals the number of monomers in the combinatorial library. Each portion is reacted with one of the desired amidites using tetrazole as catalyst, followed by oxidation of the phosphite triester to the phosphate as per the standard coupling cycle of Example 115. The DMT ether is cleaved using trichloroacetic acid in CH₂Cl₂ to regenerate the hydroxyl group at the end of the extended oligomer. The extent of the coupling reaction is optimized to be ≥90% completed by varying the amidite concentration and total equivalents and the coupling time. After a coupling, the supports from individual coupling reactions are mixed thoroughly, then divided equally and amidites are again reacted individually to a portion of the support. This cycle is repeated for each random position until the 'fixed' position is reached.

At the 'fixed' position of the oligomer, each amidite is reacted individually to a portion of the support, but the portions are not mixed. Instead each subset is further divided into the number of portions corresponding to the number of monomers. Each portion of support is then reacted with a different amidite, followed by mixing as above. Repeating this cycle for each of the different subsets of supports results in randomization in positions following the fixed position in the sequence. The resulting subsets are unique only in the fixed position.

At completion of the oligomer synthesis, the oligomers are cleaved from the solid support and phosphate protecting groups removed by incubation for 1-2 hours at room temperature in concentrated ammonia. The supernatant containing the oligomer is then removed from the silica and incubated at 55°C for 6-16 hours to cleave the protecting groups from the residues. The oligomer is desalted and protecting groups are removed by HPLC size exclusion chromatography.

### EXAMPLE 124

### Evaluation Of Coupling Efficiency Of Phosphoramidite Monomers

The following method is used to evaluate the ethylene glycol phosphoramidites or other phosphoramidites for suitability of use in a random sequence solid state oligomer synthesis. A solid-phase synthesis support containing an internal reference is used to determine coupling efficiency, estimate the extinction coefficient, and evaluate coupling-product quality of the test phosphoramidite monomers as follows:

A test monomer-support is selected as is an internal standard. Using dT as a symbol of thymidine, dC as a symbol for deoxy cytidine and other abbreviations as note in the text below, in an illustrative test system, thymidine bound to CPG, identified as dT-CPG, is used for the test monomer-support and 5'-O-acetyl capped cytosine bound to CPG, identified as 5'-Ac-dC-CPG, is used for the internal standard.

Reactive dT-CPG is mixed with a lesser molar equivalent of unreactive 5'-Ac-dC-CPG. The unreactive 5'-Ac-dC-CPG internal standard allows for accurate determination of unreacted dT present before and after a coupling reaction.

The peak area of dT can be identified as A_{T} and the peak area of dC identified as A_{C}. The initial ratio of peak areas for dT and dC, *i.e*., (A_{T}/A_{C})₀, is determined by cleavage, deprotection, and HPLC analysis of an aliquot of the CPG mixture. Measurements are taken at a wavelength of 260 nm. Relative moles of dC can be identified as C, and relative moles of dT can be identified as *T*. These are calculated from peak areas, A_{C} and A_{T}, respectively, using known extinction coefficients: C = A_{C}/ε_{C} and T = A_{T}/ε_{T}. Thus the relative peak area or molar amount of dT initially present can always be calculated from the peak area of dC:${\text{A}}_{\text{T0}} {\text{= (A}}_{\text{C}} {\text{) [(A}}_{\text{T}} {\text{/A}}_{\text{C}} {\text{)}}_{\text{0}} \text{]}$${\text{T}}_{\text{0}} {\text{= (C)[(T/C)}}_{\text{0}} \text{]}$ also,${\text{(C) (T/C) = (A}}_{\text{C}} {\text{/ε}}_{\text{C}} {\text{) [(A}}_{\text{T}} {\text{/ε}}_{\text{T}} {\text{)/ (A}}_{\text{C}} {\text{/ε}}_{\text{C}} \text{)]} {\text{= (A}}_{\text{C}} {\text{/ε}}_{\text{C}} {\text{) (A}}_{\text{T}} {\text{/A}}_{\text{C}} {\text{)) (ε}}_{\text{C}} {\text{/ε}}_{\text{T}} \text{)}$ thus,${\text{(C)[(T/C)}}_{\text{0}} {\text{] = (A}}_{\text{C}} {\text{/ε}}_{\text{C}} {\text{)) [(A}}_{\text{T}} {\text{/A}}_{\text{C}} {\text{)}}_{\text{0}} {\text{] (ε}}_{\text{C}} {\text{, ε}}_{\text{T}} \text{)} {\text{= (A}}_{\text{C}} {\text{/ε}}_{\text{T}} {\text{) [(A}}_{\text{T}} {\text{/A}}_{\text{C}} {\text{)}}_{\text{0}} \text{]}$ An amidite monomer of interest, identified as X, is reacted with an aliquot of the CPG mixture. Reacted CPG is cleaved and deprotected with ammonia, then analyzed by HPLC to determine: the area under the peak for dC, *i.e.*, A_{C}; the area under the peak for unreacted dT, *i.e.*, A_{Tur}; and the area under the peak for X-T dimer, *i.e.*, A_{XT}. These values are used to calculate coupling efficiency, *C.E.,* and *X-T* dimer extinction coefficient ε_{XT}.

The coupling efficiency, *C.E.,* is defined by the ratio of reacted dT, *i.e.*, Tᵣ, to total dT, *i.e.*, T₀. Thus *C.E*. = Tᵣ/T₀. Coupling efficiency can be determined from the relative moles of unreacted dT present before, *i.e.*, T₀, and after, *i.e.*, Tᵤᵣ, coupling with X; all three are related by the equation:${\text{T}}_{\text{0}} {\text{= T}}_{\text{r}} {\text{+ T}}_{\text{ur}} \text{.}$

Since *C.E*. is a unit-less value, HPLC peak areas can be used instead of relative molar quantities to perform the calculation:$\text{C.E.} {\text{= (T}}_{\text{r}} {\text{/T}}_{\text{0}} \text{)} {\text{= (T}}_{\text{0}} {\text{/T}}_{\text{0}} {\text{) - (T}}_{\text{ur}} {\text{/T}}_{\text{0}} \text{)} {\text{= 1 - (T}}_{\text{ur}} {\text{/T}}_{\text{0}} \text{)} {\text{= 1-(A}}_{\text{Tur}} {\text{/ε}}_{\text{T}} {\text{)/(A}}_{\text{T0}} {\text{/ε}}_{\text{T}} \text{)} {\text{= 1 - (A}}_{\text{Tur}} {\text{/A}}_{\text{T0}} \text{)} {\text{= 1 - (A}}_{\text{Tur}} {\text{)/[(A}}_{\text{C}} {\text{) [(A}}_{\text{T}} {\text{/A}}_{\text{C}} {\text{)}}_{\text{0}} \text{]]}$ The foregoing are all measurable quantities.

The extinction coefficient ε for X, *i.e*., ε_{*X-T*}*,* in the given HPLC solvent system is determined from the *C.E.* for X and the relative areas of the HPLC peaks. The amount of X-T is equal to the amount of T that has reacted. ε for dimer X-T is defined as the peak area A_{XT} divided by the moles of X-T dimer present XT, and is calculated as follows:${\text{XT = T}}_{\text{r}} \text{= (} \text{C.E} {\text{.) (T}}_{\text{0}} \text{)}$${\text{ε}}_{\text{XT}} {\text{= (A}}_{\text{XT}} \text{/XT)} {\text{= (A}}_{\text{XT}} \text{)/(} \text{C.E} {\text{.) (T}}_{\text{0}} \text{)} {\text{= (A}}_{\text{XT}} \text{)/(} \text{C.E} {\text{.) (C) [(T/C)}}_{\text{0}} \text{]} {\text{= (A}}_{\text{XT}} \text{)/(} \text{C.E} {\text{.) (A}}_{\text{C}} {\text{/ε}}_{\text{T}} {\text{) [(A}}_{\text{T}} {\text{/A}}_{\text{C}} {\text{)}}_{\text{0}} \text{]}$ The foregoing are all measurable quantities.

Finally, the quality of the coupling-product X-T can be evaluated from the appearance of the HPLC chromatogram. Significant peaks (those summing >10% of product-peak area) other than those expected might also be addressed. Often they are the desired X-T dimer that retains protective groups. Disappearance of these peaks with extended ammonia treatment will confirm that the monomer requires extended ammonia deprotection beyond the standard time. In other cases the extra peaks can be identified as undesirable side-products or in some case they cannot be identified. Generally, coupling efficiency of less than about 90%, a required ammonia deprotection time of greater than a few days, or the occurrence of side-products amounting to greater that 10% (by UV absorbance) can be selected as initial guidelines to judge the possibility of excluding an amidite from consideration for use in a particular set of amidites used in generating random oligomeric compounds.

### EXAMPLE 125

### Evaluation Of Coupling Efficiency Of Illustrative Ethylene glycol Phosphoramidite Monomer

Ethylene glycol phosphoramidites are coupled to a dT-CPG solid-phase synthesis support forming dimers and trimers. Coupling is effected as per the general procedure of Example 124 Synthesis of the dimers and trimers is performed with an ABI 394 DNA synthesizer (Applied Biosystems, Foster City, CA) using standard DNA synthesis reagents and synthesis protocols, with the exception of an extended (5 minute) coupling time added to the synthesis cycle. The oligomers are cleaved from solid support by treatment with concentrated ammonia for 3 days at 4°C. The supernatant is removed from the support and heated in a sealed vial at 55°C for eight hours. This solution is cooled, and the ammonia removed by evaporation. The oligomers are analyzed directly on reversed-phase HPLC column (Waters Nova-Pak Phenyl, cat. #10656; Millipore Corp., Milford, MA) using a gradient of 1% to 75% acetonitrile in 0.1 M ammonium acetate, pH 7, over 50 minutes. The HPLC system is a Waters with a 991 detector, 625 LC pump, and 714 WISP autoinjector. Calculations are performed using data collected at a wavelength of 260 nm.

### EXAMPLE 126

### Synthesis of Ethylene Glycol Phosphate Oligomeric Library

Ethylene glycol phosphate oligomers (EGP) were synthesized using standard DNA phosphoramidite chemistry. The following six EGP phosphoramidites were incorporated into oligomeric library: adenine, guanine, cytosine, thymine, carbazole and imidazole. EGP phosphoramidites were dissolved in anhydrous acetonitrile (CH₃CN) to 0.2 M, with the exception of EGP guanine ("egG") which was first dissolved in anhydrous dimethylformamide (DMF) to 2 M and then further diluted to 0.2 M with CH₃CN. EGP phosphoramidites were coupled to a 1-2 µmol controlled pore glass (CPG) support on an ABI 394 DNA synthesizer using the standard ABI cycle for a 1 µmol scale cyanoethyl phosphoramidite synthesis with the coupling wait time increased to 5 minutes. By substituting a 0.1 M solution of 3H-1,2-benzodithiol-3-one 1,1-dioxide (Beaucage) in acetonitrile for the iodine oxidizing solution, phosphorothioate oligomers were also synthesized. The 5' dimethoxytrityl group (DMT) of an oligomer terminating in a 5' EGP residue was not removed prior to cleaving the oligomer from the CPG support. Detritylating the 5' hydroxyl before cleavage in ammonium hydroxide (NH₄OH) results in the hydrolysis of the 5'EGP residue as a cyclic phosphate.

EGP oligomers were cleaved from CPG in 1 ml 30% NH₄OH for 1 hour at room temperature. Cleaved oligomer was deprotected in NH₄OH at 55°C overnight (typically 12-18 hours). NH₄OH was then removed from oligomer by evaporation in the Savant speedvac. The 5' hydroxyl was deprotected by cleaving the DMT from the oligomer in 1 ml 80% acetic acid (HOAc) for 1 hour. HOAc was then removed from oligomer by evaporation in the speedvac.

The oligomers were resuspended in H₂O and further purified by one or more of three methods of Example 127

### EXAMPLE 127

### General Oligomer Purification Procedures

### Procedure A - reverse phase HPLC chromatography

To desalt oligomers by reverse phase chromatography, the oligomer was loaded onto a reverse phase column in approx. 100 mM ammonium acetate (NH₄OAc) or sodium acetate (NaOAc). The column was washed with several column volumes (10-20) of H₂O to desalt. The oligomer was eluted in a gradient of 0% to 100% methanol.

### Procedure B - size exclusion chromatography

To desalt by size exclusion, the oligomer was loaded onto a size exclusion column, typically Sephadex G10 or G25 1.6 x 50 cm, that is connected to the HPLC and eluted in H₂O at a flow rate of 0.5 ml/min. The oligomers elute from the column in approximately 2-3 hours followed by salts and protecting groups.

### Procedure C - ethyl acetate extraction

The oligomers may also be purified from the protecting groups by ethyl acetate extraction; however, this method does not desalt the oligomer. After DMT cleavage with HOAc, the dried oligomer was resuspended in 1-2 ml H₂O. Ethyl acetate (1 ml) was added to the oligomer and vortexed to mix layers. After the H₂O and ethyl acetate layers separate, the top layer, which is the ethyl acetate layer containing DMT and benzamide protecting groups, was removed from the H₂O layer which contains, the oligomer. Three extractions with ethyl acetate are sufficient to remove the DMT and benzamide from the oligomer.

After purification by chromatography or extraction, the oligomer was dried by evaporation in the speedvac. The oligomer was resuspended in H₂O, typically at 0.5-2 mM, and stored at ≤-20°C.

## Claims

1. A compound having structure I: 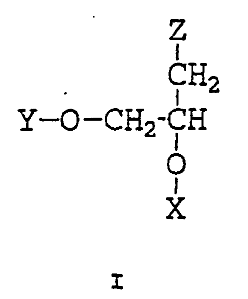 wherein:
X is an activated phosphate group, an activated phosphite group, or a solid support;
Y is a hydroxyl protecting group; and
Z is a purine, pyrimidine, nitrogen-containing heterocycle, or C₁ through C₁₀ straight or branched alkyl.

2. The compound of claim 1 wherein X is an activated phosphite group or a solid support.

3. The compound of claim 1 wherein X is an activated phosphite group and Z is adenine, guanine, cytosine, uridine or thymine.

4. The compound of claim 1 wherein Z is C₁ through C₁₀ straight or branched alkyl.

5. A compound having structure II: 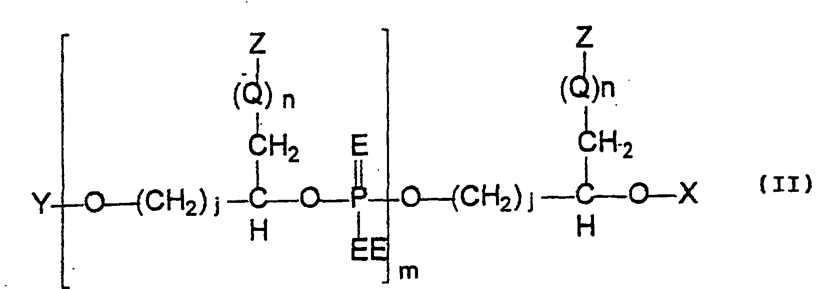 wherein:
X is H, a phosphate group, an activated phosphate group, an activated phosphite group, a solid support, a conjugate group, or an oligonucleotide;
Y is H, a hydroxyl protecting group, a conjugate group or an oligonucleotide;
E is O or S;
EE is O⁻ or N(Y₀)T₀, provided that at least one EE is N(Y₀)T₀;
Y₀ is H, or [Q₂]ⱼⱼ-Z₂;
T₀ is [Q₁]ₖₖ-Z₁, or together Y₀ and T₀ are joined in a nitrogen heterocycle;
Q₁ and Q₂ independently, are C₂-C₁₀ alkyl, C₂-C₁₀ alkenyl, C₂-C₁₀ alkynyl, C₄-C₇ carbocylo alkyl or alkenyl, a heterocycle, an ether having 2 to 10 carbon atoms and 1 to 4 oxygen or sulfur atoms, a polyalkyl glycol, or C₇-C₁₄ aralkyl;
jj and kk independently, are 0 or 1;
Z₁ and Z₂, independently, are H, C₁-C₂₀ alkyl, C₂-C₂₀ alkenyl, C₂-C₂₀ alkynyl, C₆-C₁₄ aryl, C₇-C₁₅ aralkyl, a halogen, CH=O, OR₁, SR₂, NR₃R₄, C (=NH) NR₃R₄, CH(NR₃R₄), NHC(=NH)NR₃R₄, CH (NH₂) C (=O) OH, C(=O)NR₃R₄, C(=O)OR₅, a metal coordination group, a reporter group, a nitrogen-containing heterocycle, a purine, a pyrimidine, a phosphate group, a polyether group, or a polyethylene glycol group;
Z is L₁, L₁-G₁, L₂, L₂-G₂, NR₃R₄, a nitrogen-containing heterocycle, a purine, a pyrimidine, a phosphate group, a polyether group, or a polyethylene glycol group, provided that at least two of Z and Zₘ are different moieties;
L₁ is alkyl having 1 to about 20 carbon atoms, alkenyl having 2 to about 20 carbon atoms, or alkynyl having 2 to about 20 carbon atoms;
L₂ is aryl having 6 to about 14 carbon atoms or aralkyl having 7 to about 15 carbon atoms;
G₁ is halogen, OR₁, SR₂, NR₃R₄, C(=NH)NR₃R₄, NHC(=NH)NR₃R₄, CH=O, C(=O)OR₅, CH(NR₃R₄) (C(=O)OR₅), C(=O)NR₃R₄, a metal coordination group, or a phosphate group;
G₂ is halogen, OH, SH, SCH₃, or NR₃R₄;
R₁ is H, alkyl having 1 to about 6 carbon atoms, or a hydroxyl protecting group;
R₂ is H, alkyl having 1 to about 6 carbon atoms, or a thiol protecting group;
R₃ and R₄ are, independently, H, alkyl having 1 to about 6 carbon atoms, or an amine protecting group;
R₅ is H, alkyl having 1 to about 6 carbon atoms, or an acid protecting group;
Q is L₁, G₃, L₁-G₃ or G₃-L₁-G₃;
G₃ is NR₃, C(=O), C(=S), C(O)-O, C(O)-NH, C(S)-O, C(S)-NH or S(O)₂, NR₃C(=O), NR₃C(=S), NR₃C(O) -O, NR₃C(O)-NH, NR₃C(S) -O, NR₃C(S) - NH or NR₃S(O)₂;
n is 0 or 1;
j is 1 to 6; and
m is 1 to 50.

6. The compound of claim 5 wherein Y is a hydroxyl protecting group.

7. The compound of any preceding claim wherein Y is trityl, methoxytrityl, dimethoxytrityl or trimethoxytrityl.

8. The compound of claim 5 wherein X is H, an activated phosphite group, or a solid support.

9. The compound of any preceding claim wherein X is a phosphoramidite.

10. The compound of claim 5 wherein n is 1 and Q and each Qₘ, independently, are carbonyl, thiocarbonyl, carboxy, acetyl, amido, succinyl, carbamoyl, thiocarbamoyl, ureido, thioureido, or sulfonamido acyl group.

11. The compound of claim 1 or 5, wherein Z and each Zₘ are nitrogen-containing heterocycles.

12. The compound of claim 11 wherein said nitrogen-containing heterocycle is imidazole, pyrrole or carbazole.

13. The compound of claim 12 wherein Z and each Zₘ, independently, are imidazole or carbazole.

14. The compound of claim 1 or 5, wherein Z and each Zₘ independently, are purines or pyrimidines.

15. The compound of claim 14 wherein Z and each Zₘ, independently, are adenine, guanine, cytosine, uridine or thymine.

16. The compound of claim 15 wherein n is 0.

17. The compound of claim 5, wherein Z and each Zₘ, independently, are alkyl having 1 to about 20 carbon atoms.

18. The compound of claim 5 wherein Z and each Zₘ, independently, are C₁-C₆ alkyl-NH₂.

19. The compound of claim 5 wherein Z and each Zₘ, independently, are aryl having 6 to about 14 carbon atoms or aralkyl having 7 to about 15 carbon atoms.

20. The compound of claim 19 wherein Z and each Zₘ, independently, are carbazole, phenyl or benzyl.

21. The compound of claim 5 wherein Z and each Zₘ, independently, are glutamyl.

22. The compound of claim 5 wherein m is 1 to about 25.

23. The compound of claim 5 wherein Eₘ is O.

24. The compound of claim 5. wherein each Eₘ is S.

25. The compound of claim 5. wherein Z and each Zₘ of said compound are in a predetermined sequence.

26. The compound of claim 5 wherein Z and each Zₘ of said compound are in a random sequence.

27. A process for preparing an oligomeric compound having structure II as defined in claim 5, comprising :
selecting a group of monomers, each of said monomers having structure III 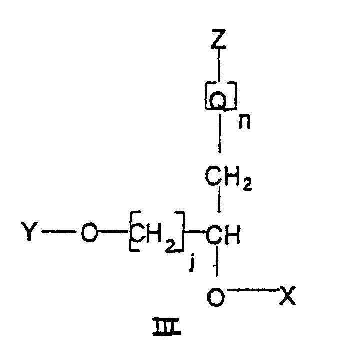 wherein:
X is H, a phosphate group, an activated phosphate group, an activated phosphite group, or a solid support;
Y is H or a hydroxyl protecting group;
Z is L₁, L₁-G₁, L₂, L₂-G₂, NR₃R₄, a nitrogen-containing heterocycle, a purine, a pyrimidine, a phosphate group, a polyether group, or a polyethylene glycol group;
L₁ is alkyl having 1 to about 20 carbon atoms, alkenyl having 2 to about 20 carbon atoms, or alkynyl having 2 to about 20 carbon atoms;
L₂ is aryl having 6 to about 14 carbon atoms or aralkyl having 7 to about 15 carbon atoms;
Gᵢ is halogen, OR₁, SR₂, NR₃R₄, C(=NH)NR₃R₄, NHC(=NH)NR₃R₄, CH=O, C(=O)OR₅, CH(NR₃R₄) (C(=O)OR₅), C(=O)NR₃R₄, a metal coordination group, or a phosphate group;
G₂ is halogen, OH, SH, SCH₃, or NR₃R₄;
R₁ is H, alkyl having 1 to about 6 carbon atoms, or a hydroxyl protecting group;
R₂ is H, alkyl having 1 to about 6 carbon atoms, or a thiol protecting group;
R₃ and R₄, independently, are H, alkyl having 1 to about 6 carbon atoms, or an amine protecting group;
R₅ is H, alkyl having 1 to about 6 carbon atoms, or an acid protecting group;
Q is L₁, G₃, L₁-G₃ or G₃-L₁-G₃;
G₃ is NR₃, C(=O), C(=S), C(O)-O, C(O)-NH, C(S)-O, C(S)-NH or S(O)₂, NR₃C(=O), NR₃C(=S), NR₃C(O)-O, NR₃C(O)-NH, NR₃C(S)-O, NR₃C(S)-NH or NR₃S(O)₂;
n is 0 or 1;
j is 1 to 6; and
covalently bonding at least two of said monomers of said group to form said oligomeric compound.

28. The process of claim 27 wherein the Z moiety of at least one monomer of said group is different from the Z moiety of another monomer of said group.

29. The process of claim 27 wherein m is from 1 to 40.

30. An oligomeric compound prepared via the process of claim 27 wherein m is from 1 to 25.

31. A chimeric oligomeric compound having a central region comprising a phosphodiester or a phosphorothioate oligodeoxynucleotide interspaced between flanking regions having structure II: 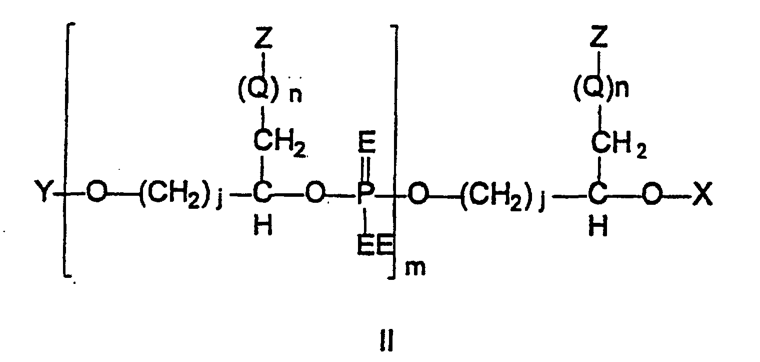 wherein X,Y,Z,Q,E,EE,n,m and j are as defined in claim 5, but without the proviso that at least two of Z and Zₘ are different moieties.

## Patentansprüche

1. Verbindung mit einer Struktur I: 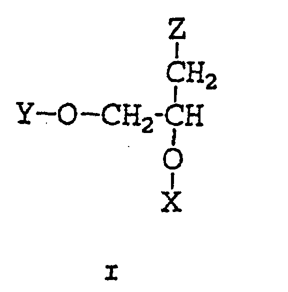 worin:
X eine aktivierte Phosphat-Gruppe, eine aktivierte 5-Phosphit-Gruppe oder ein fester Träger ist;
Y eine Hydroxyl-Schutzgruppe ist; und
Z ein Purin, ein Pyrimidin, ein Stickstoff-haltiger Heterocyclus, oder ein C₁ bis C₁₀ geradkettiges oder verzweigtes Alkyl ist.

2. Verbindung gemäß Anspruch 1, worin X eine aktivierte Phosphit-Gruppe oder ein fester Träger ist.

3. Verbindung gemäß Anspruch 1, worin X eine aktivierte Phosphit-Gruppe ist und Z Adenin, Guanin, Cytosin, Uridin oder Thyrmin ist.

4. Verbindung gemäß Anspruch 1, worin Z ein C₁ bis C₁₀ geradkettiges oder verzweigtes Alkyl ist.

5. Verbindung mit einer Struktur II: 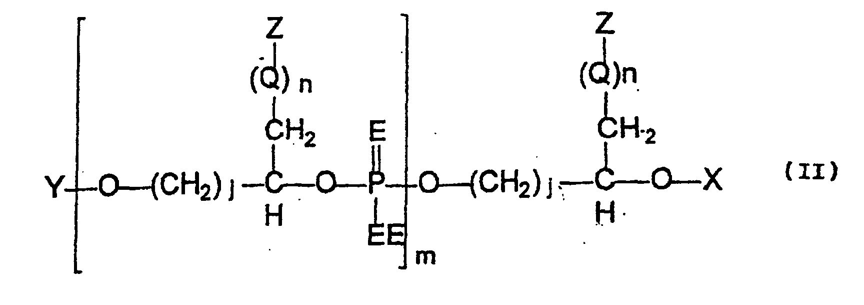 worin:
X H, eine Phosphat-Gruppe, eine aktivierte Phosphat-Gruppe, eine aktivierte Phosphit-Gruppe, ein fester Träger, eine konjugierte Gruppe oder ein Oligonucleotid ist;
Y H, eine Hydroxyl-Schutzgruppe, eine konjugierte Gruppe oder ein Oligonucleotid ist;
E O oder S ist;
EE O⁻ oder N(Y₀)T₀ ist, vorausgesetzt, daß mindestens ein EE N(Y₀)T₀ ist;
Y₀ H, oder [Q₂]ⱼⱼ-Z₂ ist;
T₀ [Q₁]ₖₖ-Z₁ ist, oder Y₀ und Tₒ zusammen in einem Stickstoff-Heterocyclus miteinander verbunden sind;
Q₁ und Q₂, unabhängig, C₂-C₁₀-Alkyl, C₂-C₁₀-Alkenyl, C₂-C₁₀-Alkynyl, C₄-C₇-Carbocycloalkyl oder -alkenyl, ein Heterocyclus, ein Ether mit 2 bis 10 Kohlenstoffatomen und 1 bis 4 Sauerstoff- oder Schwefelatomen, ein Polyalkylglycol, oder C₇-C₁₄-Aralkyl sind;
jj und kk, unabhängig, 0 oder 1 sind;
Z₁ und Z₂, unabhängig, H, C₁-C₂₀-Alkyl, C₂-C₁₀-Alkenyl, C₂-C₁₀-Alkynyl, C₆-C₁₄-Aryl, C₇-C₁₅-Aralkyl, ein Halogen, CH=O, OR₁, SR₂, NR₃R₄, C(=NH)NR₃R₄, CH(NR₃R₄), NHC(=NH)NR₃R₄, CH(NH₂)C(=O)OH, C(=O)NR₃R₄, C(=O)OR₅, eine Metall-Koordinations-Gruppe, eine Reporter-Gruppe, ein Stickstoff-haltiger Heterocyclus, ein Purin, ein Pyrimidin, eine Phosphatgruppe, eine Polyethergruppe, oder eine Polyethylenglycolgruppe sind;
Z L₁, L₁-G₁, L₂, L₂-G₂, NR₃R₄, ein Stickstoff-haltiger Heterocylus, ein Purin, ein Pyrimidin, eine Phosphatgruppe, eine Polyethergruppe, oder eine Polyethylenglycolgruppe ist, vorausgesetzt, daß mindestens zwei der Z und Zₘ verschiedene Hälften sind;
L₁ Alkyl mit 1 bis etwa 20 Kohlenstoffatomen, Alkenyl mit 2 bis etwa 20 Kohlenstoffatomen oder Alkynyl mit 1 bis etwa 20 Kohlenstoffatomen ist;
L₂ Aryl mit 6 bis etwa 14 Kohlenstoffatomen oder Aralkyl mit 7 bis etwa 15 Kohlenstoffatomen ist;
G₁ Halogen, OR₁, SR₂, NR₃R₄, C(=NH)NR₃R₄, NHC(=NH)NR₃R₄, CH=O, C(=O)OR₅, CH(NR₃R₄)(C(=O)OR₅), C(=O)NR₃R₄, eine Metall-Koordinations-Gruppe, oder eine Phosphatgruppe ist;
G₂ Halogen, OH, SH, SCH₃, oder NR₃R₄ ist;
R₁ H, Alkyl mit 1 bis etwa 6 Kohlenstoffatomen oder eine Hydroxyl-Schutzgruppe ist;
R₂ H, Alkyl mit 1 bis etwa 6 Kohlenstoffatomen oder eine Thiol-Schutzgruppe ist;
R₃ und R₄, unabhängig, H, Alkyl mit 1 bis etwa 6 Kohlenstoffatomen oder eine AminoSchutzgruppe sind;
R₅ H, Alkyl mit 1 bis etwa 6 Kohlenstoffatomen oder eine Säureschutzgruppe ist;
Q L₁, G₃, L₁-G₃ oder G₃-L₁-G₃ ist;
G₃ NR₃, C(=O), C(=S), C(O)-O, C(O)-NH, C(S)-O, C(S)-NH, oder S(O)2, NR₃C(=O), NR₃C(=S), NR₃C(O)-O, NR₃C(O)-NH, NR₃C(S)-O, NR₃C(S)-NH oder NR₃S(O)₂ ist;
n 0 oder 1 ist;
j 1 bis 6 ist; und
m 1 bis 50 ist.

6. Verbindung gemäß Anspruch 5, worin Y eine Hydroxyl-Schutzgruppe ist.

7. Verbindung gemäß irgendeinem vorstehenden Anspruch, worin Y Trityl, Methoxytrityl, Dimethoxytrityl, oder Trimethoxytrityl ist.

8. Verbindung gemäß Anspruch 5, worin X H, eine aktivierte Phosphitgruppe, oder ein fester Träger ist.

9. Verbindung gemäß irgendeinem vorstehenden Anspruch, worin X ein Phosphoramidit ist.

10. Verbindung gemäß Anspruch 5, worin n 1 ist und Q und jedes Qₘ, unabhängig, eine Carbonyl-, Thiocarbonyl-, Carboxy-, Acetyl-, Amido-, Succinyl-, Carbamoyl-, Thiocarbamoyl-, Ureido-, Thioureido-, oder Sulfonamidoacyl-Gruppe sind.

11. Verbindung gemäß Anspruch 1 oder 5, worin Z und jedes Zₘ Stickstoff-haltige Heterocyclen sind.

12. Verbindung gemäß Anspruch 11, worin der Stickstoff-haltige Heterocyclus Imidazol, Pyrrol oder Carbazol ist.

13. Verbindung gemäß Anspruch 12, worin Z und jedes Zₘ, unabhängig, Imidazol oder Carbazol sind.

14. Verbindung gemäß Anspruch 1 oder 5, worin Z und jedes Zₘ, unabhängig, Purine oder Pyrimidine sind.

15. Verbindung gemäß Anspruch 14, worin Z und jedes Zₘ, unabhängig, Adenin, Guanin, Cytosin, Uridin oder Thymin sind.

16. Verbindung gemäß Anspruch 15, worin n 0 ist.

17. Verbindung gemäß Anspruch 5, worin Z und jedes Zₘ, unabhängig, Alkyl mit 1 bis etwa 20 Kohlenstoffatomen sind.

18. Verbindung gemäß Anspruch 5, worin Z und jedes Zₘ, unabhängig, C₁-C₆ Alkyl-NH₂ sind.

19. Verbindung gemäß Anspruch 5, worin Z und jedes Zₘ, unabhäng, Aryl mit 6 bis etwa 14 Kohlenstoffatomen oder Aralkyl mit 7 bis etwa 15 Kohlenstoffatomen sind.

20. Verbindung gemäß Anspruch 19, worin Z und jedes Zₘ, unabhängig, Carbazol, Phenyl oder Benzyl sind.

21. Verbindung gemäß Anspruch 5, worin Z und jedes Zₘ, unabhängig, Glutamyl sind.

22. Verbindung gemäß Anspruch 5, worin m 1 bis etwa 25 ist.

23. Verbindung gemäß Anspruch 5, worin Eₘ 0 ist.

24. Verbindung gemäß Anspruch 5, worin jedes Eₘ S ist.

25. Verbindung gemäß Anspruch 5, worin sich Z und jedes Zₘ der Verbindung in einer vorbestimmten Sequenz befinden.

26. Verbindung gemäß Anspruch 5, worin Z und jedes Zₘ von der Verbindung sich in einer Zufallssequenz befinden.

27. Verfahren zur Herstellung einer oligomeren Verbindung mit einer wie in Anspruch 5 definierten Struktur II, enthaltend:
Auswählen aus einer Gruppe von Monomeren, wobei jedes der Monomere eine Struktur III hat 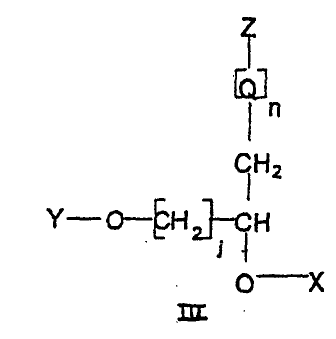 worin:
X H, eine Phosphat-Gruppe, eine aktivierte Phosphat-Gruppe, eine aktivierte Phosphit-Gruppe, ein fester Träger ist;
Y H oder eine Hydroxyl-Schutzgruppe ist;
Z L₁, L₁-G₁, L₂, L₂-G₂, NR₃R₄, ein Stickstoff-haltiger Heterocylus, ein Purin, ein Pyrimidin, eine Phosphatgruppe, eine Polyethergruppe, oder eine Polyethylenglycolgruppe ist,
L₁ Alkyl mit 1 bis etwa 20 Kohlenstoffatomen, Alkenyl mit 2 bis etwa 20 Kohlenstoffatomen oder Alkynyl mit 1 bis etwa 20 Kohlenstoffatomen ist;
L₂ Aryl mit 6 bis etwa 14 Kohlenstoffatomen oder Aralkyl mit 7 bis etwa 15 Kohlenstoffatomen ist;
G₁ Halogen, OR₁, SR₂, NR₃R₄, C(=NH)NR₃R₄, NHC(=NH)NR₃R₄, CH=O, C(=O)OR₅, CH(NR₃R₄)(C(=O)OR₅), C(=O)NR₃R₄, eine Metall-Koordinations-Gruppe, oder eine Phosphatgruppe ist;
G₂ Halogen, OH, SH, SCH₃, oder NR₃R₄ ist;
R₁ H, Alkyl mit 1 bis etwa 6 Kohlenstoffatomen oder eine Hydroxyl-Schutzgruppe ist;
R₂ H, Alkyl mit 1 bis etwa 6 Kohlenstoffatomen oder eine Thiol-Schutzgruppe ist;
R₃ und R₄, unabhängig, H, Alkyl mit 1 bis etwa 6 Kohlenstoffatomen oder eine AminoSchutzgruppe sind;
R₅ H, Alkyl mit 1 bis etwa 6 Kohlenstoffatomen oder eine Säureschutzgruppe ist;
Q L₁, G₃, L₁-G₃ oder G₃-L₁-G₃ ist;
G₃ NR₃, C(=O), C(=S), C(O)-O, C(O)-NH, C(S)-O, C(S)-NH, oder S(O)₂, NR₃C(=O), NR₃C(=S), NR₃C(O)-O, NR₃C(O)-NH, NR₃C(S)-O, NR₃C(S)-NH oder NR₃S(O)₂ ist;
n 0 oder 1 ist;
j 1 bis 6 ist; und
kovalente Bindung von wenigstens zwei der Monomere der Gruppe, um die oligomere Verbindung zu bilden.

28. Verfahren gemäß Anspruch 27, worin die Z-Hälfte von mindestens einem Monomer der Gruppe verschieden von der Z-Hälfte von einem anderen Monomer der Gruppe ist.

29. Verfahren gemäß Anspruch 27, worin m 1 bis 40 ist.

30. Oligomere Verbindung, hergestellt durch das Verfahren gemäß Anspruch 27, worin m 1 bis 25 ist.

31. Heterozygote oligomere Verbindung mit einem zentralen Bereich, der einen Phosphodiester oder ein Phosphorthioatoligodeoxynucleotid enthält, der bzw. das den Raum zwischen Seitenregionen einnimmt, die eine Struktur II haben: 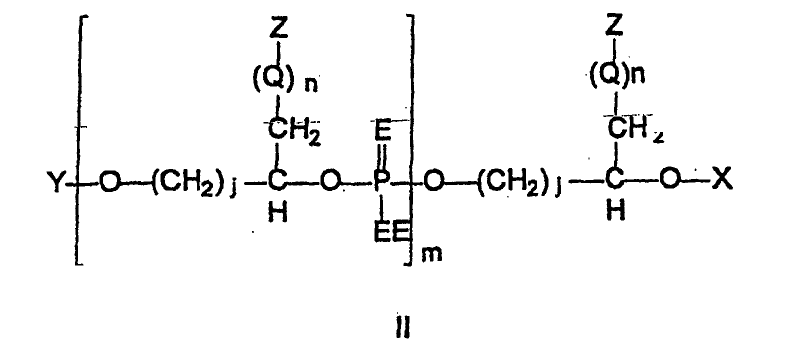 worin X, Y, Z, Q, E, EE, n, m, und j wie in Anspruch 5 definiert sind, aber ohne die Maßgabe, daß mindestens zwei von Z und Zₘ verschiedene Hälften sind.

## Revendications

1. Composé ayant la structure I : 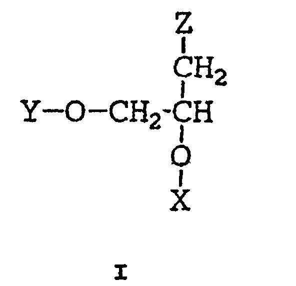 dans laquelle :
X est un groupe phosphate activé, un groupe phosphite activé, ou un support solide ;
Y est un groupe protecteur d'hydroxyle ; et
Z est une purine, une pyrimidine, un hétérocycle azoté, ou un groupe alkyle en C₁-C₁₀ linéaire ou ramifié.

2. Composé de la revendication 1 dans lequel X est un groupe phosphite activé ou un support solide.

3. Composé de la revendication 1 dans lequel X est un groupe phosphite activé et Z est une adénine, une guanine, une cytosine, une uridine ou une thymine.

4. Composé de la revendication 1 dans lequel Z est un groupe alkyle en C₁-C₁₀ linéaire ou ramifié.

5. Composé avant la structure II : 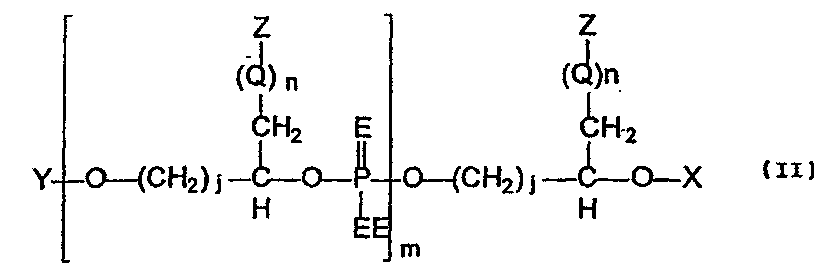 dans laquelle :
X est H, un groupe phosphate, un groupe phosphate activé, un groupe phosphite activé, un support solide, un groupe conjugué, ou un oligonucléotide ;
Y est H, un groupe protecteur d'hydroxyle, un groupe conjugué ou un oligonucléotide ;
E est O ou S ;
EE est O⁻ ou N(Y₀)T₀, à condition qu'au moins un EE soit N(Y₀)T₀ ;
Y₀ est H, ou [Q₂]ⱼⱼ-Z₂ ;
T₀ est [Q₁]ₖₖ-Z₁, ou Y₀ et T₀ ensemble sont réunis en un hétérocycle azoté ;
Q₁ et Q₂ sont indépendamment un groupe alkyle en C₂-C₁₀, alcényle en C₂-C₁₀, alcynyle en C₂-C₁₀, carbocyclo-alkyle ou -alcényle en C₄-C₇, un hétérocycle, un éther ayant 2 à 10 atomes de carbone et 1 à 4 atomes d'oxygène ou de soufre, un polyalkylglycol, ou un groupe aralkyle en C₇-C₁₄;
jj et kk valent indépendamment 0 ou 1 ;
Z₁ et Z₂ sont indépendamment H, un groupe alkyle en C₁-C₂₀, alcényle en C₂-C₂₀, alcynyle en C₂-C₂₀, aryle en C₆-C₁₄, aralkyle en C₇-C₁₅, un halogène, CH=O, OR₁, SR₂, NR₃R₄, C(=NH)NR₃R₄, CH(NR₃R₄), NHC(=NH)NR₃R₄, CH(NH₂)C(=O)OH, C(=O)NR₃R₄, C(=O)OR₅, un groupe de coordination de métal, un groupe marqueur, un hétérocycle azoté, une purine, une pyrimidine, un groupe phosphate, un groupe polyéther, ou un groupe polyéthylèneglycol ;
Z est L₁, L₁-G₁, L₂, L₂-G₂, NR₃R₄, un hétérocycle azoté, une purine, une pyrimidine, un groupe phosphate, un groupe polyéther, ou un groupe polyéthylèneglycol, à condition qu'au moins deux des Z et Zₘ soient des groupements différents ;
L₁ est un groupe alkyle ayant 1 à environ 20 atomes de carbone, alcényle ayant 2 à environ 20 atomes de carbone, ou alcynyle ayant 2 à environ 20 atomes de carbone ;
L₂ est un groupe aryle ayant 6 à environ 14 atomes de carbone ou aralkyle ayant 7 à environ 15 atomes de carbone ;
G₁ est un halogène, OR₁, SR₂, NR₃R₄, C(=NH)NR₃R₄, NHC(=NH)NR₃R₄, CH=O, C(=O)OR₅, CH(NR₃R₄) (C(=O)OR₅), C(=O)NR₃R₄, un groupe de coordination de métal, ou un groupe phosphate ;
G₂ est un halogène, OH, SH, SCH₃, ou NR₃R₄ ;
R₁ est H, un groupe alkyle ayant 1 à environ 6 atomes de carbone, ou un groupe protecteur d'hydroxyle ;
R₂ est H, un groupe alkyle ayant 1 à environ 6 atomes de carbone, ou un groupe protecteur de thiol ;
R₃ et R₄ sont indépendamment H, un groupe alkyle ayant 1 à environ 6 atomes de carbone, ou un groupe protecteur d'amine ;
R₅ est H, un groupe alkyle ayant 1 à environ 6 atomes de carbone, ou un groupe protecteur d'acide ;
Q est L₁, G₃, L₁-G₃ ou G₃-L₁-G₃ ;
G₃ est NR₃, C(=O), C(=S), C(O)-O, C(O)-NH, C(S)-O, C(S)-NH ou S(O)₂, NR₃C(=O), NR₃C(=S), NR₃C(O)-O, NR₃C(O)-NH, NR₃C(S)-O, NR₃C(S)-NH ou NR₃S(O)₂ ;
n vaut 0 ou 1 ;
j vaut 1 à 6 ; et
m vaut 1 à 50.

6. Composé de la revendication 5 dans lequel Y est un groupe protecteur d'hydroxyle.

7. Composé de l'une quelconque des revendications précédentes dans lequel X est un groupe trityle, méthoxytrityle, diméthoxytrityle ou triméthoxytrityle.

8. Composé de la revendication 5 dans lequel X est H, un groupe phosphite activé, ou un support solide.

9. Composé de l'une quelconque des revendications précédentes dans lequel X est un phosphoramidite.

10. Composé de la revendication 5 dans lequel n vaut 1 et Q et chaque Qₘ, indépendamment, sont un groupe carbonyle, thiocarbonyle, carboxy, acétyle, amido, succinyle, carbamoyle, thiocarbamoyle, uréido, thiouréido, ou sulfonamidoacyle.

11. Composé de la revendication 1 ou 5, dans lequel Z et chaque Zₘ sont des hétérocycles azotés.

12. Composé de la revendication 11 dans lequel ledit hétérocycle azoté est un imidazole, un pyrrole ou un carbazole.

13. Composé de la revendication 12 dans lequel Z et chaque Zₘ, indépendamment, sont un imidazole ou un carbazole.

14. Composé de la revendication 1 ou 5, dans lequel Z et chaque Zₘ, indépendamment, sont des purines ou des pyrimidines.

15. Composé de la revendication 14 dans lequel Z et chaque Zₘ, indépendamment, sont une adénine, une guanine, une cytosine, une uridine ou une thymine.

16. Composé de la revendication 15 dans lequel n vaut 0.

17. Composé de la revendication 5, dans lequel Z et chaque Zₘ, indépendamment, sont un groupe alkyle ayant 1 à environ 20 atomes de carbone.

18. Composé de la revendication 5 dans lequel Z et chaque Zₘ, indépendamment, sont un groupe (alkyl en C₁-C₆) -NH₂.

19. Composé de la revendication 5 dans lequel Z et chaque Zₘ, indépendamment, sont un groupe aryle ayant 6 à environ 14 atomes de carbone ou aralkyle ayant 7 à environ 15 atomes de carbone

20. Composé de la revendication 19 dans lequel Z et chaque Zₘ, indépendamment, sont un carbazole ou un groupe phényle ou benzyle.

21. Composé de la revendication 5 dans lequel Z et chaque Zₘ, indépendamment, sont un groupe glutamyle.

22. Composé de la revendication 5 dans lequel m vaut 1 à environ 25.

23. Composé de la revendication 5 dans lequel Eₘ est O.

24. Composé de la revendication 5 dans lequel chaque Eₘ est S.

25. Composé de la revendication 5 dans lequel Z et chaque Zₘ dudit composé sont dans une séquence prédéterminée.

26. Composé de la revendication 5 dans lequel Z et chaque Zₘ dudit composé sont dans une séquence aléatoire.

27. Procédé de préparation d'un composé oligomère ayant la structure II telle que définie dans la revendication 5, comprenant :
la sélection d'un groupe de monomères, chacun desdits monomères ayant la structure III 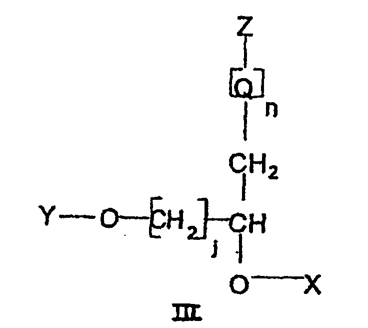 dans laquelle :
X est H, un groupe phosphate, un groupe phosphate activé, un groupe phosphite activé, ou un support solide ;
Y est H ou un groupe protecteur d'hydroxyle ;
Z est L₁, L₁-G₁, L₂, L₂-G₂, NR₃R₄, un hétérocycle azoté, une purine, une pyrimidine, un groupe phosphate, un groupe polyéther, ou un groupe polyéthylèneglycol ;
L₁ est un groupe alkyle ayant 1 à environ 20 atomes de carbone, alcényle ayant 2 à environ 20 atomes de carbone, ou alcynyle ayant 2 à environ 20 atomes de carbone ;
L₂ est un groupe aryle ayant 6 à environ 14 atomes de carbone ou aralkyle ayant 7 à environ 15 atomes de carbone ;
G₁ est un halogène, OR₁, SR₂, NR₃R₄, C(=NH)NR₃R₄, NHC(=NH)NR₃R₄, CH=O, C(=O)OR₅, CH (NR₃R₄) (C (=O) OR₅), C(=O)NR₃R₄, un groupe de coordination de métal, ou un groupe phosphate ;
G₂ est un halogène, OH, SH, SCH₃, ou NR₃R₄ ;
R₁ est H, un groupe alkyle ayant 1 à environ 6 atomes de carbone, ou un groupe protecteur d'hydroxyle ;
R₂ est H, un groupe alkyle ayant 1 à environ 6 atomes de carbone, ou un groupe protecteur de thiol ;
R₃ et R₄ sont indépendamment H, un groupe alkyle ayant 1 à environ 6 atomes de carbone, ou un groupe protecteur d'amine;
R₅ est H, un groupe alkyle ayant 1 à environ 6 atomes de carbone, ou un groupe protecteur d'acide ;
Q est L₁, G₃, L₁-G₃ ou G₃-L₁-G₃ ;
G₃ est NR₃, C(=O), C(=S), C(O)-O, C(O)-NH, C(S)-O, C(S)-NH ou S(O)₂, NR₃C(=O), NR₃C(=S), NR₃C(O)-O, NR₃C(O)-NH, NR₃C(S)-O, NR₃C(S)-NH ou NR₃S(O)₂ ;
n vaut 0 ou 1 ;
j vaut 1 à 6 ; et
la liaison covalente d'au moins deux desdits monomères dudit groupe pour former ledit composé oligomère.

28. Procédé de la revendication 27 dans lequel le groupement Z d'au moins un monomère dudit groupe est différent du groupement Z d'un autre monomère dudit groupe.

29. Procédé de la revendication 27 dans lequel m vaut 1 à 40.

30. Composé oligomère préparé par le procédé de la revendication 27 dans lequel m vaut 1 à 25.

31. Composé oligomère chimérique ayant une région centrale comprenant un oligodésoxynucléotide phosphodiester ou phosphorothioate intercalé entre des régions adjacentes ayant la structure II : 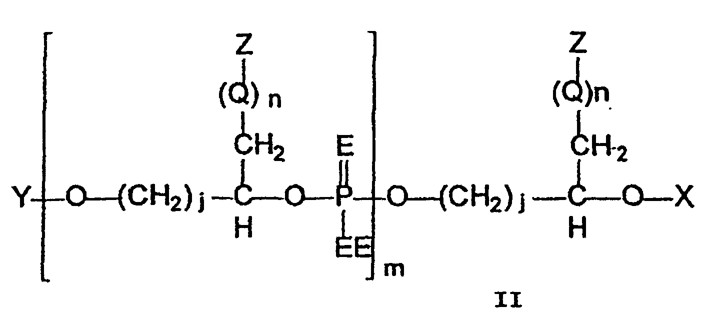 dans laquelle X, Y, Z, Q, E, EE, n, m etj sont tels que définis dans la revendication 5, mais sans la condition qu'au moins deux des Z et Zₘ soient des groupements différents.
